# EUROPÄISCHE PATENTSCHRIFT

(11) **EP 1 675 623 B1**
(45) Veröffentlichungstag und Bekanntmachung des Hinweises auf die Patenterteilung: **20.03.2013**
(21) Anmeldenummer: 05793770.8
(22) Anmeldetag: 11.10.2005
(51) Int. Cl.: C07K 14/00

(54) **UBIQUITIN ODER GAMMA-KRISTALLIN-KONJUGATE ZUR VERWENDUNG IN THERAPIE, DIAGNOSE UND CHROMATOGRAPHIE**
UBIQUITIN OR GAMMA-CRYSTALLINE CONJUGATES FOR USE IN THERAPY, DIAGNOSIS AND CHROMATOGRAPHY
CONJUGUES D'UBIQUITINE OU DE GAMMA-CRYSTALLINE ET LEUR UTILISATION POUR LA THERAPIE, LE DIAGNOSTIC ET LA CHROMATOGRAPHIE

(30) Priorität: 11.10.2004 DE 102004049479
(43) Veröffentlichungstag der Anmeldung: 05.07.2006
(73) Patentinhaber: Scil Proteins GmbH, 06120 Halle/Saale (DE)
(72) Erfinder: FIEDLER, Erik, 06114 Halle/saller (DE); EBERSBACH, Hilmar, CH-8304 Wallisellen (CH); HEY, Thomas, 61231 Bad Nauheim (DE); FIEDLER, Ulrike, 06114 Halle/Saale (DE)
(74) Vertreter: Isarpatent
(86) Internationale Anmeldenummer: PCT/EP2005/010932
(87) Internationale Veröffentlichungsnummer: WO 2006/040129

(56) Entgegenhaltungen:
- WO-A-01/04144
- WO-A-99/16873
- YEH E T H ET AL: "Ubiquitin-like proteins: new wines in new bottles" GENE, ELSEVIER, AMSTERDAM, NL, Bd. 248, Nr. 1-2, Mai 2000 (2000-05), Seiten 1-14, XP004198791 ISSN: 0378-1119
- VIJAY-KUMAR S ET AL: "Structure of ubiquitin refined at 1.8 A resolution", JOURNAL OF MOLECULAR BIOLOGY, ACADEMIC PRESS, UNITED KINGDOM, vol. 194, no. 3, 5 April 1987 (1987-04-05) , pages 531-544, XP024020230, ISSN: 0022-2836, DOI: 10.1016/0022-2836(87)90679-6 [retrieved on 1987-04-05]

## Beschreibung

Die vorliegende Erfindung betrifft ein Verfahren zur Herstellung eines Konjugates umfassend ein oder mehrere auf humanem Ubiquitin basierende Polypeptidmoleküle und einen oder mehrere funktionelle Bestandteile.

WO 01/04144 A offenbart Konjugate eines Proteins mit Beta-Faltblattstruktur (Gamma-Kristallin-Mutante), das an Estradiol bindet. Insbesondere wird die terminale Fusion von Gamma-Kristallin-Mutanten mit verschiedenen Molekülen, nämlich einem E-Tag, mit dem minor coat Protein 3 sowie mit einem His-Tag beschrieben.

WO 01/04144 A beschreibt ferner, stabile Beta-Faltblatt-Proteine durch ortspezifische Mutagenese im Beta-Faltblatt an der Oberfläche so zu verändern, dass aus dem nicht-bindenden Protein ein Protein mit spezifischen Bindungseigenschaften entsteht.

Als *scaffold (Gerüst*) Protein für die Generierung alternativer Bindemoleküle findet humanes Ubiquitin Anwendung. Ubiquitin ist ein kleines, monomeres und zytosolisches Protein, das - in seiner Sequenz hochkonserviert - von den Protozoen bis zu den Vertebraten in allen bekannten eukaryotischen Zellen vorkommt. Es spielt im Organismus eine grundlegende Rolle bei der Regulation des kontrollierten Abbaus zelleigener Proteine.

Die Polypeptidkette des Ubiquitins besteht aus 76 Aminosäuren, die in einer äußerst kompakten alpha/beta-Struktur gefaltet sind (Vijay-Kumar, 1987): Nahezu 87 % der Polypeptidkette sind durch Wasserstoffbrücken an der Ausbildung der Sekundärstrukturelemente beteiligt. Als prominente Sekundärstrukturen können dreieinhalb alpha-helikale Windungen sowie ein aus fünf Strängen bestehendes, antiparalleles Beta-Faltblatt gelten. Die charakteristische Anordnung dieser Elemente - ein zu einer Oberfläche des Proteins exponiertes antiparalleles Beta-Faltblatt, welches auf seiner Rückseite von einer senkrecht darüber liegenden alpha-Helix bedeckt wird, - gilt generell als sogenanntes Ubiquitin-artiges Faltungsmotiv. Ein weiteres Merkmal der Struktur ist ein ausgeprägter hydrophober Bereich im Inneren des Proteins zwischen alpha-Helix und beta-Faltblatt.

Die künstliche Herstellung von Ubiquitin ist aufgrund der geringen Größe sowohl durch chemische Synthese, als auch mittels biotechnologischer Verfahren möglich. Wegen der günstigen Faltungseigenschaften kann Ubiquitin bei der gentechnischen Gewinnung mit Hilfe von Mikroorganismen wie z. B. *E.coli* in verhältnismäßig großen Mengen wahlweise im Zytosol oder dem periplasmatischem Raum produziert werden.

Die einfache und effiziente bakterielle Herstellung ermöglicht die Verwendung von Ubiquitin als Fusionspartner für andere herzustellende Fremdproteine, deren Produktion problematisch ist. Durch die Fusion mit Ubiquitin kann eine verbesserte Löslichkeit und damit eine verbesserte Ausbeute erzielt werden (Butt et al., 1989).

Ausgehend von vorliegenden Kristallstrukturdaten (PDB Datenbankeintrag 1UBI) konnten mittels rechnergestützter Analyse die Positionen solcher Aminosäuren im Ubiquitin-Proteingerüst lokalisiert werden, deren Seitenketten oberflächenexponiert d. h. dem Lösungsmittel oder einem potentiellen Bindungspartner zugewandt sind. Die ausgewählten Positionen befinden sich in räumlicher Nähe zueinander am Beginn des ersten aminoterminalen beta-Faltblattstrangs (Pos. 2, 4, 6) sowie im *loop* (Pos. 62, 63) bzw. am Beginn des carboxyterminalen beta-Faltblattstrangs (Pos. 64, 65, 66) und bilden mit ihren Aminosäureseitenketten einen zusammenhängenden Bereich auf der Oberfläche des Ubiquitins. Durch zufällige Aminosäure-Substitutionen in der analysierten Region wurde so ein hypervariabler oberflächenexponierter Bereich auf der weiterhin intakten Proteinstruktur des Ubiquitins generiert (PCT/EP2004/005730, nicht veröffentlicht).

Die Generierung einer künstlichen Bindungsfläche auf einem beta-Faltblatt Protein stellt eine neuartige und interessante Alternative zu herkömmlichen Antikörpern dar. Es konnte der Beweis erbracht werden, das eine neue, künstlich generierte Bindungsstelle auf der Oberfläche von gamma-Kristallinen oder Ubiquitin-ähnlichen Proteinen zu funktionsfähigen Bindemolekülen führt (DE 19932688 A1) (PCT/EP2004/005730, nicht veröffentlicht).

Jedoch fehlte bisher jede Anregung oder Hinweis darauf, diese Polypeptidmoleküle zur Bildung eines Konjugat an einen weiteren Bestandteil zu koppeln, um sie für diagnostische, therapeutische und analytische Anwendungen brauchbar zu machen, ohne dabei einen Funktionsverlust eines der beiden oder beider Bestandteile zu erleiden.

Der vorliegenden Erfindung liegt somit die Aufgabe zugrunde, ein Verfahren zur Herstellung eines Konjugats aus einem auf Ubiquitin basierenden Polypeptid und einem funktionellen Bestandteil bereitzustellen, wobei das jeweilige Polypeptidmolekül eine gegenüber dem Wildtyp-Polypeptid veränderte Bindungseigenschaft zur spezifischen Bindung an einen Liganden aufweist, wobei beide Bestandteile des Konjugates nach Kopplung aneinander ihre Funktionalität beibehalten oder diese durch die Kopplung sogar gesteigert wird.

Diese Aufgabe wird durch den Gegenstand der unabhängigen Ansprüche gelöst. Bevorzugte Ausführungsformen ergeben sich aus den abhängigen Ansprüchen.

Die vorliegende Erfindung betrifft die ortsspezifische und selektiv, ungerichtete Kopplung von neuartigen Bindeproteinen, die auf Ubiquitin basieren, an verschiedenste Moleküle, wie z.B. Proteine (chromophore Proteine und Enzyme), Matrices (z.B. Dextran, Polymethacrylat, Agarose, Sepharose, Polystyrenderivate und Cellulose) und kleine Moleküle (beispielweise Fluoreszenzmarker, Biotin, Digoxigenin, Radioisotope, Antibiotika u.a.). Diesen, so genannten Affilin™ Molekülen eigen ist das *de novo* Design einer Bindungsregion in Beta-Faltblattstrukturen der Proteine. Somit unterscheiden sie sich von der prominentesten Klasse an Bindeproteinen in der Natur, den Antikörpern, bei denen die Bindungsregion in flexiblen *loop*-Bereichen (CDR's) des Proteins lokalisiert ist. Ein weiterer Unterschied zwischen Affilin™ und Antikörpern ist die Molekülgröße. Die als Basis für die erste Komponente des erfindungsgemäßen Konjugates eingesetzten Polypeptide besitzen ein Molekulargewicht von 10 kDa (Ubiquitin), Antikörper ein Molekulargewicht von 150 kDa (IgG).

Die vorliegende Erfindung beinhaltet Verfahren zur Herstellung von Konjugaten aus diesen Polypeptiden und den entsprechenden Kopplungspartnern. Ferner ist deren Einsatz in verschiedenen Anwendungen beschrieben und überraschenderweise gezeigt worden, dass diese Kopplungsmethoden weder zum Verlust der biologischen Aktivität des Kopplungspartners noch zum Verlust der Bindungseigenschaften der Polypeptide an ihre Liganden führen.

Beispielhaft für die Erfindung werden die Kopplung der Polypeptid-Komponenten an eine Dextranmatrix im BIACORE System, die Kopplung an den Fluoreszenzfarbstoff Oyster^{®}556, das chromophore Protein Phycoerythrin (PE) und das Enzym Meerrettich-Peroxidase, sowie die Immobilisierung eines Polypeptides an ein Trägermaterial zur Verwendung in der Affinitätschromatographie beschrieben. Erfindungsgemäß wurden die Kopplungen dabei entweder direkt an das Polypeptid-Molekül, z.B. an nucleophile Seitenketten der Proteine, oder zielgerichtet an C-terminale Peptidlinker realisiert. Überraschenderweise waren die Kopplungsmethoden auf beide *scaffolds* übertragbar und führten zu keiner Beeinträchtigung der Bindungseigenschaften dieser Moleküle. Gerade die Kopplung der relativ kleinen Polypeptid-Moleküle (10 kDa) mit großen Proteinen wie z.B. Phycoerythrin (240 kDa), bei gleichzeitiger Beibehaltung der Bindungsaktivität, war nicht zu erwarten.

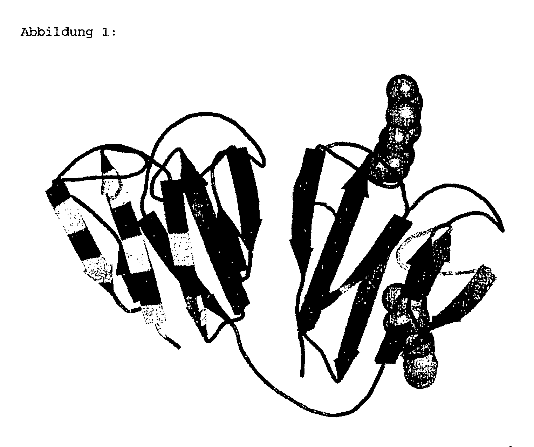

Die so erhaltenen Konjugate schränken die Bindungsfähigkeit der Affilin™ Moleküle überraschenderweise nicht ein, vielmehr konnte bei bestimmten Konjugaten eine Erhöhung der makroskopischen Dissoziationskonstante beobachtet werden (Aviditätseffekt), was weitere Möglichkeiten für den Einsatz der Polypeptid-Moleküle in z.B. der Therapie bietet. Außerdem zeigte sich, dass diese Polypeptid-Moleküle auch nach Kopplung an wasserunlösliche Matrices Bindungsaktivität zeigen und nach Behandlung mit denaturierenden Agenzien wie Harnstoff, Guanidinium, Ethanol, Natronlauge oder Salzsäure regenerierbar sind, das heißt ihre Bindungseigenschaften wieder hergestellt werden können. Durch detaillierte Analyse der Strukturdaten von Ubiquitin basierten Molekülen eröffnete sich die Möglichkeit, die unspezifische Kopplung selektiv zu gestalten, indem gezielt Lysinreste angesteuert werden. Die Strukturanalyse zeigte, dass sich Lysine beim gamma-Kristallin in der C-terminalen Domäne des Proteins befinden und damit für eine Kopplung geeignet sein sollten (Abb.1). Auch beim Ubiquitin gelang die Identifizierung solcher Lysinreste. Allerdings muss für solche Kopplungsstrategien sichergestellt sein, dass sich keine weiteren Lysine in der Bindungsregion befinden.

Für die vorliegende Erfindung wurden aus der humanen Ubiquitin Bibliothek (UB10) ein NGFbindendes Polypeptid selektiert und nachfolgend gereinigt. Durch Einführung eines speziellen C-terminalen Peptidlinkers definierter Länge inklusive eines Cysteins gelang es Polypeptid-Moleküle so zu modifizieren, dass eine selektive Kopplung ohne Beeinträchtigung der Bindungsaktivität erhalten wurde. Für eine effiziente Kopplung ist es notwendig, alle restlichen zugänglichen Cysteine zu eliminieren. Bei der unspezifischen Kopplungsmethode der Polypeptid-Moleküle gelang es überraschenderweise sowohl die Bindungsaktivität der Polypeptide zu erhalten und darüber hinaus eine Erhöhung der Affinität durch Avidität zu erreichen und damit ein sehr attraktives Polypeptid-basiertes Molekülkonjugat für Diagnostik und Therapie herzustellen.

Solche alternativen Bindemoleküle lassen sich vielseitig in der Therapie, der Diagnostik und der Chromatographie einsetzen. Durch die Kopplung von Polypeptiden an unterschiedlichste Partner eröffnet sich diesen neuartigen Bindemolekülen ein noch breiteres Einsatzgebiet.

Die vorliegende Erfindung umfasst die folgenden Aspekte und Ausführungsformen:
Gemäß eines ersten Aspektes betrifft die vorliegende Erfindung ein Verfahren zur Herstellung eines Konjugats das die folgenden Bestandteile umfasst: ein oder mehrere auf humanem Ubiquitin basierende Polypeptidmoleküle (I), und, damit kovalent verknüpft, einen oder mehrere funktionelle Bestandteile (II) ausgewählt aus der Gruppe, die aus Polypeptiden und Proteinen, organischen Polymeren, Zuckern, niedermolekularen Substanzen, Peptiden, sowie Derivaten dieser Substanzen, besteht, wobei nach Kopplung von (I) an (II) die Funktionalität aller Bestandteile erhalten bleibt, und wobei das Verfahren folgende Schritte umfasst: a) Bereitstellen von humanem Ubiquitin; b) Bereitstellen eines Liganden, ausgewählt aus (Poly)Peptiden/Proteinen; c) Auswählen der Aminosäuren 2, 4, 6, 62, 63, 64, 65 und 66 des humanen Ubiquitins; d) Modifizieren der ausgewählten Aminosäuren durch Substitution unter Beibehaltung des Ubiquitin-artigen Faltungsmotivs, e) Inkontaktbringen des modifizierten Proteins mit dem in Schritt b) bereitgestellten Liganden; f) Ermitteln derjenigen Proteine, die zur spezifischen Bindung an den Liganden mit einer Dissoziationskonstante von KD=10-5M oder kleiner geeignet sind, wobei der Nachweis der Bindungsaktivität über ELISA-Technik oder Oberflächenplasmonresonanz erfolgt; g) Kopplung der Bestandteile (I) und (II) in einem Bereich außerhalb der oberflächenexponierten Region des β-Faltblattes des Polypeptidmoleküls (I), die zur spezifischen Bindung an den Liganden vorgesehen ist, über eine terminale Peptidfusion an (I), oder über Cystein- oder Lysinseitenketten in (I); h) Isolierung des Konjugates; und i) Nachweis der Funktionalität beider Komponenten des Konjugates..

Mit anderen Worten umfasst das erfindungsgemäß verwendete Konjugat nicht Ubiquitin in seiner Wildtyp-Form, sondern nur in an den speziellen Liganden angepasster Form. Diese speziell angepasste Form sieht gegenüber der Wildtyp-Form veränderte (verbesserte) oder neu generierte Bindungseigenschaften an den jeweiligen Liganden vor. Als gemeinsames Prinzip der auf Ubiquitin basierenden Polypeptidmoleküle sei darauf hingewiesen, dass die Generierung einer künstlichen Bindungsfläche auf einer Beta-Faltblatt-Struktur erfolgt, um eine spezifische Bindung an einen Liganden von Interesse zu ermöglichen. Das Vorhandensein wenigstens einer Beta-Faltblattstruktur zur Bereitstellung einer künstlichen Bindungsfläche ist also ein essentielles Merkmal der Erfindung.

Ein erfindungsgemäß hergestelltes Konjugat bezeichnet in diesem Zusammenhang die posttranslationale, kovalente Verknüpfung eines Polypeptidmoleküles mit einem weiteren Bestandteil und unterscheidet sich daher z.B. von einer Fusion von Polypeptiden auf genetischer Ebene. Fusionspolypeptide sind das Ergebnis einer sog. Translationsfusion.

Das auf Ubiquitin basierende Polypeptid-Molekül wird erfindungsgemäß bevorzugt aus der Gruppe ausgewählt, bestehend aus Proteinen der Protein-Superfamilie "ubiquitin-like proteins", Proteinen, die ein Ubiquitin-artiges Faltungsmotiv aufweisen sowie Fragmenten oder Fusionsproteinen hiervon, die je das Ubiquitin-artige Faltungsmotiv besitzen, wobei das Protein unter Beibehaltung des Ubiquitin-artigen Faltungsmotivs, aufgrund einer oder mehrerer Modifikationen von Aminosäuren in zumindest einem oberflächenexponierten Bereich des Proteins, der mindestens einen beta-Faltblattstrang des Beta-Faltblattbereichs und wahlweise Nicht-Beta-Faltblattbereiche beinhaltet, eine vorher nicht vorhandene Bindungsaffinität gegenüber einem vorbestimmten Bindungspartner bzw. Liganden aufweist.

Beschrieben ist somit auch ein durch Substitution modifiziertes Protein, das aus der Gruppe ausgewählt ist, bestehend aus Proteinen der Protein-Superfamilie "ubiquitin-like proteins", Proteinen, die ein Ubiquitin-artiges Faltungsmotiv aufweisen sowie Fragmenten oder Fusionsproteinen hiervon, die alle jeweils ein Ubiquitin-artiges Faltungsmotiv aufweisen, wobei das Protein aufgrund dieser Modifikation eine vorher nicht vorhandene Bindungsaffinität gegenüber einem vorbestimmten Bindungspartner besitzt.

Das im erfindungsgemäßen Konjugat eingesetzte auf Ubiquitin basierende Polypeptid-Molekül (I) wird folglich durch Modifikation von humanem Ubiquitin hergestellt. Die Modifikationen umfassen insbesondere den Austausch von Aminosäuren. Diese Modifikationen werden in mindestens einem oberflächenexponierten Bereich des zu modifizierenden Proteins vorgenommen. Die Modifikation zumindest einer Aminosäure umfaßt mindestens einen Beta-Faltblattstrang des Beta-Faltblattbereichs, wobei der Beta-Faltblattstrang an der Oberfläche des Proteins lokalisiert sein muß, so daß er für den Bindungspartner bzw. Liganden, der mit einer bestimmbaren Affinität an das modifizierte Protein binden kann, zugänglich ist. In einer weiteren Ausführungsform der Erfindung werden, zusätzlich zu den Änderungen im Beta-Faltblattstrang des Beta-Faltblattbereichs, auch nicht-beta-Faltblattbereiche modifiziert, welche bevorzugt oberflächenexponiert sind, um die Bindungsaffinität gegenüber dem vorbestimmten Bindungspartner zu beeinflussen, insbesondere zu erhöhen und damit die Spezifität zu steigern.

Dem Fachmann stehen verschiedenste, an sich bekannte Techniken zur Modifikation einer oder mehrerer Aminosäuren zur Verfügung. Diese werden nachfolgend detaillierter beschrieben. Ergänzend wird auch hingewiesen auf die Veröffentlichungen von Ausuebel et al., 1994, sowie Sambrook et al., 1989.

Modifikationen von Aminosäuren des nicht oberflächenexponierten Kernbereichs des Ubiquitins sind bereits bekannt (Finucane et al., 1999; Lazar et al., 1997). Die dort vorgenommenen Änderungen betreffen Positionen, die aufgrund der Lokalisation im hydrophoben Kern nicht an Bindungen beteiligt sind, da sie dem Lösungsmittel oder möglichen Bindungspartnern nicht zugänglich sind.

Im folgenden soll erläutert werden, was unter dem Begriff "vorher nicht vorhandene Bindungseigenschaft" bzw. *de novo* generierte, künstliche Bindungsstelle bzw. "gegenüber dem Wildtyp-Polypeptid veränderte Bindungseigenschaft zur spezifischen Bindung an einen Liganden" in dieser Erfindung verstanden wird. Hierunter ist zu verstehen, dass das modifizierte Protein an dem modifizierten Bereich vorher keine Bindungseigenschaft zu einem vorbestimmten Bindungspartner oder einem natürlichen Bindungspartner von Ubiquitin aufweist.

Die Bindungspartner, die auch als Liganden definiert werden können, weisen eine meßbare Affinität zum erfindungsgemäß modifizierten Protein auf. Als Mindestwert für das Vorliegen einer quantifizierbaren Bindungseigenschaft, d.h. der Affinität, mit welcher der Partner gebunden wird, kann erfindungsgemäß eine Dissoziationskonstante für den gebildeten Komplex von K_{D} = 10⁻⁵ M oder kleiner angesehen werden. Ab einem Wert von 10⁻⁵ M kann von einer quantifizierbaren Bindungsaffinität ausgegangen werden. Bevorzugt ist je nach Anwendung ein Wert von 10⁻⁶ M bis 10⁻¹² M, weiterhin bevorzugt 10⁻⁶ bis 10⁻¹¹ M für z. B. chromatographische Anwendungen oder 10⁻⁹ bis 10⁻¹² M für z. B. diagnostische oder therapeutische Anwendungen. Weitere bevorzugte Bindungsaffinitäten liegen im Bereich von 10⁻⁷ bis 10⁻¹⁰M, bevorzugt bis 10⁻¹¹M. Die Verfahren zur Bestimmung der Bindungsaffinitäten sind an sich bekannt und werden auf den nachfolgenden Seiten weiter beschrieben.

Unter Modifikation sind erfindungsgemäß Substitutionen von Aminosäuren zu verstehen.

Die Superfamilie der "ubiquitin-like proteins" umfasst erfindungsgemäß die in Murzin et al. (1995) aufgezählten Untergruppen. Hierzu gehören bspw. die Protein-Familien "ubiquitin-related proteins", "UBX domain", "GABARAP-like", RAS-binding domain" etc. Auch sind solche Proteine umfasst, die ein Ubiquitin-artiges Faltungsmotiv aufweisen. Beispiele hierfür sind SUMO-1, FAU, NEDD-8, UBL-1 und GDX sowie Rub1, APG8, ISG15, URM1, HUB1, Elongin B, PLIC2. (N-terminale Domäne), human Parkin (N-terminale Domäne).

Die Proteine der Superfamilie ubiquitin-like proteins sind genauestens definiert. Nur beispielsweise wird auf folgende Internetseite hingewiesen: http://bip.weizmann.ac.il/scop/index.html. Danach ist die Familie der ubiquitin-like proteins als Superfamilie definiert, zu welcher die Familie der ubiquitin-related-proteins gehört. Alle Mitglieder dieser Superfamilie zeichnen sich hauptsächlich durch antiparallel angeordnete β-Faltblätter aus, die in α- und β-Abschnitte unterteilt sind. Die Faltung wird als beta-Grasp und damit ubiquitin-ähnlich definiert. Der Kernbereich ist wie folgt definiert: beta(2)-alpha-beta(2), wobei die Ziffern die Anzahl der Stränge bezeichnen, wobei die Summe der Stränge das β-Faltblatt bildet. Die Anordnung des mixed-beta-sheets ist 2143, worunter die Lage der Stränge bei Aufsicht auf das Faltblatt von links nach rechts (Aminoterminus unten, Carboxyterminus oben) bezeichnet wird. Charakteristisch für die Mitglieder der ubiquitin-like-proteins ist damit ein zu einer Oberfläche des Proteins exponiertes antiparalleles β-Faltblatt, welches auf seiner Rückseite von einer senkrecht darüberliegenden α-Helix bedeckt wird. Dieses ubiquitin-artige Faltungsmotiv ist charakteristisch für die Proteine und grenzt die Mitglieder der Familie von anderen Proteinen eindeutig ab. Unter Berücksichtigung dieser Definition werden von auch umfaßt die ubiquitin-ähnliche N-terminale Domäne von PLIC-2 und die ubiquitin-ähnliche Domäne von Parkin.

Der Fachmann kann entweder anhand von Sequenzvergleichen, sogenannten Alignments oder Strukturüberlagerungen erste Schlüsse ziehen, ob es sich bei den Proteinen um ein Mitglied der Protein-Superfamilie der ubiquitin-like-proteins handelt. Letzte Sicherheit bietet natürlich immer eine Strukturanalyse, beispielsweise eine röntgenkristallographische Strukturanalyse oder multidimensionale Kernresonanz-Spektroskopie. Gute Voraussagen lassen sich mittlerweile auch durch Strukturanalysen mit Hilfe genetischer Algorithmen erzielen.

Weitere Informationen über die Ubiquitin-Superfamilie finden sich beispielsweise in der Veröffentlichung von Larsen et al., 2002. Ergänzend sei auch auf die Veröffentlichung von Buchberger et al., 2001, hingewiesen. Buchberger beschreibt die typische β-Grasp-Faltung als Charakteristikum für ubiquitin-like-proteins mit einer Sekundärstruktur der Organisation beta-betaalpha-beta-beta-alpha-beta, d.h. die Anordnung von fünf beta-Strängen in Form einer "mixedsheet" in der Anordnung 21534. Bemerkenswert ist hierbei, dass UBX keine signifikanten Homologie in der Primärsequenz zu z. B. Ubiquitin aufweist (Buchberger et al., 2001), trotzdem aber - aufgrund seiner dreidimensionalen Struktur, die identisch zu der von z. B. Ubiquitin ist - unter die ubiquitin-like proteins subsummiert wird. In diesem Zusammenhang sei darauf hingewiesen, dass im Ubiquitin die Aminosäuren an Positionen 48 und 49 z. T. ebenfalls als eigenständiger beta-Strang angesehen werden (Vijay-Kumar, 1987). Dieser fünfte Strang, welcher in der Struktur des Ubiquitins nach der Helix lokalisiert wäre und dem "mixed sheet" die Anordnung 21534 gäbe, weist allerdings nur zwei Aminosäuren auf, und es ist durchaus zweifelhaft, diesen aus zwei Aminosäuren bestehenden Strang als beta-Faltblatt-Strang zu bezeichnen. Nach Buchberger et al. (2001) könnten jedoch wie oben angeführt auch Proteine mit der Anordnung 21534 zwanglos unter die Superfamilie der ubiquitin-like-proteins subsummiert werden. Für die vorliegende Erfindung wurde für die Anordnung der beta-Stränge im Ubiquitin die oben näher beschriebene 21543-Definition gewählt.

Die Proteine der genannten Familie und Superfamilie sind in der Regel hochkonserviert. Bspw. weist Ubiquitin nach bisherigen Erkenntnissen in allen Säugern die identische Aminosäuresequenz auf. Ubiquitin aus Hefe weist lediglich eine Abweichung in drei Aminosäuren davon auf. Humanes Ubiquitin bzw. Ubiquitin aus Säugern besteht aus 76 Aminosäuren und hat die eingangs beschriebene Struktur.

Das erfindungsgemäß eingesetzte modifizierte Protein sollte eine mindestens 30 %ige, bevorzugt mindestens 40%ige oder 50%ige, weiterhin bevorzugt mindestens 60%, mindestens 70%, mindestens 80%, mindestens 90%, oder mindestens 95 %ige Übereinstimmung in der Aminosäuresequenz zum Ausgangsprotein, welches modifiziert wird, d. h. zu humanem Ubiquitin, aufweisen, wobei das Protein jedenfalls ein Ubiquitin-artiges Faltungsmotiv, wie es oben eingehend definiert wurde, aufweist.

Gemäß der vorliegenden Erfindung ist das Protein, das zur Herstellung des modifizierten Proteins ausgewählt wird, humanes Ubiquitin. Ist das Einsatzgebiet der erfindungsgemäß hergestellten Proteine bekannt, d.h. soll beispielsweise das modifizierte Protein als Arzneimittel zur Behandlung von Erkrankungen beim Menschen eingesetzt werden, kann bevorzugt ein humanes Protein als zu modifizierendes Ausgangsprotein eingesetzt werden; diese gilt analog für entsprechende Einsatzgebiete.

Humanes bzw. Säuger-Ubiquitin weist 76 Aminosäuren auf. Die Aminosäuren der vier Betastränge, die zu Bildung des antiparallelen beta-Faltblatts beitragen, sind erfindungsgemäß entsprechend der Struktur 1UBQ in der PDB-Datenbank (http://www.rcsb.org/pdb/index.html) die folgenden Aminosäurepositionen:
Erster (aminoterminaler) Strang: 2 bis 7; zweiter beta-Faltblattstrang: 12 bis 16; dritter Strang: 41 bis 45; vierter (carboxyterminaler) Strang: 66 bis 71. Die Lage der Stränge bei Aufsicht auf das Faltblatt (Aminoterminus unten, Carboxyterminus oben) von links nach rechts ist: 2., 1., 4., 3., Strang, wobei die Polypeptidkette zwischen 1. und 4. Strang die alpha-Helix bildet.

Auswahl und Modifikation der zu modifizierenden Aminosäuren:

Ausgehend von entsprechenden Strukturdaten, wie sie z. B. in der Protein Data Bank™ (Berman et al., 2000; http://www.rcsb.org/pdb) frei verfügbar sind, können mittels rechnergestützter Analyse die Positionen solcher Aminosäuren im Ausgangsprotein, z.B. im Ubiquitin-Proteingerüst, lokalisiert werden, deren Seitenketten oberflächenexponiert, d. h. dem Lösungsmittel oder einem potentiellen Bindungspartner zugewandt, sind. Weiterhin können durch rechnergestützte Analyse solche Aminosäuren im Ausgangsprotein, d. h. im Ubiquitin, identifiziert werden, deren zufällige Substitution vermutlich keinen oder nur einen geringen negativen Effekt auf die Stabilität des Proteingerüstes haben könnte.

Diese Informationen können einen ersten Anhaltspunkt für die Eignung jeder einzelnen Aminosäure als Element einer Bindungsstelle darstellen, was dann einer Überprüfung in der Praxis bedarf. Erfindungsgemäß wurden aufgrund ihrer Oberflächenexposition und der Toleranz der Gesamtstruktur gegenüber ihrem zufälligen Austausch die Aminosäuren an den Positionen 2, 4, 6, 62, 63, 64, 65 und 66 im humanen Ubiquitin ausgewählt.

Die genannten Positionen befinden sich in räumlicher Nähe zueinander am Beginn des ersten aminoterminalen beta-Faltblattstrangs (Pos. 2, 4, 6) sowie im Loop (Pos. 62, 63) bzw. am Beginn des carboxyterminalen beta-Faltblattstrangs (Pos. 64, 65, 66) und bilden mit ihren Aminosäureseitenketten einen zusammenhängenden Bereich auf der Oberfläche des Ubiquitins (Abb. 1). Durch zufällige Aminosäure-Substitutionen ("Randomisierung") in dem analysierten Bereich kann so - analog zu der Antigen-Bindungsstelle von Antikörpern - ein hypervariabler oberflächenexponierter Bereich auf der weiterhin intakten Proteinstruktur des Ubiquitins generiert werden.

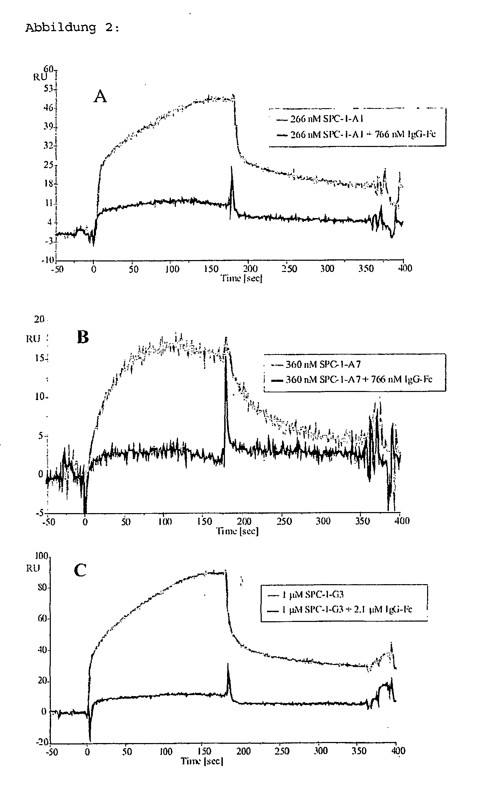

Mit Hilfe der ProSAII-Software ("Protein Structure Analysis"; Proceryon Biosciences, Salzburg) konnte bspw. die Proteinstabilität von 10⁴ Varianten im Vergleich zum Ubiquitin (WT) und einer gleichgroßen Stichprobe von Varianten, bei denen die Reste eines "Kontrollepitopes" (randomisierte Positionen 24, 28, 31, 32, 35, 37, 38, 39) substituiert wurden, bestimmt werden. Dabei weisen ca. 19 % der in silico generierten Varianten, die im Bereich der Bindungsstelle zufällig substituiert worden waren, eine mindestens so hohe Stabilität wie Ubiquitin (WT) auf, während ca. 90 % stabiler als die Träger des "Kontrollepitopes" waren (Abb. 2). Dieses rechnergestützte Ergebnis kann dann als Hinweis für die Auswahl geeigneter Aminosäuren dienen.

Bevorzugt wurden - ausgehend von den verfügbaren Strukturdaten des humanen Ubiquitins - zunächst acht Aminosäurepositionen im Bereich der zu generierenden Bindungsstelle ausgewählt. Durch zufällige Veränderungen der Primärsequenz in diesem Bereich (Random-Mutagenese) und anschließende spezifische Auswahl (Selektion) wurden solche Varianten gewonnen, welche die gewünschte Bindungsaktivität für ein vorgegebenes Hapten oder Antigen bzw. allgemein einen vorbestimmten Bindungspartner aufweisen. Obwohl den erhaltenen modifizierten Proteinen auf diese Weise eine de novo Bindungseigenschaft verliehen wird, bleiben sie hinsichtlich Struktur und proteinchemischen Eigenschaften weitestgehend mit dem Ausgangsprotein identisch. Damit weisen sie Vorteile wie z.B. geringe Größe, hohe Stabilität, kostengünstige Herstellung sowie einfache Modifizierbarkeit, gepaart mit hoher Affinität und Spezifität für einen vorher definierten Liganden auf. Die Eignung des Ubiquitins als Gerüststruktur für die Generierung künstlicher Bindungsproteine war dabei nicht vorhersehbar, da 1.) die Toleranz des Gerüsts gegenüber den umfangreichen Aminosäure-Austauschen aufgrund der geringen Größe des Ubiquitins nicht zu erwarten war und 2.) die Funktionalität der künstlichen Bindungsstelle unter Einbeziehung des als starr und unflexibel angesehenen beta-Faltblatts nicht von vorneherein gegeben scheint.

Nachfolgend werden auch auf gamma-Kristallin basierte Polypeptidmoleküle beschrieben (nicht erfindungsgemäß).

Gamma-Kristalline, eine Klasse der Kristalline bei Vertebraten, sind monomere Proteine mit einer Molekularmasse von etwa 22 kDa. Das Hauptstrukturmotiv der gamma-Kristalline ist das antiparallele Beta-Faltblatt (Hazes und Hol, 1992, Richardson et al., 1992, Hemmingsen et al., 1994). Gamma-Kristalline bestehen aus zwei sehr ähnlichen globulären Domänen, einer N- und einer C-terminalen Domäne, die miteinander durch ein V-förmiges Linkerpeptid verbunden sind. Das für gamma-Kristalline charakteristische Faltungsmuster ("Greek Key-Motiv, Slingsby, 1985, Wistow und Piatigorsky, 1988) ist höchstwahrscheinlich die Ursache für die beträchtliche Thermostabilität sowie die Stabilität gegenüber Denaturierungsmitteln (Mandal et al., 1987).

Gamma-II-Kristallin weist im normal gefalteten Zustand keinerlei Bindungseigenschaften auf. Die Veränderung (Mutagenese) einer ausgewählten, lösungsmittelexponierten Region dieses Proteins, die aus dem Strukturmotiv des Beta-Faltblattes besteht, führte überraschenderweise zur Veränderung der Oberflächenstruktur und dem Ladungsmuster des Proteins und somit zur Herstellung neuer Bindungseigenschaften. Dabei wurden nur Bereiche oder Aminosäurepositionen ausgewählt, die nicht maßgeblich an der Strukturerhaltung des Proteins beteiligt sind. Die Mutagenese eines kleinen Beta-Faltblatt-Proteins (Riddle et al., 1997) hat gezeigt, daß Proteine trotz erheblicher Sequenzänderungen zu einem hohen Prozentsatz korrekt die native Struktur des Beta-Faltblatts ausbilden können.

Bei dem hier beschriebenen Verfahren wird an einem Protein ohne jegliche Bindungseigenschaften in dem starren Bereich des Beta-Faltblatts eine gezielte Mutagenese durchgeführt. Dadurch wurde unerwarteterweise ein Protein mit erheblicher Stabilität und mit spezifischen Bindungseigenschaften, vergleichbar mit Antikörpermolekülen, erzeugt.

Als geeignetes System für die Isolierung mutagenisierter Beta-Faltblatt-Proteine mit neu entstandenen Bindungseigenschaften dient das *Phage Display-System.* Das System ermöglicht ein sehr effizientes Screening eines großen Repertoires an Proteinvarianten auf spezifische Bindungseigenschaften (Smith, 1985). Dabei wird jeweils eine Proteinvariante auf der Oberfläche eines filamentösen Phagen dargestellt und kann mit den Zielmolekülen, die an einer Festphase immobilisiert sind, in Wechselwirkung treten. An das Zielmolekül bindende Proteine können durch Elution der Phagen erhalten werden. Nach Isolierung der Phagen-DNA kann die DNA-Sequenz der spezifisch bindenden Proteinvarianten bestimmt werden. Neben dem *Phage* Display-Systems können auch andere Selektions-Systeme, wie z. B. das Bakterien-Display (Stahl und Uhlen, 1997) oder das Ribosomen-Display (Hanes et al., 1997), Anwendung finden.

Mit dem beschriebenen Vorgehen gelingt es überraschenderweise, beispielsweise das sehr stabile Beta-Faltblatt-Protein Gamma-II-Kristallin durch gezielte ortsspezifische Mutagenese im Beta-Faltblatt an der Oberfläche so zu verändern, daß aus dem nicht-bindenden Protein ein Protein mit spezifischen Bindungseigenschaften entsteht. Durch eine Randomisierung von acht Aminosäurepositionen erfolgt damit erstmals eine Mutagenese in einem Gerüstmolekül innerhalb eines relativ starren Bereich des Proteins. Somit wird aus dem Beta-Faltblatt-Protein gamma-II-Kristallin eine bezüglich ihrer spezifischen Bindungseigenschaften "antikörperähnliche" Proteinspezies hergestellt. Gamma-II-Kristallin oder andere kleine, stabile Beta-Faltblatt-Proteine können mit dem beschriebenen Verfahren generell als neuartige Gerüstmoleküle für das Design neuartiger Bindungseigenschaften verwendet werden. Die modellierten Beta-Faltblatt-Proteine können z.B. rekombinante Antikörper in verschiedenen Applikationen ersetzen.

Weitere und eingehendere Informationen hierzu finden sich in der WO 01/04144, auf die hier vollinhaltlich Bezug genommen wird.

Der Begriff "Ligand", wie hierin definiert bedeutet eine Substanz, die von einem auf Ubiquitin basierenden Polypeptidmolekül spezifisch gebunden wird.

Als solche Bindungspartner, den sogenannten Liganden, werden erfindungsgemäß (Poly)peptide und Proteine (z.B. Immunglobuline und Immunglobulinderivate, Proteine, die aus Blutplasma gewonnen werden können, Blutgerinnungsfaktoren- und -inhibitoren, Wachstumsfaktoren, Interleukine, Cytokine, Rezeptorproteine, virale Proteine und Oberflächenzellmarker, wie CD14, CD25, CD34), Peptide (z.B. Affinitätsanhängsel, wie S-Tag, T7-Tag, His-Tag, Strep-Tag, Myc-Tag, FLAG-Tag und Peptide viralen Ursprungs), eingesetzt. Siehe hierzu auch die bevorzugten Ausführungsformen, die unten dargestellt sind.

Der Begriff "funktioneller Bestandteil", wie hierin verwendet, definiert den zweiten Bestandteil des Konjugates, der kovalent an das auf Ubiquitin basierende Polypeptidmolekül bindet. Mit dem Ausdruck "funktionell" soll zum Ausdruck kommen, dass es sich um einen zum Einsatz in der Diagnose, Therapie, Chromatographie und Analytik geeigneten und möglicherweise bereits bekannten Bestandteil handelt. Allgemein ausgedrückt ist ein "funktioneller Bestandteil" als jegliche Komponente mit messbaren Eigenschaften definiert, z.B. eine Enzymaktivität, eine spektroskopisch messbare Eigenschaft oder eine toxische Eigenschaft. Die Struktur und Funktion des funktionellen Bestandteils im vorliegenden Konjugat ist davon abgesehen nicht beschränkt. Das einzige Erfordernis besteht darin, dass nach der kovalenten Bindung von Polypeptid-Molekül und funktionellem Bestandteil die Funktionalität aller Bestandteile erhalten bleibt. Diese Funktionalität ist im Falle des Polypeptid-Moleküls die Bindungsfähigkeit an den speziellen Liganden, im Falle des funktionellen Bestandteils beispielsweise seine Wirkung als Farbstoff.

Erfindungsgemäß können einer oder auch mehrere, z.B. zwei, funktionelle Bestandteile an ein Polypeptidmolekül (wie oben definiert) gebunden sein. Um die spezifische Bindung der Bestandteile an das Polypeptidmolekül zu erreichen, kann beispielsweise einer der Bestandteile zur spezifischen Bindung an einen Lysin-Rest, der andere an einen Cystein-Rest im Polypeptidmolekül ausgebildet sein. Beispiele für zwei derartige Bestandteile sind Fluoreszenzfarbstoffe, bei denen einer als Fluoreszenzdonor, der andere als Fluoreszenzakzeptor dient.

Details über die Art der Bindung der Bestandteile aneinander sowie der bevorzugt in Frage kommenden Bindungsbestandteile werden nachstehend erklärt.

Der Nachweis der Bindungsaktivität des Polypeptidmoleküls an den Liganden und der Aktivität des Kopplungspartners (funktioneller Bestandteil) stellt -wie oben angesprochen- einen wichtigen Gesichtspunkt dar. Der Nachweis der Bindungsaktivität an den Liganden kann dabei durch verschiedene Methoden erfolgen. In der ELISA-Technik wird die Bindung mittels Antikörper-Peroxidase-Konjugate nachgewiesen, dagegen nutzt das Biacore-System das Phänomen der Oberflächenplasmonresonanz zum Nachweis aus. Weitere Techniken, wie die Fluoreszenztitration, die Fluoreszenzpolarisation oder die Fluoreszenz-Korrelationsspektroskopie (FCS), beruhen auf den fluorophoren Eigenschaften des Affilin™. Beim Nachweis der Aktivität des Kopplungspartners, dem so gennannten weiteren funktionellen Bestandteil, sind dessen bekannte Eigenschaften ausschlaggebend. So werden chromophore oder fluorophore Moleküle, wie Fluoreszenzfarbstoffe oder Phycoerythrin, über ihre spektralen Eigenschaften analysiert. Enzyme, wie Peroxidase oder alkalische Phosphatase, werden über ihren katalytischen Umsatz von Modellsubstraten getestet. Andere Moleküle, wie Toxine, können direkt in Zellkultur-Experimenten auf ihre biologische Aktivität getestet werden. Bei Radioisotopen kann die Radioaktivität mit Hilfe von entsprechenden Countern gemessen werden. Für eine Reihe weiterer Moleküle, wie Zucker und Nukleinsäuren gibt es kommerziell erhältliche Nachweisreagenzien. Gemäß einer Ausführungsform beruht - wie oben angesprochen - die gegenüber dem Wildtyp-Polypeptid neu generierte oder veränderte Bindungseigenschaft auf einem oder mehreren Aminosäureaustauchen in einer oberflächenexponierten Region eines β-Faltblattes des Ubiquitin-Polypeptidmoleküls (I). Hierbei wird eine Zahl von ungefähr 6-10, bevorzugt 8 Aminosäuren pro Polypeptidmolekül (I) ausgetauscht.

Es ist besonders wichtig zu erwähnen, dass die Kopplung von (I) an (II) bevorzugt in einem Bereich außerhalb der oberflächenexponierten Region des β-Faltblattes des Polypeptidmoleküls (I) erfolgt, die zur spezifischen Bindung an einen Liganden vorgesehen ist. Es hat sich herausgestellt, dass hierdurch eine wichtige Anforderung an das erfindungsgemäße Konjugat, nämlich die Aufrechterhaltung der Funktionalität aller Bestandteile, ermöglicht wird.

Als Beispiel sei hier eine Kopplung an Lys 29, 33 von Ubiquitin genannt, das sich ausserhalb der Bindungsfläche für den Liganden befindet, genauer sogar ausserhalb des für die Bindung an den Liganden verantwortlichen Beta-Faltblattes (Lys 29, 33 befinden sich in der Alpha-Helix). Eine Kopplung über diese Aminosäureseitenkette fernab der Bindungsstelle an den Liganden führt zu keiner Beeinträchtigung der Funktionalität der Bestandteile des Konjugates.

In diesem Sinne ist es besonders bevorzugt, wenn die Kopplung von (I) an (II) in einem Bereich außerhalb des β-Faltblatts des Polypeptidmoleküls (I) erfolgt, das die neu generierten oder veränderten Bindungseigenschaft zur spezifischen Bindung an einen Liganden aufweist. Dieser Bereich ist im obigen Fall z.B. die Alpha-Helix des Ubiquitin-Moleküls. Besonders deutlich wird dies aus Abb. 1, worin die N-terminale Domäne (Beta-Faltblatt) des Kristallins eine neu generierte Bindungsfläche für den Liganden aufweist, wobei sich die im C-terminalen Anteil befindlichen Lysin-Reste (hervorgehoben) zur Kopplung an den funktionellen Bestandteil (II) eignen.

Gemäß einer Ausführungsform findet die Kopplung bzw. Verknüpfung von (I) an (II) über Aminosäurereste von (I) statt. Mit anderen Worten erfolgt hier eine Kopplung über Aminosäurereste, die bereits per se im Polypeptidmolekül (I) vorhanden waren.

Die Kopplung erfolgt hierbei ortsspezifisch oder selektiv ungerichtet über Cystein- oder Lysinseitenketten in (I). Der Begriff "ortsspezifisch" bedeutet hierbei, dass ein Cystein- oder Lysin an genau definierter Stelle im Molekül (I) vorhanden ist oder eingebracht wird, um eine exakt vorherbestimmte Bindungsstelle zu definieren. "Selektiv ungerichtet" bedeutet, dass mehrere solcher Reste vorhanden sind, wobei die Bindung an diese Reste zwar selektiv erfolgt, aufgrund ihrer Anzahl jedoch auch einem gewissen Zufallsfaktor unterliegt.

Zur Kopplung der beiden Bestandteile des erfindungsgemäßen Konjugates, d.h. deren kovalenter Verknüpfung, sei Folgendes ausgeführt:
Proteine enthalten eine Vielzahl funktioneller Gruppen, über die eine Kopplung an andere Moleküle erreicht werden kann. Konkrete Beispiele hierfür finden sich unten. Durch die kovalente Verknüpfung der Partner, z.B. über ein geeignetes Quervernetzungsreagenz, ist es so möglich, unterschiedliche Aktivitäten in einem Molekül zusammenzuführen. Beispielhaft seien hier Antikörper genannt, die häufig mit Farbstoffmolekülen gekoppelt werden und auf diese Weise leicht detektierbare Nachweisreagenzien liefern, welche anschließend in der Diagnostik eingesetzt werden.

Analog werden Antikörper auch mit anderen Proteinen, bevorzugt sog. Reporterenzymen wie Alkalische Phosphatase oder Peroxidase, konjugiert. Diese Reporterenzyme setzen ein Substrat um, wodurch ein durch Absorption, Fluoreszenz oder Lumineszenz detektierbares Signal erhalten wird.

Durch eine Auswahl von geeigneten Quervernetzungsreagenzien lassen sich Proteine, d.h. auch die erfindungsgemäßen Polypeptid-Moleküle, auch an weitere funktionelle Bestandteile aus vielen anderen Substanzklassen koppeln: So kommen dafür eine Vielzahl organischer und auch anorganischer Polymere, Proteine, Peptide, Nukleinsäuren, Lipide, Zucker und auch niedermolekulare Substanzen in Frage. Bevorzugte Kopplungspartner aus der Klasse der Polymere sind z.B. Dextran, Polymethacrylat, Sepharose, Agarose, Polystyren, Polyvinyl, Kieselgel, Cellulose oder Polyethylenglykol (PEG). Letztere Modifikation wird beispielsweise zur Modulation der pharmakokinetischen Eigenschaften von Biotherapeutika eingesetzt. Eine große Auswahl von PEG-Derivaten, die bereits für die Kopplung geeignete voraktivierte, funktionelle Gruppen enthalten sind kommerziell erhältlich (Nektar Therapeutics). Die anderen genannten Polymer-Materialien dienen als Trägersubstanzen bei vielen biochemischen, biotechnologischen und diagnostischen Verfahren. Hervorzuheben sind hier z.B. chromatographische Anwendungen, insbesondere Affinitätschromatographie, wo diese Polymersubstanzen als Gelmatrix Verwendung finden. Dazu wird die Oberfläche von Polymerkügelchen mit geeigneten chemischen Gruppen funktionalisiert und aktiviert, so daß die Kopplung, z.B. eines Proteins, mit einer gewünschten Aktivität möglich ist. Auf diese Weise lassen sich auch Bindeproteine mit einer Spezifität für einen bestimmten Liganden immobilisieren und die resultierende Gelmatrix kann dann für die effiziente und schnelle Anreicherung des Liganden aus einem komplexen Stoffgemisch eingesetzt werden. Diese Anreicherung kann dabei entweder säulenchromatographisch erfolgen oder aber auch auf der Oberfläche von Filtern. Weiterhin können für die Affinitätsanreicherung auch vorteilhaft solche Polymerkügelchen verwendet werden, die magnetische Eigenschaften besitzen (magnetic *BEADS*) und so effizient aus einem Gemisch abgetrennt werden können.

Eine wichtiges Anwendungsbeispiel ist die großtechnische Reinigung von Antikörpern für therapeutische Zwecke aus dem Überstand von eukaryotischen Zellkulturen, die mittels Affinitätschromatographie an matrixgekoppeltem Protein A, einem bakteriellen Protein mit einer intrinsischen Affinität für Antikörpermoleküle, erfolgt.

Bei der Kopplung von zwei Proteinkomponenten gibt es über die oben erwähnten Antikörper-Reporterenzymfusionen hinaus zahlreiche Anwendungsbeispiele. So können im einfachsten Fall durch Kopplung zweier oder mehrerer identischer Proteinmoleküle Homo-Dimere oder -Multimere erzeugt werden.

Im Fall von Bindeproteinen lassen sich durch eine Multimerisierung Aviditätseffekte erzeugen, d.h. die Affinität des Multimeres für die Zielsubstanz ist signifikant erhöht gegenüber der Affinität des monomeren Bindeproteins. Durch die Kopplung zweier Bindeproteine mit Spezifität für unterschiedliche Zielsubstanzen lassen sich hingegen sog. bispezifische Bindemoleküle gewinnen, die z.B. wie ein Adapter genutzt werden können, um die jeweiligen Zielsubstanzen in räumliche Nähe zu bringen.

Auch Proteine, die toxische Eigenschaften für lebende Zellen besitzen, sog. Toxine (z.B. Ricin, Choleratoxin, Pseudomonas-Exotoxin u.a.) stellen interessante Kopplungspartner für Antikörper oder andere Bindemoleküle dar. Nach der Kopplung kann dieses bifunktionelle Konjugat über das Bindeprotein an eine spezifische Zielsubstanz z.B. auf der Oberfläche einer Tumorzelle selektiv andocken und im Anschluß daran wird die betroffene Zelle durch die cytotoxische Aktivität zerstört.

Weitere relevante Kopplungspartner mit Protein-Natur sind Protein-Chromophore wie z.B. GFP und Derivate oder pigmenthaltige Proteine wie Phycoerythrine. Die resultierenden, chromophoren oder fluoreszenten Kopplungsprodukte sind wiederum als gut detektierbare Reportersubstanzen wertvolle Werkzeuge für die Forschung oder Diagnostik.

Chromophore oder fluoreszente Proteinkonjugate sind aber auch wie bereits oben erwähnt durch die Kopplung niedermolekularer Farbstoffmoleküle zugänglich. Eine Vielzahl geeigneter Farbstoffmoleküle mit quervernetzungsaktiven Gruppen ist z.B. kommerziell bei Fa. Invitrogen erhältlich. Weitere niedermolekulare Kopplungspartner, die Protein-Konjugate für den Einsatz in Diagnostik und Therapie liefern, sind z.B. Biotin, Digoxigenin, Schwermetallderivate, Chelatbildner, Radioisotope, cytotoxische Substanzen und Antibiotika.

Bei den in Proteinen und auch in den erfindungsgemäßen Polypeptid-Molekülen sowie funktionellen Bestandteilen enthaltenen funktionellen Gruppen, die für eine Kopplung geeignet sind, handelt es sich vor allem um Amino-, Carboxy-, Hydroxy- und Sulfhydryl-Gruppen, aber auch die Phenolfunktion von Tyrosin und die aromatischen Ringsysteme können als Angriffspunkte für Kopplungsreagenzien dienen. Für eine Kopplung geeignete Aminosäurereste, zeichnen sich durch besondere Eigenschaften aus: Zum einen müssen sie eine reaktive Seitenkette besitzen, die für das Kopplungsreagenz zugänglich sein muß. Idealerweise befindet sich diese funktionelle Gruppe an der Proteinoberfläche und ist lösemittelexponiert. Weiterhin sollte die Modifikation dieses Restes nicht mit der Funktion des Proteins interferieren. Bevorzugt liegen die zu modifizierenden Reste in signifikantem Abstand vom aktiven Zentrum eines Enzyms, oder der Bindefläche des erfindungsgemäßen Polypeptid-Moleküls; zudem sollten in diesen Bereichen auch keine Aminosäurereste des gleichen Typs vorkommen, die nach Modifikation zu einem Funktionsverlust des Proteins führen könnten. Vorteilhaft sind daher auch Aminosäurereste, die selten im betreffenden Protein vorkommen.

Wie bereits oben angesprochen kommen für das auf Ubiquitin basierende Polypeptidmolekül (I) die Kopplung über die Lysinreste 29 und 33 des Ubiquitinmoleküls bevorzugt in Betracht. Etwas weniger bevorzugt, aber dennoch möglich, ist eine Kopplung über die Lysinreste 11 und 48, die jeweils von der Bindungsfläche an den Liganden weniger weit entfernt sind als die Reste 29 und 33.

Im Falle eines auf gamma-Kristallin basierenden Polypeptidmoleküls (I) (nicht erfindungsgemäß) ist die Peptid-Domäne mit der gegenüber dem Wildtyp-Polypeptid neu generierten oder veränderten Bindungseigenschaft zur spezifischen Bindung an einen Liganden die N-terminale Domäne und die Kopplung von (I) an (II) erfolgt über die C-terminale Domäne. Dies gilt gleichermaßen für die Möglichkeit einer C-terminalen Peptidfusion, die bevorzugt Cystein enthält, wie auch für in der C-terminalen Domäne vorhandene Aminosäureseitenketten wie Lysin, die für eine Kopplung an den funktionellen Bestandteil zur Verfügung stehen. Bevorzugt eingesetzt werden hier die Reste 91 und 163 von Gamma-Kristallin (siehe Abb. 1).

Unter den proteinogenen Aminosäuren hervorzuheben sind dabei vor allem Lysin, das in seiner Seitenkette eine epsilon-Aminogruppe besitzt, sowie Cystein mit seiner Sulfhydryl-Funktion. Diese funktionellen Gruppen weisen eine besondere Reaktivität auf und sind daher gut geeignet als Partner für eine spezifische Kopplung eines erfindungsgemäßen Polypeptid-Moleküls an einen funktionellen Bestandteil. So sind in der Literatur zahlreiche Reagenzien beschrieben, die spezifisch nur mit Sulfhydrylgruppen reagieren und daher erfindungsgemäß eingesetzt werden können, z.B. Maleinimid, Iodacetat, Hydroxymercuribenzoat, Ellmans Reagenz u.a. Weitere Beispiele finden sich in einschlägigen Lehrbüchern wie Voet & Voet (1995) oder Lottspeich & Zorbas (1998).

Dort beschrieben sind auch zahlreiche Lysin-spezifische Seitenkettenreagenzien wie z.B. Säureanhydride (Acetanhydrid, N-Hydroxysuccinimid u.a., die ebenfalls in der vorliegenden Erfindung Anwendung finden). Lysine weisen neben ihrer Reaktivität noch weitere vorteilhafte Eigenschaften für eine Kopplung auf: Aufgrund der geladenen Seitenkette befindet sich diese zumeist an der Oberfläche des Proteins, d.h. sie ist dem Lösemittel, in biologischen Systemen zumeist Wasser, zugänglich. Diese Zugänglichkeit ist auch für das Kopplungsreagenz notwendig und sollte somit gegeben sein.

Freie, oberflächenexponierte Cysteine kommen in Proteinen vergleichsweise selten vor, bei extracellulären Proteinen sind diese Cysteine zumeist in Disulfidbrücken eingebunden, die oft auch Dimerwechselwirkungen stabilisieren. Disulfidbrücken lassen sich allerdings auch reduktiv spalten, so daß enthaltene Cysteine dann einer Modifikation zugänglich werden. Sind keine, für ein Kopplungsreagenz zugängliche Cysteinreste in einem erfindungsgemäßen Polypeptidmolekül enthalten, ist es möglich, solche Reste an geeigneter Stelle durch Mutagenese einzuführen. Dabei ist die Kenntnis der Raumstruktur des Proteins von Vorteil, da hierdurch die Vorhersage von für die Kopplung günstigen, oberflächenexponierten Aminosäurepositionen erleichtert wird. Sind aber mehrere Cysteinreste in einem Protein vorhanden, die eine ortsspezifische Kopplung (an einen definierten Cysteinrest, s.o.) unmöglich machen, können diese mittels ortsgerichteter Mutagenese eliminiert werden. Bevorzugt erfolgt dabei der Austausch gegen einen Serinrest, der dem Cysteinrest ähnliche Eigenschaften besitzt.

Oftmals ist es aber nicht möglich, durch Mutagenese in der interessierenden Proteinsequenz für die Kopplung geeignete Aminosäurereste zu erhalten. Toleriert das Protein allerdings Insertionen von Aminosäureresten oder Fusionen am N- oder C-Terminus, können dort auf genetischer Ebene gezielt Peptidsequenzen eingeführt werden, die für die Kopplung geeignete Aminosäurereste enthalten.

Insofern erfolgt gemäß einer weiteren Ausführungsform die Kopplung von (I) an (II) über Aminosäurereste in einer zusätzlichen terminalen Peptidfusion an (I).

Eine Überprüfung der Zugänglichkeit dieser terminalen Peptidfusionen kann z.B. durch die Verwendung eines seitenkettenspezifischen Kopplungsreagenz mit chromophoren Eigenschaften erfolgen, z.B. Ellmanns Reagenz bei Cystein (vgl. auch Beispiele). Die Zugänglichkeit dieser terminalen Peptidfusionen kann u.a. über ihre Länge gesteuert werden. Eine Erhöhung der Zugänglichkeit der zu koppelnden Aminosäureseitenkette ist z.B. durch die Insertion von sog. Spacern d.h. Abstandshaltern zwischen der zu koppelnden Aminosäureseitenkette und dem Protein möglich, hierbei handelt es sich im Falle von Peptidfusionen um zusätzliche, inerte Aminosäurereste. Dafür besonders geeignet sind Glycin- und Serinreste, da sie nur eine geringe Größe aufweisen und dadurch eine sehr flexible Struktur einnehmen können.

Abhängig von der Anzahl der für das Kopplungsreagenz zugänglichen funktionellen Gruppen im Protein und seiner Spezifität kann man unterschiedliche Kopplungstypen unterscheiden. Beispielsweise reagieren bei Verwendung eines Cystein-spezifischen Kopplungsreagenz selektiv nur Cystein-Reste; sind aber mehrere z.B. Cystein-Reste vorhanden ist der genaue Ort der Kopplung nicht vorhersagbar, man spricht dann von einer selektiven, aber ungerichteten Kopplung. Existiert hingegen nur ein für das Kopplungsreagenz zugängliches Cystein, erfolgt die Kopplung ortsspezifisch.

Kommerziell ist eine Vielzahl von geeigneten Kopplungsreagenzien erhältlich (z.B. bei Fa. Pierce). Eine spezielle Form von Kopplungsreagenzien, die erfindungsgemäß eingesetzt werden können, bezeichnet man auch als Quervernetzer (engl. *cross-linker*) oder einfach als Linker (Herrmann & Morse, 1973; Takamiya et al., 1975; Reichlin, 1980).

Ein Linker ist definiert als eine Substanz, die zwei (oder mehr) Moleküle durch eine kovalente Bindung verknüpft. Linker enthalten zwei (oder mehr) reaktive (aktivierte), funktionelle Gruppen, deren räumlicher Abstand über weitere, sie verbindende chemische Gruppen gesteuert werden kann. Linker, die identische funktionelle Gruppen aufweisen bezeichnet man als homobifunktional, in Abgrenzung von hetero-bifunktionalen Linkern, die unterschiedliche funktionelle Gruppen besitzen. Durch die geeignete Auswahl von Linkersubstanzen ist es daher möglich auch vollkommen unterschiedliche Substanzklassen miteinander zu verknüpfen.

Als Beispiel für einen erfindungsgemäß einsetzbaren Linker sei auf den C-terminalen Anteil von SPC-1-A7-Cys verwiesen, siehe Tabelle 2 unten.

Ein Spezialfall der Kopplung von Proteinen kommt allerdings auch ohne aktivierte Linker aus: Die Ausbildung von Disulfidbrücken z.B. bei der Dimerisierung von Proteinen. Unter oxidierenden Bedingungen besitzen Sulfhydrylreste eine ausreichend hohe Reaktivität, um ein Disulfid auszubilden, d.h. eine kovalente Bindung zwischen den beiden Schwefelatomen.

Eine Kopplungsreaktion kann erfindungsgemäß entweder als Ein-Schritt-Reaktion, oder aber in mehreren Schritten ablaufen. Bei einer einfachen Dimerisierung zweier identischer Moleküle, die jeweils nur einen reaktiven Rest tragen, reicht es aus, die Kopplungskomponenten mit einem homo-bifunktionalen Linker zu inkubieren, um ein definiertes Konjugat zu erhalten. Bei unterschiedlichen Kopplungspartnern, welche auch unterschiedliche reaktive Gruppen tragen, ist die Ein-Schritt-Reaktion nur mit einem entsprechenden hetero-bifunktionalen Linker möglich.

Alternativ kann die Kopplung zwischen unterschiedlichen Kopplungspartnern (Reaktanden) mit identischen reaktiven Gruppen aber auch in einem Mehrschritt-Prozeß ablaufen. Dazu wird zunächst nur ein Reaktionspartner zumeist mit einem Überschuß des Linkers inkubiert und das entstandene monovalente Reaktand-Linker-Konjugat isoliert, bevor es mit dem zweiten Reaktanden über die noch freie funktionelle Gruppe des Linkers verknüpft wird. Eine Vielzahl chemischer Substanzen, die in der Biochemie und Biotechnologie Verwendung finden sind in sog. aktivierter Form, d.h. bereits mit einem noch reaktiven Linker verknüpft, kommerziell erhältlich (Fa. Pierce, Fa. Invitrogen).

Der Kopplungsgrad, d.h. das relativeVerhältnis der einzelnen Komponenten im Konjugat, ist bei Vorliegen mehrerer reaktiver Gruppen in einem gewissen Ausmaß durch die Stöchiometrie der eingesetzten Reaktanden steuerbar. Eine definierte Mehrfachkopplung mit unterschiedlichen Kopplungspartnern ist durch eine sequentielle Kopplung oder die Verwendung unterschiedlicher Kopplungschemie möglich, z.B. durch Koppeln des ersten funktionellen Bestandteils an Cysteine, während der funktionelle Bestandteil 2 an Lysine des betreffenden Polypeptids angehängt wird. Ein typisches Beispiel ist das Anhängen von Fluoreszenzdonor-/-akzeptor-Paaren für FRET-Messungen. Dafür geeignete Kombinationen von Farbstoffmolekülen sind bei der Fa. Invitrogen erhältlich.

Unabhängig vom gewählten Kopplungsmechanismus, der zum erfindungsgemäßen Konjugat führt, sei nochmals darauf hingewiesen, dass die Funktionsfähigkeit der vorliegenden Konjugate so nicht zu erwarten und überraschend war. Insofern wird auch auf die vorstehenden Ausführungen verwiesen.

Darüberhinaus sei erwähnt, das für den Fachmann nicht zu erwarten war, dass Konjugate wie in der vorliegenden Erfindung offenbart erzeugt werden können, wobei die volle Funktionalität der Einzelbestandteile erhalten bleibt oder sogar gesteigert wird.

Die Größenverhältnisse zwischen den erfindungsgemäßen Polypeptid-Molekülen einerseits und den funktionellen Bestandteilen andererseits sind sehr unterschiedlich. Teilweise sind die zu bindenden Moleküle, beispielsweise Phycoerythrin, 10 - 12 mal größer als die Polypeptid-Moleküle, teilweise, wie der Fluroreszenzfarbstoff Oyster, aber auch kleiner. Erstaunlicherweise bleiben, insbesondere bei den viel größeren gebundenen Molekülen, sowohl die Struktur des Polypeptidmoleküls als auch die Bindungsaffinität an den Liganden erhalten. Dies ist so nicht zu erwarten gewesen.

Wie bereits oben angesprochen, liegen die Seitenketten zur Kopplung an den funktionellen Bestandteil vorzugsweise außerhalb der Bindungsfläche von (I) mit dem Liganden, um die Funktionalität der Bindung an den Liganden nicht zu stören.

Gemäß einer weiteren bevorzugten Ausführungsform enthält - wie oben angesprochen - die terminale Peptidfusion an (I) einen oder mehrere Cysteinreste oder einen oder mehrere Lysinreste, wobei diese Aminosäurereste vorzugsweise nicht an der Wechselwirkung von (I) mit dem Liganden beteiligt sind.

Der funktionelle Bestandteil (II) ist aus der Gruppe ausgewählt ist, die aus Polypeptiden und Proteinen, organischen Polymeren, Zuckern, niedermolekularen Substanzen, Peptiden, sowie Derivaten dieser Substanzen, besteht. Zu den Bindungsprinzipien und Kopplungsreagenzien, siehe auch die obigen Ausführungen.

Gemäß einer bevorzugten Ausführungsform ist der funktionelle Bestandteil (II) ein Peptid, Polypeptid oder ein Protein, bevorzugt ein Protein-Chromophor, ein Enzym, ein Immunglobulin, ein Immunglobulin-Derivat, ein Toxin oder ein Polypeptid gemäß I.

Falls der funktionelle Bestandteil (II) ein Polymer ist, wird er bevorzugt aus Dextran, Polymethacrylat, Sepharose, Agarose, Polyvinyl, Polystyren, Kieselgel, Cellulose oder Polyethylenglykol, oder einem Polymerderivat ausgewählt.

Falls es sich bei dem funktionellen Bestandteil (II) um eine niedermolekulare Substanz handelt, ist diese bevorzugt ein Farbstoff, Biotin, Digoxigenin, ein Schwermetall, ein Chelatbildner, ein Radioisotop, ein Antibiotikum oder eine cytotoxische Substanz.

Der an den Bestandteil (I) spezifisch bindende Ligand ist aus der Gruppe ausgewählt, die aus Polypeptiden, Peptiden und Proteinen besteht.

Falls es sich hierbei um ein Polypeptid oder ein Protein handelt, werden vorzugsweise Immunglobuline und Immunglobulinderivate, aus Blutplasma gewonnene Proteine, Blutgerinnungsfaktoren- und -inhibitoren, Wachstumsfaktoren, Interleukine, Cytokine, Rezeptorproteine, virale Proteine und Oberflächenzellmarker, bevorzugt CD14, CD25, CD34 eingesetzt.

Falls der Ligand ein Peptid ist, ist er bevorzugt ein Affinitätsanhängsel, vorzugsweise S-Tag, T7-Tag, His-Tag, Strep-Tag, Myc-Tag, oder FLAG-Tag, oder ein Peptid viralen Ursprungs.

Gemäß einer bevorzugten Ausführungsform ist der Bestandteil (II) des erfindungsgemäßen Konjugates ein oder mehrere auf Ubiquitin basierende Polypeptide, die mit (I) identisch sind, und mit diesem kovalent verknüpft sind, wodurch eine Steigerung der Affinität zu dem Liganden von (I) über Aviditätseffekte erreicht wird. Für eine ausführliche Erklärung wird auf die obigen Erläuterungen verwiesen.

Weiterhin ist der Bestandteil (II) vorzugsweise ein Polypeptid, Protein oder Polymer, an das Bestandteil (I) mehrfach kovalent verknüpft ist, wodurch eine Steigerung der Affinität zu dem Liganden von (I) über Aviditätseffekte erreicht wird. Alternativ ist der Bestandteil (II) ein Polypeptid oder Polymer, das nach kovalenter Verknüpfung mit Bestandteil (I) kovalente oder nicht kovalente Bindung mit weiteren Konjugaten dieser Art eingeht, wodurch eine Steigerung der Affinität zu dem Liganden von (I) über Aviditätseffekte erreicht wird.

Gemäß einer bevorzugten Ausführungsform ist der Bestandteil (I) eines der Moleküle SPU11-3-A1 (SEQ ID NO: 12 und 13).

Die Erfindung beschreibt nicht nur die exakten Nukleinsäuresequenzen, sondern auch Varianten hiervon. "Varianten" sind erfindungsgemäß insbesondere solche Nukleinsäuren, bei denen eine oder mehrere Substitutionen, Insertionen und/oder Deletionen im Vergleich zu den in den SEQ ID NO definierten Nukleinsäuren vorliegen. Bei diesen fehlen vorzugsweise zumindest 1, aber auch 2, 3, 4 oder mehr Nukleotide an einem oder beiden Enden der Nukleinsäuren oder auch im Inneren der Nukleinsäuren oder sind durch andere Nukleotide ersetzt.

Die Nukleinsäuren der vorliegenden Erfindung umfassen also auch Nukleinsäuren, die Sequenzen aufweisen, die im Wesentlichen zu den Nukleinsäuren der jeweiligen SEQ ID NO äquivalent sind. Erfindungsgemäße Nukleinsäuren können z.B. zumindest ungefähr 80%, typischerweise zumindest ungefähr 90% oder 95% Sequenzidentität zu den Nukleinsäuren der SEQ ID NO aufweisen.

Der Begriff "Nukleinsäuresequenz" betrifft ein Heteropolymer von Nukleotiden oder die Sequenz dieser Nukleotide. Der Begriff "Nukleinsäure", wie hierin verwendet, umfasst sowohl RNA, DNA, einschließlich cDNA, genomische DNA und synthetische (bsw. chemisch synthetisierte) als auch an andere Polymere gebundene Basen wie PNA.

Die Erfindung umfaßt auch solche Varianten, die mit den erfindungsgemäßen Nukleinsäuren unter moderat stringenten Bedingungen hybridisieren.

Unter stringenten Hybridisierungs- und Waschbedingungen versteht man im allgemeinen die Reaktionsbedingungen, unter denen nur noch Duplexmoleküle zwischen Oligonukleotiden und gewünschten Zielmolekülen (perfekte Hybride) entstehen bzw. nur noch der gewünschte Zielorganismus nachgewiesen wird. Unter stringenten Hybridisierungsbedingungen werden dabei insbesondere 0,2 x SSC (0,03 M NaCl, 0,003 M Natriumcitrat, pH 7) bei 65°C verstanden. Bei kürzeren Fragmenten, beispielsweise Oligonukleotiden aus bis zu 20 Nukleotiden, liegt die Hybridisierungstemperatur unter 65°C, beispielsweise bei über 55°C, bevorzugt über 60°C, jeweils aber unter 65°C. Stringente Hybridisierungstemperaturen sind abhängig von der Größe bzw. Länge der Nukleinsäure und ihrer Nukleotidzusammensetzungen und sind vom Fachmann durch Handversuche zu ermitteln. Moderat stringente Bedingungen werden beispielsweise bei 42°C und Waschen in 0,2 x SSC/0,1% SDS bei 42°C erreicht.

Die jeweiligen Temperaturbedingungen können in Abhängigkeit von den gewählten Versuchsbedingungen und in Abhängigkeit von der zu untersuchenden Nukleinsäure-Probe unterschiedlich sein und müssen dann entsprechend angepaßt werden. Der Nachweis des Hybridisierungsprodukts kann beispielsweise durch Autoradiographie im Falle radioaktiv markierter Moleküle oder durch Fluorimetrie bei Verwendung von Fluoreszenz-markierten Molekülen erfolgen.

Der Fachmann kann in an sich bekannter Weise die Bedingungen an das gewählte Untersuchungsverfahren anpassen, um tatsächlich moderat stringente Bedingungen zu erzielen und ein spezifisches Nachweisverfahren zu ermöglichen. Geeignete Stringenzbedingungen lassen sich beispielsweise anhand von Referenzhybridisierungen ermitteln. Eine geeignete Nukleinsäure- bzw. Oligonukleotidkonzentration muß eingesetzt werden. Die Hybridisierung muß bei geeigneter Temperatur stattfinden (je höher die Temperatur, um so schwächer die Bindung der Hybride).

Gemäß einer Ausführungsform betrifft die vorliegende Erfindung ein Verfahren zur Herstellung eines wie oben definierten Konjugats, ausgehend von Bestandteil (I) mit bekannter Sequenz, das folgende Schritte umfasst:
- Identifizierung für die Kopplung geeigneter Aminosäurereste durch Analyse der Raumstruktur des Proteins, bevorzugt von Resten außerhalb der Wechselwirkungsfläche von (I) mit dem Liganden;
- Aktivierung eines Kopplungspartners durch ein geeignetes Kopplungsreagenz;
- Durchführung der Kopplungsreaktion;
- Isolierung des Konjugates; und
- Nachweis der Funktionalität beider Komponenten des Konjugates.

Ein modifiziertes Verfahren zur Herstellung eines Konjugates der Erfindung umfasst, ausgehend von Bestandteil (I) mit bekannter Sequenz, in dem keine für die Kopplung geeigneten Aminosäurereste identifiziert wurden, folgende Schritte:
- Einführung für die Kopplung geeigneter Aminosäurereste durch Substitution, Insertion oder Fusion, bevorzugt oberflächenexponierter Reste außerhalb der Wechselwirkungsfläche von (I) mit dem Liganden;
- Nachweis der Zugänglichkeit der eingeführten Aminosäurereste;
- Nachweis der Funktionalität des derart veränderten Bestandteils (I);
- Aktivierung eines Kopplungspartners durch ein geeignetes Kopplungsreagenz;
- Durchführung der Kopplungsreaktion;
- Isolierung des Konjugates; und
- Nachweis der Funktionalität beider Komponenten des Konjugates.

Nähere Ausführungen zu den Kopplungsverfahren etc. finden sich oben.

Hierin beschrieben ist eine pharmazeutische Zusammensetzung, die ein erfindungsgemäß hergestelltes Konjugat und einen pharmazeutisch verträglichen Träger umfasst.

In der pharmazeutischen Zusammensetzung wird das Konjugat mit geeigneten Trägern oder Trägerstoffen in Dosen vermischt, so daß die Erkrankung behandelt oder zumindest gelindert wird. Eine derartige Zusammensetzung kann (zusätzlich zu den Wirkstoffen und dem Träger) Verdünnungsmittel, Füllmaterialien, Salze, Puffer, Stabilisatoren, Solubilisierungsmittel und andere Materialien enthalten, die in der Technik wohlbekannt sind. Der Begriff "pharmazeutisch verträglich" definiert ein nichttoxisches Material, das die Wirksamkeit der biologischen Aktivität des aktiven Inhaltsstoffes bzw. Wirkstoffes nicht stört. Die Auswahl des Trägers hängt vom Verabreichungsweg ab.

Die pharmazeutische Zusammensetzung kann zusätzlich weitere Mittel enthalten, die die Aktivität des Wirkstoffes steigern oder dessen Aktivität oder Verwendung bei der Behandlung ergänzen. Derartige zusätzliche Faktoren und/oder Mittel können in der pharmazeutischen Zusammensetzung enthalten sein, um eine synergistische Wirkung zu erzielen oder um Nebenwirkungen bzw. unerwünschte Wirkungen zu minimieren.

Techniken zur Formulierung bzw. Zubereitung und Verabreichung der Konjugate der vorliegenden Anmeldung sind in "Remington's Pharmaceutical Sciences", Mack Publishing Co., Easton, PA, letzte Ausgabe, zu finden. Eine therapeutisch wirksame Dosis bezieht sich ferner auf eine Menge der Verbindung, die ausreicht, um eine Verbesserung der Symptome zu erreichen, beispielsweise eine Behandlung, Heilung, Prävention oder Verbesserung derartiger Zustände. Geeignete Verabreichungswege können beispielsweise orale, rektale, transmucosale oder intestinale Verabreichung und parenterale Verabreichung einschließen, einschließlich intramuskulärer, subkutaner, intramedulärer Injektionen ebenso wie intrathekaler, direkt intraventrikulärer, intravenöser, intraperitonealer oder intranasaler Injektionen. Die intravenöse Verabreichung an einen Patienten wird bevorzugt.

Ein wie hierin definiertes Konjuga kann in der Diagnostik, Therapie und Affinitätschromatographie verwendet werden.

Die dargestellten Kopplungsmethoden und die damit erhaltenen Daten ermöglichen die verschiedensten Anwendungen der Konjugate, darunter der Einsatz der Konjugate in der Affinitätschromatographie. Beispiele dafür sind der Ersatz von Protein A zur Reinigung von Antikörpern und die Aufreinigung von Blutplasmaproteinen, Wachstumsfaktoren oder Influenzaimpfstoffen mittels Affinitätschromatographie sowie die Reinigung von rekombinant hergestellten Proteinen mit Affinitätsanhängseln oder die Abreicherung von Endotoxinen bzw. Albuminen. Durch eine Kopplung an Matrices ist weiterhin die Verwendung in der Blutplasmaphorese oder der Bioremedation vorstellbar.

Ein weiteres Anwendungsgebiet ergibt sich in der Diagnostik. Denkbar sind hier der Einsatz im Screening von Blutbanken auf bakterielle oder virale Infektionen oder in klassischen Nachweistechniken wie ELISA oder neuen Entwicklungen wie in einem Luminex System. Ebenfalls in der Diagnostik aber auch in der Therapie sind solche Konjugate in der Separation von Zellen einsetzbar.

Die Verwendung der erfindungsgemäß hergestellten Konjugate in der Therapie ist ebenfalls möglich, insbesondere der allgemeine Gebrauch als Transport-Moleküle. Weitere Anwendungen in der Therapie wären in der Gentherapie beim gezielten Targeting durch ein Polypeptidmolekül gemäß der Erfindung und der Kopplung an Systeme zum Gentransfer. Ebenfalls durch ein gezieltes Targeting und die Kopplung an ein bakterielles Toxin würde sich der Einsatz als Immunotoxin in der Therapie ergeben.

Die vorliegende Erfindung wird nachfolgend anhand mehrerer Abbildungen und den begleitenden Beispielen beschrieben, die jedoch den Umfang der Erfindung nicht einschränken sollen, sondern diesen lediglich veranschaulichen.

Abbildung 1: Räumliche Struktur des gamma-II-Kristallins. Die *de novo* generierte Bindungsfläche in der N-terminalen Domäne ist in gelb dargestellt. Die C-terminale Lage der außerhalb der Bindungsfläche lokalisierten Lysinreste ist durch rote Kalotten hervorgehoben.

Abbildung 2: Sensorgramme der Biacore-Experimente zur Kompetition der Bindung der Affilin™-Varianten SPC1-A1 (A), SPC1-A7 (B) und SPC-1-G3 (C) an einen CM5-Chip mit immobilisierten IgG-Fc. Für die Experimente wurden 180 RU an polyklonalem IgG-Fc immobilisiert. Zur Kompetition der Bindung wurden die angegebenen Konzentrationen der Varianten und IgG-Fc eingesetzt. Als Laufpuffer wurde HBS-EP mit einer Flussgeschwindigkeit von 30 µl/min verwendet.

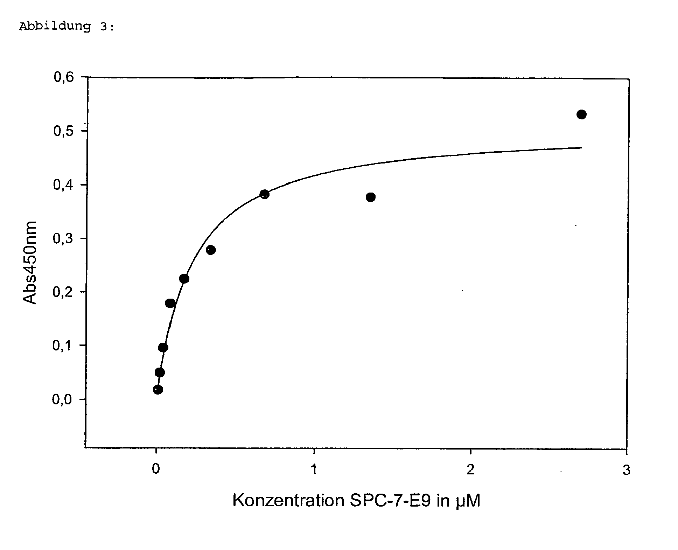

Abbildung 3: Konzentrationsabhängiger ELISA zum Nachweis der Bindung von SPC7-E9 an proNGF. Die Mikrotiterplatte wurde mit 10 µg/ml proNGF beschichtet. Als Detektionsantikörper diente ein anti-humanes gamma-II-Kristallin Antikörper-POD Konjugat in einer Verdünnung von 1:1000. Die dargestellten Absorptionswerte sind Mittelwerte aus zwei Parallelmessungen. Es konnte ein apparenter K_{D}-Wert von 200 nM berechnet werden.

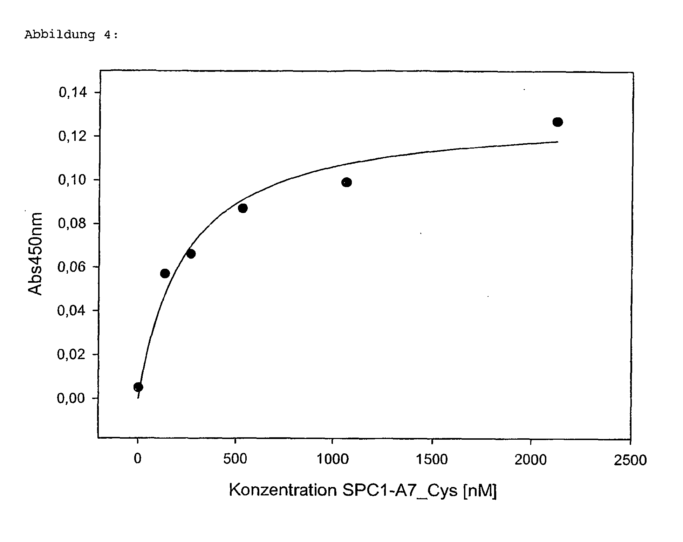

Abbildung 4: Konzentrationsabhängiger ELISA zum Nachweis der Bindung von SPC1-A7_Cys an humanes IgG-Fc. Die Mikrotiterplatte wurde mit 10 µg/ml IgG-Fc beschichtet. Als Detektionsantikörper diente ein anti-humanes gamma-II-Kristallin Antikörper-POD Konjugat in einer Verdünnung von 1:1000. Die dargestellten Absorptionswerte sind Mittelwerte aus zwei Parallelmessungen. Es konnte ein apparenter K_{D}-Wert von 233 nM berechnet werden.

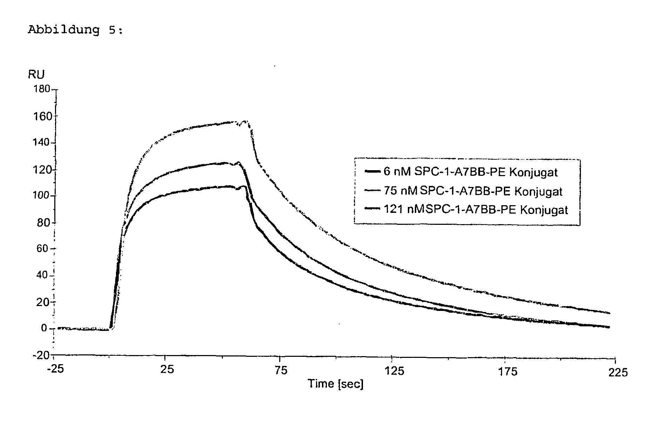

Abbildung 5: Sensorgramme der Biacore-Experimente zur Bindung von SPC1-A7BB-PE Konjugat an einen CM5-Chip mit immobilisiertem IgG-Fc. Es wurden 3000 RU an IgG-Fc immobilisiert und die Konzentrationen 121 nM (rot), 75 nM (grün) und 6 nM (blau) an SPC1-A7BB-PE Konjugat über den Chip geleitet. Die Assoziationsphase betrug 1 min, gefolgt von 3 min Dissoziationsphase. Als Laufpuffer diente HBS-EP mit einer Flussgeschwindigkeit von 30 µl/min. Aus den Kurven konnte ein makroskopischer K_{D}-Wert von 15 nM berechnet werden.

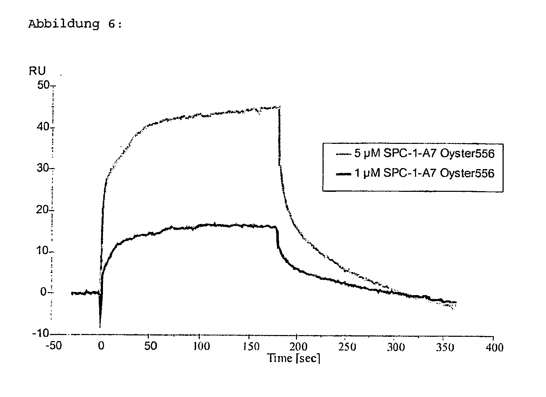

Abbildung 6: Sensorgramme der Biacore-Experimente zur Überprüfung der Bindung von SPC1-A7 Oyster556 an einen CM5-Chip mit immobilisiertem IgG-Fc. Es wurden 1000 RU IgG-Fc auf dem Chip immobilisiert und SPC1-A7Oyster556 in Konzentrationen von 1 µM (blau) und 5 µM (rot) über den Chip geleitet. Die Assoziations- und Dissoziationsphase betrug jeweils 3 min. Als Laufpuffer diente HBS-EP mit einer Flussgeschwindigkeit von 30 µl/min.

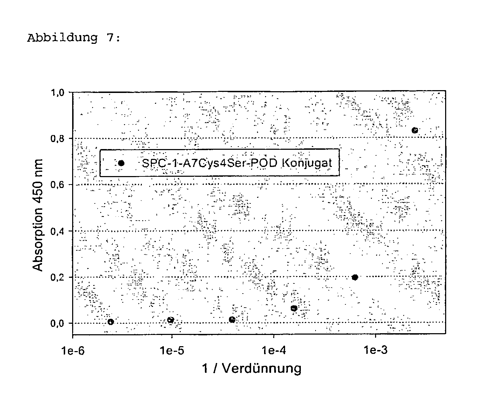

Abbildung 7: Nachweis der Bindung eines Affilin™-POD Konjugates an IgG durch ELISA. Es wurden 10 µg/ml humanes IgG an einer Mikrotiterplatte immobilisiert. Verschiedene Verdünnungen des Affilin™-POD Konjugates in PBS wurden 1 h auf der Mikrotiterplatte inkubiert. Nach einem Waschschritt wurde die Aktivität der gebundenen POD durch TMB-Substratlösung nachgewiesen.

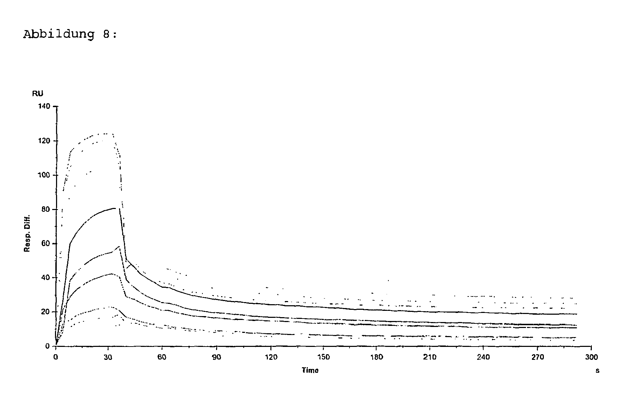

Abbildung 8: Sensorgramme der Biacore-Experimente zur Bindung von SPU11-3-A1_Cys an NGF. Es wurden 200 RU an SPU11-3-A1_Cys an den CM5 Chip mit Hilfe von PDEA gekoppelt und verschiedene Konzentrationen an NGF über den Chip geleitet. Als Laufpuffer diente PBS (1 mM EDTA, 0,005 % Surfactant P20) mit einer Flussgeschwindigkeit von 30 µl/min. Aus den Kurven konnte ein K_{D}-Wert von 46 nM berechnet werden.

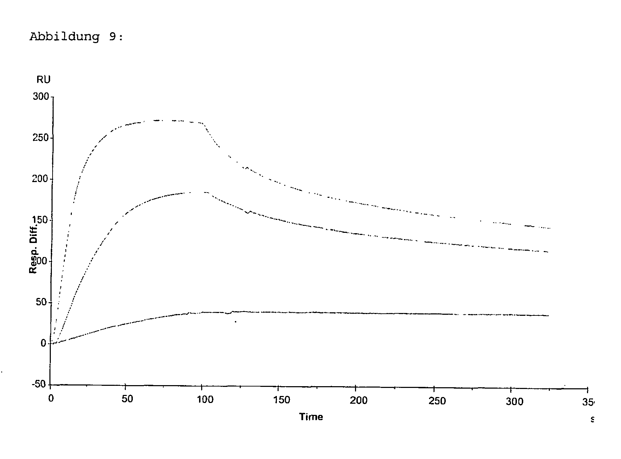

Abbildung 9: Sensogramme der Biacore-Experimente zur Bindung von SPC7-E9 an einen CM5-Chip mit proNGF. Es wurden 280 RU an proNGF immobilisiert und verschieden Konzentrationen proNGF über den Chip geleitet. Als Laufpuffer diente HBS-EP mit einer Flussgeschwindigkeit von 30 µl/min. Aus den Kurven konnte ein K_{D}-Wert von 1,4 nM berechnet werden.

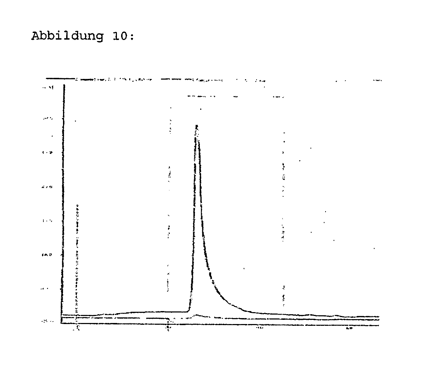

Abbildung 10: Elution von proNGF von einer SPC7-E9 Affinitätssäule. Es wurden 400 µg gereinigtes proNGF aufgetragen (Zeitpunkt 0, gestrichelte pinke Linie) und anschließend wurde mit 20 Säulenvolumen Laufpuffer gespült. Die Elution erfolgte mit 0,1 M Glycin pH 2,2 (grüne Linie). Der Lauf wurde bei einem Fluss von 1 ml/min durchgeführt. Die Detektion des Proteins erfolgte bei 280 nm (blaue Linie).

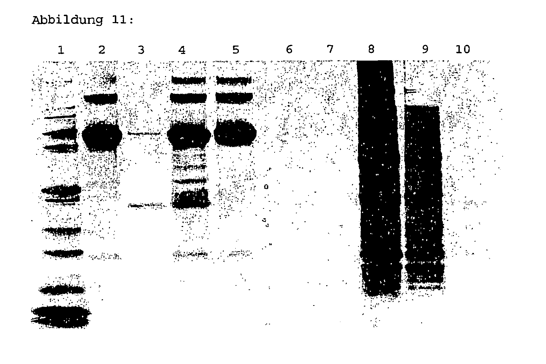

Abbildung 11: SDS-PAGE der Separation von proNGF aus einer BSA-Lösung und aus E.coli Rohextrakt. (v.l.n.r) Bahn 1: Markerproteine, Bahn 2: BSA-Standard, Bahn 3: proNGF-Standard, Bahn 4: Gemisch aus BSA und proNGF Standards (Auftrag), Bahn 5: Durchlauf, Bahn 6: Elution mit 0,2 M Glycin (pH 2,2), Bahn 7: leer, Bahn 8: Gemisch aus *E.coli* Rohextrakt (BI 21) und proNGF-Standard, Bahn 9: Durchlauf, Bahn 10: Elution mit 0,2 M Glycin (pH 2,2)

### Beispiel 1

### Selektion IgG-Fc und proNGF bindender Affilin™-Variante aus der humanen gamma-II-Kristallin Bibliothek, Expression und Reinigung

Ausgehend von der humanen gamma-Kristallin Bibliothek CR20 wurde ein Selektionsprozess mit Hilfe des Phage Display Systems durchgeführt. Dabei konnten bereits nach einer Runde mehrere Affilin™ Varianten selektiert und isoliert werden, die im Einzelphagen-ELISA eine spezifische Bindung an IgG-Fc aufwiesen. Es sei darauf hingewiesen, dass der wie hierin verwendete Begriff "Affilin™" dem erfindungsgemäßen Polypeptidmolekül-Bestandteil des Konjugates entspricht, der auf Ubiquitin oder gamma-Kristallin basiert und gegenüber dem Wildtyp eine veränderte Bindungseigenschaft zur spezifischen Bindung an einen Liganden aufweist. Nach Umklonierung der Gene in den Expressionsvektor pET20b (Novagen) wurden die Affilin™-Varianten rekombinant in *E.coli* (BL21(DE3), Stratagene) überexprimiert und anschließend in zwei Chromatographieschritten (IMAC und Gelfiltration) gereinigt. In einem proteinkonzentrationsabhängigen ELISA und in BIACORE-Experimenten konnte eine spezifische Bindung an IgG-Fc mit einer Dissoziationskonstanten im nM-Bereich bestimmt werden.

Zur Selektion von IgG-Fc bindenden Affilin™-Varianten wurde 1 ml der Bibliothek CR20 (6.5 x 10¹⁰ cfu) in 1 12 x YT-Medium mit 2% Glucose und 100 µg/ml Ampicillin bei 37°C und 220 rpm bis zu einer optischen Dichte von OD₆₀₀ = 0,4 inkubiert. Anschließend wurde die Bakterienkultur mit einem 10fachen Überschuss von Helferphagen M13K07 (Invitrogen, Karlsruhe, Deutschland) zur Infektion für 1 h bei 37°C und 100 rpm inkubiert. Die Bakteriensuspension wurde dann 20 min bei 1000 x g zentrifugiert, und das Pellet in 112 x YT-Medium mit 8 mM GSH, 100 µg/ml Ampicillin und 50 µg/ml Kanamycin resuspendiert. Die Phagenproduktion bzw. Phagenfreisetzung erfolgte bei 30 °C und 200 rpm über Nacht. Zur Isolierung der Phagen wurde das von Kay, Winter & McCafferty (1996) beschriebene Protokoll verwendet.

1 ml der isolierten Phagen (1.4 x 10¹⁴ cfu) wurden mit 1 ml 6 % BSA in PBS für 1 h bei Raumtemperatur (RT) geblockt. Währenddessen wurden 10 Vertiefungen einer Mikrotiterplatte (NUNC), welche über Nacht bei RT mit 100 µl einer 10 µg/ml Lösung des monoklonalen IgG-Fc (Roche) in PBS beschichtet wurden, dreimal mit PBS; 0,1 % Tween 20 gewaschen. Anschließend wurden freie Bindungsstellen der Vertiefungen mit je 300 µl PBS (3% BSA, 0,5% Tween 20) für 2 h bei RT geblockt. Es folgte ein dreimaliges Waschen der Vertiefungen mit PBS (0,1 % Tween 20). Nach Zugabe von je 100 µl der geblockten Phagen pro Vertiefung erfolgte eine Inkubation für 1 h bei RT und 20 rpm. Ungebundene bzw. schwach gebundene Phagen wurden durch 2maliges Waschen mit 2 x PBS, 2maliges Waschen mit 2 x PBS, 3 % BSA und abschließend 2 x mit 2x PBS entfernt. Die noch gebundenen Phagen wurden durch Zugabe von 100 µl/well 100 mM Triethylamin und Inkubation für 10 min bei RT eluiert. Zur Neutralisation der basisch eluierten Phagen wurden (gesamt 1ml) diese mit 500 µl 1 M Tris/HCl pH 7,4 versetzt. Danach wurden die Vertiefungen dreimal mit PBS gewaschen.

Stark gebundene Phagen, die trotz Elution mit Triethylamin in der Mikrotiterplatte verblieben waren, wurden direkt zur Reinfektion mit 100 µl einer expotentiell wachsenden Zellkultur (OD₆₀₀ = 0,4) von XL1-Blue für 30 min bei 37°C inkubiert. Zur Reinfektion von XL1-Blue Zellen mit den basisch eluierten Phagen wurden 750 µl des neutralisierten Eluates mit 9 ml XL1-Blue Zellen mit einer OD₆₀₀ = 0,4 für 30 min bei 37°C inkubiert. Anschließend wurden die reinfizierten Zellen von basisch eluierten Phagen und stark gebundenen Phagen vereinigt, auf 16 x 16 cm Platten mit SOBAG - Medium (inklusive Ampicillin) ausplattiert und über Nacht bei 37°C inkubiert. Nach einen Panningprozess wurden ca. 2000 Klone erhalten, die mit ca. 12,5 ml 2 x YT-Medium; 20 % Glycerin von den Platten abgeschwemmt und bei -80°C aufbewahrt wurden.

Zur Anzucht von Einzelphagen wurde der nach der ersten Panning-Runde erhaltene Zellpool auf Selektionsmedium (SOBAG) ausplattiert und über Nacht bei 37°C inkubiert. Von der SOBAG-Platte wurden 92 Einzelklone in 24 x 5 ml *Deep-well*-Platten mit je 2 ml/Vertiefung 2 x YT-Medium mit 2 % Glucose und 100 µg/ml Amp überführt und über Nacht bei 37°C und 180 rpm inkubiert. Zusätzlich wurde pro Platte eine Einzelkolonie (X11-blue) mit dem Gen für das humanegamma-Kristallin Wildtyp im Phagemid-Vektor als Kontrolle mitgeführt. Sterile 24 x 5 ml *Deep-well*-Platten wurden mit je 2,5 ml/Vertiefung 2 x YT-Medium mit 2 % Glucose und 100 µg/ml Amp mit 1 % Inokulum der Übernacht-Kultur beimpft und die Bakterienkulturen bei 37°C und 180 rpm bis OD₆₀₀ ca. 0,4 inkubiert. Anschließend wurden die Kulturen mit 2,5 µl pro Vertiefung Helferphagen M13K07 mit 10¹³ cfu/ml infiziert und für 1 h bei 37°C und 100 rpm inkubiert. Im Anschluss wurden die Bakterien durch Zentrifugation pelletiert, der Überstand wurde verworfen und die Pellets in 2,5 ml pro well 2 x YT-Medium, 8 mM GSH, 100 µg/ml Ampicillin, 50 µg/ml Kanamycin resuspendiert und über Nacht bei 30°C und 200 rpm inkubiert. Zur Gewinnung des Phagen-Überstandes erfolgte eine Zentrifugation der Platten bei 4600 rpm. Fällung und Pelletierung der Phagen wurden, wie bei Kay, Winter & McCafferty beschrieben, durchgeführt und das Phagenpellet in ca. 200 µl PBS, 3% BSA, pH 7,4 resuspendiert. Durch diese Prozedur konnten die Phagen konzentriert und anschließend in einem ELISA eingesetzt werden.

Hierzu wurden die Vertiefungen einer NUNC-Platte mit 100 µl Antigenlösung (10 µg/ml humaner monoklonaler IgG-Fc, bzw. BSA) über Nacht bei 4°C beschichtet. Die ELISA-Platte wurde am nächsten Tag mit Blockierungspuffer (PBS, 3% BSA, 0,5% Tween 20, pH 7,4) für 2 h bei Raumtemperatur inkubiert. Nach Waschen der Vertiefungen mit Waschpuffer (PBS, 0.1% Tween 20, pH 7,4) wurden jeweils 100 µl der geblockten Phagenpräparationen in die Vertiefungen gegeben und für 1 h bei RT inkubiert. Nach erneutem Waschen der Vertiefungen mit Waschpuffer (PBS, 0,1% Tween 20, pH 7,4) wurde der monoklonale anti-M13 Antikörper (POD-konjugiert; MoBiTec, Göttingen) in einer Verdünnung von 1:5000 in PBS, pH 7,4 aufgetragen (100 µl/Vertiefung) und wieder für 1 h bei RT inkubiert. Danach wurden die Vertiefungen 3 x mit Waschpuffer (PBS, 0,1% Tween 20, pH 7,4) und 3 x PBS gewaschen und die Farbreaktion mit TMB Plus (Kementec, DK) initiiert (100 µl/ Vertiefung). Nach 20 Minuten wurde die Farbreaktion durch Zugabe von 0,2 M H₂SO₄ abgestoppt. Die erhaltene Gelbfärbung wurde bei 450 nm eingelesen (Referenzwellenlänge: 620 nm) und dokumentiert.

Von den Phagenpräperationen, welche ein deutliches Signal hinsichtlich der Bindung an monoklonales IgG-Fc und eine kaum nachweisbare Bindung an BSA zeigten, wurden die Gene der gamma-II-Kristallin Varianten mit dem primer *pCAN700* sequenziert. Drei daraus resultierende Klone SPC1-A1, SPC1-A7 und SPC1-G3 wurden in den Expressionsvektor pET20b über die Restriktionsschnittstellen *Nco*I und *Bst*EII umkloniert und in den Expressionsstamm BL21(DE3), pUBS520 (Stratagene) eingebracht.

Die Zellen wurden in 2 x YT-Medium mit 100 µg/ml Ampicillin und 50 µg/ml Kanamycin bis zu einer optischen Dichte von OD₆₀₀= 0,6 - 0,8 bei 37°C und 200 rpm angezogen und anschließend die rekombinante Proteinexpresssion mit IPTG (1mM Endkonzentration) induziert. Nach vierstündigem Wachstum bei 30°C und 200 rpm wurden die Zellen durch Zentrifugation (6000 x g, 20 min, 4°C) geerntet. Der Aufschluss erfolgte in NPI-10 Puffer (Qiagen) durch Lysozym (0,1 mg/ml) und Ultraschall (6 x 15 sec, unter Eiskühlung) in Anwesenheit von 5 mM beta-Mercaptoethanol. Nach Zentrifugation (40000 x g, 30 min, 4°C) wurde der Überstand auf eine IMAC-Säule (HiTrap Chelating HP, Amersham Bioscience) appliziert und mit NPI-20 (Quiagen, + 5 mM beta-Mercaptoethanol) gewaschen (20 Säulenvolumen). Die Elution erfolgte durch einen linearen Gradienten mit NPI-500 (Quiagen, + 5 mM beta-Mercaptoethanol) in 30 Säulenvolumen. Die gamma-II-Kristallin enthaltenen Fraktionen wurden mittels SDS-PAGE analysiert, entsprechende Proben gepoolt und auf eine Gelfiltrationssäule (1,6 x 60, Sephadex 75, Amersham Biosciences) aufgetragen. Als Laufpuffer diente PBS, bei einer Flussrate von 0,75 ml/min. Die Analyse der Gelfiltration erfolgte mittels SDS-PAGE, die gamma-II-Kristallin enthaltenen Fraktionen wurden vereinigt und bei 4°C aufbewahrt. Die Affilin™ Varianten hatten nach dieser Reinigungsprozedur eine Reinheit von >95% (SDS-PAGE).

Die Bindungseigenschaften der gereinigten Proteine wurden nun wie oben beschrieben in einem konzentrationsabhängigen ELISA getestet. Dabei wurden verschiedene Konzentrationen (100 nM - 10 µM) der Affilin™-Varianten eingesetzt und ein anti-gamma-II-Kristallin Antikörper (monoklonales Antikörperkonjugat mit POD) als Detektionsantikörper eingesetzt. Es zeigte sich dabei, dass alle drei getesteten Affilin™-Varianten eine spezifische Bindung gegenüber humanen IgG-Fc besitzen und eine unspezifische Bindung an BSA oder die Mikrotiterplatte nicht nachweisbar war. Der als Kontrolle mitgeführte humane gamma-II-Kristallin-Wildtyp zeigte keinerlei Bindung an IgG-Fc, BSA, oder die Mikrotiterplatte.

In BIACORE-Experimenten wurden die Dissoziationskonstanten der drei Affilin™-Varianten bestimmt. Dazu wurde auf einem CM5 Chip ca. 180 RU an humanen IgG-Fc (50 µg/ml in 50 mM NaCitrat, pH 5,0) immobilisiert. Freie Bindungsstellen wurden abschließend durch 1 M Ethanolamin (pH 8,5) inaktiviert.

Bei einem Fluss von 30 µl/min wurden nun 6 verschiedene Konzentrationen (166 nM - 1µM) für 180 sec über den Chip geleitet. Anschließend wurde bei dem gleichen Fluss der Chip mit HBS, 0,005 % Surfactant P20 (Biacore) für 180 sec gespült. Aus den resultierenden Sensogrammen konnte mit Hilfe der BiaEvaluation Software (Biacore, Uppsala, Schweden) folgende Dissoziationskonstanten der Affilin™-Varianten gegenüber IgG-Fc bestimmt werden: SPC1-A1 mit 230 nM, SPC1-A7 mit 280 nM und SPC1-G3 mit 800 nM. In Kompetitionsexperimenten konnte die spezifische Bindung der Affilin™-Varianten an IgG-Fc und nicht an die Chip-Matrix nachgewiesen werden (Abb.2).

Analog zur Selektion der Varianten SPC1-A1, SPC1-A7 und SPC1-G3 wurde die Affilin™ Variante SPC7-E9 gegen das Zielmolekül proNGF isoliert. Die Dissoziationskonstante konnte mit Hilfe von BIACORE-Messungen mit 1 - 6 nM bestimmt werden (Abb.3).

### Beispiel 2

### Selektion von Affilin™-Varianten aus der humanen Ubiquitin Bibliothek (UB10) gegen ein Cystein-Knotenprotein - Expression und Reinigung

### Bereitstellung eines synthetischen Ubinuitin-Gens für die Selektion von modifizierten Proteinen mit neu erzeugter Bindungsaffinität

Die gentechnischen Arbeiten wurden sofern nicht anders angegeben nach dem Fachmann geläufigen Standardprotokollen wie z. B. von Sambrook *et al.* (1989) durchgeführt.

Für die Herstellung der DNA-Sequenz (Seq-ID No. 25) für ein modifiziertes Ubiquitin-Proteingerüst mit den Substitutionen Ile44Ala, Lys48Arg, Arg54Leu, Val70Ala, Arg72Leu, Gly75Ala sowie mit der Deletion von Gly76 als Ausgangspunkt für die Gewinnung von künstlichen Bindungsproteinen wurde folgendermaßen vorgegangen: Für die Gensynthese wurde eine PCR-Reaktion in einem Volumen von 50 µl durchgeführt, in dem jeweils 2,5 µl der sechs Oligodesoxynukleotide (Seq-ID No. 26, Seq-ID No. 27, Seq-ID No. 28, Seq-ID No. 29, Seq-ID No. 30, Seq-ID No. 31; jew. 0,1 µM), die in ihrer Basenpaarabfolge insgesamt das zu synthetisierende Gen repräsentierten, als Templates vorlagen. Die Sequenzen der eingesetzten Oligodesoxynukleotide entsprachen dabei jeweils 40 bis 50 Basenpaar langen Abschnitten des kodierenden bzw. nicht-kodierenden DNA-Stranges des künstlichen Gens und überlappten alternierend an ihren 3'- und 5'-Enden mit ca. 15 Basen. Zusätzlich enthielt der Ansatz jeweils 2,5 µl flankierende Primer (Seq-ID No. 32, Seq-ID No. 33; 10 µM) sowie 5 µl 10x Taq-Puffer (100 mM Tris/HCl pH 9,0, 500 mM KCl, 1 % (v/v) Triton X-100), 3 µl 25 mM MgCl₂ und 4 µl dNTP-Mix (je 2,5 mM dATP, dCTP, dGTP, dTTP). Nach Auffüllen mit H₂O wurde der Reaktionsansatz im Thermozykler zwecks Denaturierung für 2 min auf 94 °C erhitzt. Dann wurden 2,5 U der *Taq-*Polymerase (Promega) in der Hitze zugegeben (Hot Start) und das PCR-Programm gestartet. In 25 Zyklen wurde für je 1 min bei 94 °C, 1 min bei 55 °C und für 1,5 min bei 72 °C inkubiert. Eine abschließende Inkubation erfolgte für 5 min bei 72 °C.

Das gewünschte PCR-Produkt wurde mittels analytischer Agarose-Gelelektrophorese identifiziert und aus dem Ansatz mit Hilfe des MinElute Reaction Cleanup-Kit (Qiagen) gereinigt. 1,0 ng der isolierten DNA wurden als Template für eine zweite Amplifizierung verwendet, die diesmal unter Verwendung der *Pfu*-Polymerase (Promega) ebenfalls in einem Volumen von 50 µl realisiert wurde. Dazu wurden 5 µl des mitgelieferten 10x *Pfu*-Puffers (200 mM Tris/HCl, pH 8,8, 20 mM MgCl₂, 100 mM KCl, 100 mM (NH₄)₂SO₄, 1 % (v/v) Triton X-100, 1 mg/ml BSA) sowie 4 µl dNTP-Mix verwendet und mit H₂O aufgefüllt. Zusätzlich enthielt der Ansatz flankierende Primer (Seq-ID No. 32, Seq-ID No. 33; 10 µM) zur Einführung geeigneter Schnittstellen. Das gewünschte PCR-Produkt wurde mittels präparativer Agarose-Gelelektrophorese isoliert und mit Hilfe des Zero Blunt^{®} TOPO^{®} PCR Cloning Kits (Invitrogen) nach Angaben des Herstellers in den Klonierungsvektor pCR^{®}4Blunt-TOPO^{®} inseriert. Mit dem entsprechenden Ligations-Reaktionsansatz wurden mitgelieferte chemisch kompetente Zellen transformiert und auf einer Agar-Platte mit LB/Amp/Kan-Medium ausplattiert. Die Platte wurde für 16 Std. bei 37 °C bebrütet und gewachsenen Kolonien hinsichtlich des erwünschten Ligationsproduktes analysiert. Dazu wurde Plasmid-DNA im Mini-Maßstab mit Hilfe des Plasmid-Isolierungskits der Firma Qiagen nach Angaben des Herstellers präpariert und einem Restriktionsverdau mit den DNA-Endonukleasen *Nde*I und *Xho*I (New England Biolabs), deren Erkennungssequenzen durch die flankierenden Primer in das PCR-Produkt eingeführt worden waren, unterworfen. Mit Plasmiden, die das erwartete Schnittmuster aufwiesen, wurde im Bereich der inserierten Gen-Kassette mit Hilfe der *Taq* DNA-Polymerase eine DNA-Sequenzanalyse durchgeführt. Dabei wurde der CycleReader™ AutoDNA Sequencing Kit (Fermentas) nach Angaben des Herstellers sowie 0,5 µg Plasmid-DNA und 1,0 pmol des entsprechenden fluoreszenz-markierten Primers verwendet. Der dabei neusynthetisierte DNA-Strang wurde während der Polymerasereaktion markiert und durch den Einbau von Di-desoxynukleotiden statistisch, aber basenspezifisch terminiert. Die entstandenen fluoreszierenden DNA-Fragmente wurden anschließend in einem Liquor-Sequenzierautomaten durch Polyacrylamid-Harnstoff-Gelelektrophorese aufgetrennt und als Bandenmuster für A, C, G, T in benachbarten Spuren sichtbar gemacht.

Genkassetten mit korrekter DNA-Sequenz wurden durch präparativen *Nde*I/*Xho*I-Restriktionsverdau aus dem Klonierungsvektor pCR^{®}4Blunt-TOPO^{®} herausgeschnitten und durch präparative Agarose-Gelelektorphorese isoliert. Die Insertion des Gens für das modifizierte Ubiquitin-Proteingerüst erfolgte in den Expressionsvektor pET20B(-) (Novagen) für die Produktion des entsprechenden Proteins bzw. in den Phasmid-Vektor pMUBI-1 zur Konstruktion einer Bibliothek von Ubiquitin-Varianten.

### Herstellung einer Bibliothek von Ubiquitin-Varianten

Zur zufälligen ortsgerichteten Mutagenese von 8 Kodons am Amino- bzw. Carboxyterminus des synthetischen Ubiquitin-Gens wurden zwei aufeinanderfolgende PCR-Reaktionen durchgeführt. Der erste Amplifizierungsschritt fand unter Verwendung der *Pfu*-Polymerase (Promega) in einem Volumen von 10 x 50 µl statt. Dazu wurden pro Ansatz 5 µl des mitgelieferten 10x *Pfu*-Puffers sowie 4 µl dNTP-Mix verwendet und mit H₂O aufgefüllt. Weiterhin enthielt der Ansatz jeweils 2,5 µl flankierende Primer (Seq-ID No. 34, Seq-ID No. 35; 10 µM) zur Einführung der gewünschten Basenpaar-Austausche. Als Template wurden 1,0 ng pMUBI-1 verwendet, der das nicht-mutierte synthetische Ubiquitin-Gen trug. Nach Zugabe von 2,5 U der *Pfu*-Polymerase (s. o.) wurde in 25 Zyklen für je 1 min bei 94 °C, 1 min bei 60 °C und für 1,5 min bei 72 °C inkubiert. Eine abschließende Inkubation erfolgte für 5 min bei 72 °C. Für den selektiven Abbau der eingesetzten Matrizen-DNA, wurden pro Reaktionsansatz 10 U *Dpn*I zugegeben und für 1 Std. bei 37 °C inkubiert. Das gewünschte PCR-Produkt wurde mittels präparativer Agarose-Gelelektrophorese und dem QIAquick Gel Extraction Kit (Qiagen) isoliert.

Der zweite Amplifizierungsschritt wurde in einem 1.000 µl-Ansatz durchgeführt, wobei ca. 1,0 ng des in der ersten PCR-Reaktion gewonnenen Produktes eingesetzt und die *Taq*-Polymerase verwendet wurden. Der Reaktionsansatz wurde - adaptiert auf das 20fache Volumen - wie oben ausgeführt aus 10x *Taq*-Puffer, 25 mM MgCl₂, dNTP-Mix sowie den flankierenden Primern (Seq-ID No. 36, Seq-ID No. 37; 10 µM), die an ihren 5'-Enden biotinyliert waren und jeweils nicht miteinander kompatible Erkennungssequenzen für die Endonuklease *Sfi*I trugen, pipettiert. Nach Auffüllen mit H₂O wurden 2,5 U der Taq-Polymerase in der Hitze zugegeben (s. o.) und das PCR-Programm gestartet. In 25 Zyklen wurde für je 1 min bei 94 °C, 1 min bei 60 °C und für 1,5 min bei 72 °C inkubiert. Eine abschließende Inkubation erfolgte für 5 min bei 72 °C.

Die darauf folgende Spaltung des erhaltenen Amplifizierungsproduktes erfolgte direkt im PCR-Reaktionsansatz. Dazu wurden in einem Gesamtvolumen von 4.000 µl die komplette PCR-Reaktionslösung mit entsprechenden Volumen des mitgelieferten 10x Puffer II (100 mM Tris/HCl, pH 7,9, 100 MgCl₂, 500 mM NaCl, 10 mM Dithiothreitol), 10x BSA-Lösung und H₂O gemischt. Weiterhin wurden 4.000 U des Restriktionsenzyms *Sfi*I (New England Biolabs) zugegeben und für 16 Std. bei 50 °C inkubiert. Die DNA wurde aus dem Ansatz mit Hilfe des MinElute Reaction Cleanup Kit (Qiagen) isoliert und in 400 µl sterilem H₂O aufgenommen. Zur Abtrennung von nicht *Sfi*I-geschnittenem PCR-Produkt wurde die isolierte DNA mit dem gleichen Volumen "Binding-Solution" (Dynal), das 1,0 mg/ml magnetische Kügelchen mit oberflächengekoppeltem Streptavidin ("Dynabeads Kilobase Binder") enthielt, gemischt und für 4,5 Std. auf einem Rollenmischer bei Raumtemperatur (RT) inkubiert. Die Kügelchen mit eventuell noch vorhandener biotinylierter DNA wurden präzipitiert, während komplett mit *Sfi*I gespaltene DNA, die keine biotinylierten Enden mehr aufweisen sollte, im Überstand verblieb und über Nacht gefällt wurde. Das so erhaltene mit *Sfi*I gespaltene und an den gewünschten Positionen mutagenisierte Ubiquitin-Gen wurde in sterilem H₂O gelöst, nochmals mit Hilfe des QIAquick PCR Purification Kit (Qiagen) entsalzt und wies schließlich eine Konzentration von 200 fmol/µl in H₂O auf.

Zur Vorbereitung des Empfängervektors wurde das Phasmid pMUBI-1 nach Herstellerangaben mit *Sfi*I geschnitten und das größere (Vektor-)Fragment mittels präparativer Agarose-Gelelektrophorese und dem QIAquick Gel Extraction Kit (Qiagen) isoliert. Um intramolekulare Ligation zu verhindern wurden seine 5'-Enden dephosphoryliert. Hierfür wurden 0,5 U der Alkalische Phosphatase aus Shrimp (*Pandalus borealis)* sowie der mitgelieferte Puffer in einem Gesamtvolumen von 200 µl verwendet. Die Mischung wurde für 90 min bei 37 °C inkubiert, die DNA aus dem Ansatz mit Hilfe des QIAquick PCR Purification Kit (Qiagen) isoliert und nochmals entsalzt (QIAquick PCR Purification Kit). Die DNA des Vektorfragments wies schließlich eine Konzentration von 50 fmol/µl in H₂O auf.

Zur Ligation wurden 1,6 pmol des PCR-Fragments und 8,0 pmol des Vektorfragments von pMUBI-1 in Gegenwart von 2 U T4 DNA-Ligase (GibcoBRL) in einem Gesamtvolumen 1.600 µl (50 mM Tris/HCl, pH 7,6, 10 mM MgCl₂, 1 mM ATP, 1 mM DTT, 5 % (w/v) PEG-8.000) für drei Tage bei 16 °C inkubiert. Nach Erhitzen des Ansatzes auf 65 °C für 15 min wurde die DNA gefällt. Dazu wurden jeweils 100 µl der Reaktionslösung mit 100 µl Ethanol sowie 10 µl 5 M NaAc, pH 3,0 gemischt und für 16 Std. bei -20 °C gelagert. Anschließend wurde zentrifugiert (60 min, 12.500 g), mit Ethanol (70 % v/v, -20 °C) gewaschen, erneut zentrifugiert und die präzipitierte DNA schließlich in 60 µl sterilem H₂O gelöst.

Zur Elektroporation wurde das Gene Pulser^{®} II-System (Biorad) sowie Küvetten mit Elektrodenabstand von 1,0 mm (Biozym) bei 4 °C im Kühlraum verwendet. Mit jeweils 3,5 µl der oben erhaltenen Lösung wurden elektrokompetente *E. coli* XL1Blue (Stratagene) nach Angaben des Herstellers transformiert. Die erhaltene Zellsuspension wurde auf fünf Agarplatten (20 x 20 cm) mit LB/Chloramphenicol-Medium ausplattiert. Die Platten wurden für 16 Std. bei 37 °C bebrütet und die gewachsenen Kolonien ausgezählt. Die konstruierte Bibliothek enthielt demnach 2,8 x 10⁷ unabhängige Klone von denen jeder 10.000fach in der Bibliothek vorliegen sollte. Die Kolonien wurden dann mit insgesamt 100 ml SOC-Medium mit 10 % (v/v) Glycerin abgeschwemmt und in Aliquots á 1,0 ml bei -80 °C gelagert. Von den erhaltenen Klonen wurde mit Hilfe des DNA-Miniprep Kits der Firma Qiagen von 12 willkürlich ausgewählten der Phasmid-Vektor isoliert und die DNA-Sequenz im Bereich des mutagenisierten Ubiquitin-Gens analysiert. Dabei wiesen alle Klone funktionelle Sequenzen - d. h. keine Verschiebung des Leserasters durch Insertionen oder Deletionen - sowie qualitativ völlig unterschiedliche Substitutionen an den mutagenisierten Positionen auf. Zufällige Austausche außerhalb der mutagenisierten Bereiche waren nicht vorhanden.

Auf Grundlage dieser Bibliothek basierend auf dem humanen Ubiquitin wurde in Analogie zu Beispiel 1 mittels der dem Fachmann bekannten Methode des Phage Display Systems eine Selektion durchgeführt. Kleinere Abweichungen ergaben sich lediglich in Hinsicht auf die Auswahl des verwendeten Antibiotikums (Chloramphenicol statt Ampicillin).

Als Target diente ein Wachstumsfaktor aus der Familie der Cystein-Knotenproteine. Das Ubiquitin Affilin™ SPU11-3-A1 wies eine, im ELISA bestimmte, Dissoziationskonstante im nM Bereich auf und wurde für Kopplungsstudien im BIACORE System verwendet.

Eine der daraus selektierten Varianten, SPU11-3-A1, wurde in den Expressionsvektor pET20b über die Restriktionsschnittstellen *Nde*I und *Xho*I einkloniert. Die Anzuchtbedingungen und Reinigungsprozedur waren identisch der für SPC Affilin™ (IMAC, Gelfiltration), wie in Beispiel 1 aufgeführt. Zur Detektion der Bindungseigenschaften wurde ein konzentrationsabhängiger ELISA durchgeführt. Dabei wurden verschiedene Konzentrationen (10 nM - 1 µM) der Affilin™-Variante auf die mit dem Targetmolekül beschichtete Mikrotiterplatte (MTP) aufgetragen und ein polyklonales anti-Ubiquitin Antiserum (Sigma) als primäres Nachweisreagenz eingesetzt. Nach Inkubation (1h) bei RT wurden die Vertiefungen der MTP 3x mit PBS gewaschen und in einem zweiten Schritt ein monoklonales Antikörperkonjugat (anti IgG, Sigma) mit POD als Detektionsantikörper eingesetzt. Es zeigte sich dabei, die getestete Affilin™-Variante eine spezifische Bindung gegenüber humanen NGF besitzt und eine unspezifische Bindung an BSA oder die Mikrotiterplatte nicht nachweisbar war. Der als Kontrolle mitgeführte humane Ubiquitin-Wildtyp zeigte keinerlei Bindung an NGF, BSA, oder die Mikrotiterplatte.

### Beispiel 3

### C-terminale Fusion von Affilin™ mit einem Cystein haltigen Peptid-Linker zur selektiven Kopplung an verschiedene Moleküle

Im folgenden Beispiel wird gezeigt, dass die IgG-Fc bindende Affilin™-Variante SPC1-A7 selektiv über ein C-terminales Cystein an verschiedene Moleküle gekoppelt werden konnte.

Die eingesetzte Affilin™-Variante SPC1-A7 weist, neben den im Inneren des Proteins lokalisierten 7 Cysteinen, in der variablen Position 4 bereits ein lösungsmittelzugängliches und somit freies Cystein auf. Dieses wurde zunächst durch QuickChange® PCR durch ein Serin substituiert. Ausgehend von diesem modifizierten Affilin™ (SPC1-A7BB) wurden C-terminal zwei Glycine und ein Cystein sowie zusätzlich zu den vorhandenen sechs Histidinen vier weitere Histidine durch QuickChange® PCR inseriert. Der auf 10 Histidine verlängerte Affinitätstag sollte einer verbesserten Reinigung dienen. In Titrationexperimenten mit Ellmann's-Reagenz erwies sich das eingeführte Cystein aufgrund von Cystein-Shuffling Ereignissen mit in der Affilin™-Variante vorhandenen weiteren Cysteine für Kopplungsexperimente als ungeeignet. Aufgrund dessen wurde das Cystein auf DNA-Ebene durch ein Serin (TCT) substituiert und ausgehend von diesem Konstrukt wurde nach einem Gly₄Ser-Linker wiederum ein Cystein eingeführt. Dies sollte den Abstand des inserierten Cystein von den Cysteinen im Protein vergrößern und ein Cystein-Shuffling unterdrücken. Das entstandene Konstrukt wurde abschließend sequenziert und erwies sich in Titrationsexperimenten mit Ellmann's-Reagenz für Kopplungsexperimente als geeignet.

Zur Substitution des Cystein in Position 4 durch ein Serin in der Affilin™-Variante SPC1-A7 wurde die Methode der QuickChange® PCR (Stratagen, La Jolla, USA) mit den Primern *A7Cys4Ser_for* und *A7Cys4Ser_rev* verwendet. Für die PCR-Reaktion wurden 5 µl 10 x Reaktionspuffer (100 mM KCl,100 mM (NH4)₂SO₄, 200 mM Tris-HCl, pH 8,8, 20 mM MgSO₄, 1% Triton^{®} X-100, 1 mg/ml BSA), je 125 ng der beiden Primer, 1 µl Pfu-Turbo DNA Polymerase, 1µl dNTP-Mix und H₂O bis zu einem Gesamtvolumen von 50 µl eingesetzt. Als Template-DNA diente das Gen der Affilin™-Variante SPC1-A7 in dem Vektor pET20b. Begonnen wurde mit einem ersten Denaturierungsschritt von 3 min bei 95°C. Danach folgten 18 wiederholende Zyklen der Denaturierung, Primer-Anlagerung und Synthese. Die Denaturierung bei 95°C dauerte 30 sec, die Primer-Anlagerung erfolgte für 1 min bei 60°C. Die Dauer des Syntheseschrittes betrug 5 min bei 68°C. Zum Abschluss der PCR erfolgte eine Endsynthese für 5 min bei 68°C. Die Amplifikation wurde durch Agarose-Gelelektrophorese überprüft. Nach erfolgreicher Amplifikation schloss sich ein Restriktionsverdau der eingesetzten Template-DNA mit Hilfe des Restriktionsenzymes DpnI an. Es wurde 1 µl des Enzymes (10 U/µl) zu dem PCR-Ansatz pipettiert, gemischt und für 1 h bei 37°C inkubiert. Der Vektor wurde nun mittels Elektroporation in einen kompetenten Stamm (X1-1blue, Stratagene) eingebracht. Dazu wurde 1µl des DpnI behandelten Restriktionsansatzes auf Eis zu 50 µl elektrokompetenten XL-1 blue Zellen pipettiert, gemischt und in einer eisgekühlten Elektroporationküvette (0,1 mm) bei 2,5 kV, 25 µF und 200 Ω gepulst. Die Zellen wurden in 1 ml SOC-Medium aufgenommen und 60 min bei 37°C unter Schütteln bei 500 rpm inkubiert. Anschließend wurden die Zellen auf Selektionsmedium (2 x YT, 100 µg/ml Ampicillin) ausplattiert und bei 37°C für 16 h inkubiert. Von den erhaltenen Klonen wurden 12 zur Kontrolle des korrekten Einbaus separat mit dem Primer *pETTerm* sequenziert. Der Vektor eines korrekten Klones diente als Template-DNA für die nächste QuickChange^{®} PCR zum C-terminalen Einbau von zwei Glycinen, einem Cystein und vier zusätzlichen Histidinen. Wie oben beschrieben wurde mit den beiden Primern *A7Gly2Cys_for* und *A7Gly2Cys_rev* eine QuickChange^{®} PCR, der anschließende DpnI-Verdau und die Transformation des Stammes XL1-Blue mittels Elektroporation durchgeführt. Zur Kontrolle des korrekten Einbaus wurden wiederum die Plasmide von 12 Klonen sequenziert. Das Plasmid eines korrekten Klones (SPC-1-A7JJ) wurde in den Expressionsstamm BL21 mit dem Plasmid pUBS520 eingebracht, um anschließend die Affilin™-Variante SPC1-A7BB zu exprimieren und wie oben (Beispiel1) beschrieben in zwei Chromatographieschritten (Affinitätschromatographie an Ni-NTA und Gelfiltration an Sephadex 75) zu reinigen.

Zur Überprüfung der Zugänglichkeit (Haber, 1972) des eingeführten Cysteinrestes sollten alle freien SH-Gruppen mit Hilfe von Ellmann's-Reagenz (DTNB-Lösung) titriert werden. Dazu wurden zu 1 ml Proteinlösung der Affilin™-Variante SPC1-A7JJ (50 - 350 µg Protein in 100 mM Tris/HCl; pH 8,0) 30 µl DTNB-Lösung (4 mg/ml DTNB in 100 mM Tris/HCl; pH 8,0) zugegeben. Leerwert 1 stellte 1 ml Proteinlösung mit 30 µl Puffer (100 mM Tris/HCl; pH 8,0) dar. Als Leerwert 2 diente 1 ml Puffer (100 mM Tris/HCl; pH 8,0) mit 30 µl DTNB-Lösung. Die Ansätze wurden 15 min bei Raumtemperatur inkubiert und die Absorption bei 410 nm gemessen. Die Absorptionen der Leerwerte 1 und 2 wurden von der Absorption des Testansatzes abgezogen. Aus dem resultierenden Absorptionswert wurde mit Hilfe des Extinktionskoeffizienten von DTNB-SH die molare Konzentration an freien SH-Gruppen berechnet (ε_{410 [DTNB-SH]} = 13600 M⁻¹cm⁻¹) und durch die eingesetzte Proteinkonzentration dividiert. Als Ergebnis wurde die Anzahl der freien Thiolgruppen pro Proteinmolekül erhalten. Dabei konnten bei der konstruierten Affilin™-Variante SPC1-A7JJ 3-4 freie Cystein-Reste titriert werden. Erwartet wurde ein freier Cysteinrest, da die im Protein vorhandenen Cysteine, wie bei der Kontrolle der Titration mit der Affilin™-Variante SPC-1-A7Cys4Ser nachgewiesen, nicht zugänglich sind. Dies deutet auf ein Cystein-Shuffling des eingeführten C-terminalen Cysteins mit den im Protein vorborgenen Cysteinen und somit einen zu geringen Abstand des eingeführten Cysteins zum Protein hin. Aufgrund dessen wurde das C-terminale Cystein der Affilin™-Variante SPC-1-A7JJ durch ein Serin durch eine QuickChange^{®} PCR wie oben beschrieben mit den Primern *A7Gly2Ser_for* und *A7Gly2Ser rev* substituiert. Nach Überprüfung des korrekten Einbaus diente dieses Konstrukt als Template für eine PCR zum Einbau eines Gly₄Ser-Linkers gefolgt von einem Cystein. Mit Hilfe der Primer *Gly4SerCys_HindIII* und *A7Cys4Ser_Nde* wurde eine PCR durchgeführt. Für die PCR-Reaktion wurden 5 µl 10 x Reaktionspuffer (100 mM KCl,100 mM (NH₄)₂SO₄, 200 mM Tris-HCl, pH 8.8, 20 mM MgSO₄, 1% Triton^{®} X-100, 1 mg/ml BSA), je 125 ng der beiden Primer, 1 µl Pfu-Turbo DNA Polymerase, 1µl dNTP-Mix und H₂O bis zu einem Gesamtvolumen von 50 µl eingesetzt. Außerdem wurden zur Auflösung von Sekundärstrukturen der Primer 2 µl DMSO dem Reaktionsgemisch zugesetzt. Entgegen der oben beschriebenen Vorgehensweise wurden 25 wiederholende Schritte an Denaturierung, Primer-Anlagerung und Synthese und bei einer veränderten Temperatur der Primer-Anlagerung von 58°C durchgeführt. Die Amplifikation des PCR-Produktes dieser Affilin™-Variante (SPC1-A7_Cys) wurde mittels Agarosegel-Elektrophorese überprüft. Tabelle 2 gibt eine Übersicht über die hier beschriebenen Konstrukte (Tab.2). Im Primer *A7Cys4Ser_Nde* ist eine Restriktionsschnittstelle für das Enzym NdeI und im Primer *Gly4SerCys_HindIII* eine für das Enzym *Hind*III integriert, womit es möglich ist, das PCR-Produkt nach dessen Reinigung und Restriktion durch die beiden Enzyme in den ebenfalls mit NdeI und HindIII behandelten Vektor pET20b zu ligieren. Die Restriktion durch die beiden Enzyme wurde gleichzeitig in einem Doppel-Verdau durchgeführt. Dazu wurden ca. 1 µg PCR-Produkt der Afffilin™-Variante SPC1-A7_Cys bzw. 1µg Vektor pET20b mit 1µl des Restriktionsenzyms NdeI (New England Biolabs, Frankfurt am Main, Deutschland, 20 U/µl) und 1µl des Restriktionsenzyms HindIII (New England Biolabs, Frankfurt am Main, Deutschland, 20 U/µl) sowie 10 µl 10 x Reaktionspuffer NEB Puffer 2 (50 mM NaCl, 10 mM Tris-HCl, pH 7,9, 10 mM MgCl₂, 1 mM DTT) in einem Gesamtvolumen von 100µl für 4 h bei 37°C inkubiert. Die entstandenen Fragmente wurden separat über eine präperative Agarosegel-Elektrophorese gereinigt. Für die Ligation wurden 20 ng des gereinigten und NdeI/HindIII behandelten Fragmente des Vektor pET20 und 120 ng des gleich behandelten Fragmentes der Affilin™-Variante SPC1-A7Cys sowie 2 µl des 10 x Reaktionspuffer (300 mM Tris-HCl, pH 7,8, 100 mM MgCl₂, 100 mM DTT, 10 mM ATP) und 0,5 µl T4 DNA Ligase (Promega, Mannheim, Deutschland, 1 - 3 U/µl) in einem Gesamtvolumen von 20 µl eingesetzt. Der Reaktionsansatz wurde 16 h bei 16°C inkubiert und der entstandene Vektor wurde wie oben beschrieben in XL-1 blue Zellen durch Elektroporation eingebracht. Der korrekte Einbau des Genes wurde von 12 nach der Transformation erhaltenen Klonen durch Sequenzierung mit dem Primer *pETTerm* kontrolliert. *E. coli Zellen* (BL21(DE3), + pUBS520) wurden anschließend mit dem Vektor der Affilin™-Variante SPC1-A7_Cys mit korrekter Sequenz transformiert. Nach Expression und Reinigung wie für die Affilin™-Variante SPC1-A7 (Beispiel 1) beschrieben, wurde für die Affilin™-Variante SPC1-A7_Cys wiederum wie oben beschrieben die freien Cystein-Reste mit Hilfe von Ellmann's-Reagenz titriert. Dabei konnte wie erwartet nur ein Cystein-Rest nachgewiesen werden, womit sich der erfolgreiche Einbau eines lösungsmittel-zugängliche C-terminalen Cysteins in ausreichender Entfernung zum Protein zur selektiven Kopplung an entsprechende Partner bestätigt (Tabelle 1). Dieser Nachweis eines einzelnen zugänglichen Cysteins stellt die Grundlage für die selektive Kopplung von Affilin™ an geeignete Kopplungspartner und Matrices dar.

Um das Affilin™ SPU11-3-A1 mit einem C-terminalen Cystein zu versehen, wurde das Gen für SPU11-3-A1 in den für das Affilin™ SPC1-A7 modifizierten pET20b (siehe oben) über die Restriktionsschnittstellen NcoI und XhoI einkloniert. Die Expression und Reinigung des Affilin™ SPU11-3-A1_Cys gestaltete sich identisch zur Vorgehensweise wie bei SPC1-A7_Cys.

### Beispiel 4

### Analyse der Bindungseigenschaften der mit einem Peptidlinker (C-terminales Cystein) modifizierten Affilin™-Variante SPC1-A7_Cys

Die Bindungseigenschaften des wie in Beispiel 3 gereinigten Afiilin™ SPC1-A7_Cys wurden in einem konzentrationsabhängigen ELISA getestet. Hierzu wurden die Vertiefungen einer NUNC-Platte mit 100 µl Antigenlösung (10 µg/ml humaner monoklonaler IgG Fc-Teil, Roche) über Nacht bei 4°C beschichtet. Die ELISA-Platte wurde am nächsten Tag mit PBS (3% BSA, 0,5% Tween 20) für 2 h bei Raumtemperatur geblockt. Nach Waschen der Vertiefungen mit PBS (0,1% Tween 20) wurde das modifizierte Affilin™ konzentrationsabhängig in die Vertiefungen gegeben (Konzentrationsbereich 10 µM - 0 µM) und für 1 h bei RT inkubiert. Nach erneutem Waschen der Vertiefungen mit PBS (0,1% Tween 20) wurde der monoklonale anti-hGC Antikörper (POD-konjugiert; Biogenes, Berlin) in einer Verdünnung von 1:1000 aufgetragen (50 µl/Vertiefung) und wieder für 1 h bei RT inkubiert. Danach wurden die Vertiefungen mit 3 x mit PBS (0,1 Tween 20) und 3 x PBS gewaschen und die Farbreaktion mit TMB Plus (Kementec, DK) initiiert (50 µl/Vertiefung). Nach 20 Minuten Inkubation bei Raumtemperatur wurde die Farbreaktion durch Zugabe von 0,2 M H₂SO₄ abgestoppt (50 µl/Vertiefung). Die erhaltene Gelbfärbung wurde bei 450 nm eingelesen (Referenzwellenlänge: 620 nm) und dokumentiert. (Abb.4) Die Auswertung der Messpunkte ergab einen apparenten KD-Wert von 233 nM, der etwa dem der unmodifizierten Affilin™ SPC1-A7 und SPC1-A7BB entspricht (280 nM). Somit hat die C-terminale Modifizierung des Affilin™ SPC1-A7 mit einem Peptidlinker, inklusive Cystein, keine Auswirkungen auf die Bindungsfähigkeit der Variante.

### Beispiel 5

### Selektive Kopplung des IgG bindenden Affilin™ SPC1-A7_Cys an Phycoerythrin (PE)

Die Kopplung des IgG-bindenden Affilin™ SPC1-A7_Cys an aktiviertes PE wurde wie folgt durchgeführt:
1 mg/ml SPC1-A7_Cys (in PBS) wurden mit 10 mM DTT für 30 min bei Raumtemperatur reduziert. Während der Reduktionsphase wurde eine PD-10 Säule (Amersham Biosciences) mit 5 Säulenvolumen PBS gespült. Nach erfolgter Reduktion wurde das Reaktionsgemisch auf die äquilibrierte PD-10 Säule gegeben, um überschüssiges DTT abzutrennen. Das so reduzierte SPC1-A7_Cys wurde im molaren Verhältnis von 5:1 mit Maleimid-aktiviertem Phycoerythrin (Prozyme) versetzt und für 1 h bei Raumtemperatur unter leichtem Schütteln inkubiert. Danach wurden freie Sulfhydrylgruppen des Affilin™, welche nicht reagiert hatten, durch Zugabe von NEM (N-ethylmaleimid) für 20 min bei RT geblockt. Das Reaktionsgemisch wurde anschließend über eine Gelfiltration (Sephadex S-200 HR) gereinigt und die entsprechenden Fraktionen vereinigt und bei 4°C gelagert. Die Analyse des Konjugates erfolgte spektroskopisch. Dazu wurden Absorptionsspektren im Bereich 250-750 nm aufgenommen und die Konzentrationen von PE und Affilin™ über die ermittelten bzw. mitgelieferten Extinktionskoeffizienten bestimmt. Das so erhaltenen Konjugat aus Affilin™ SPC1-A7_Cys und PE (SPC1-A7_Cys_PE) wurde im BIACORE auf seine Bindungseigenschaften gegenüber IgG-Fc untersucht. Dazu wurde bei einem kontinuierlichem Fluss von 30 µl/min und HBS-EP als Laufpuffer ein mit IgG-Fc gekoppelter CM5-Chip verwendet. Nacheinander wurden unterschiedliche Konzentrationen von SPC1-A7_Cys_PE über den Chip geleitet und die erhaltenen Sensogramme wurden mit der BIACORE-Evaluation Software analysiert. Dabei zeigte sich, dass durch die Kopplung ein Aviditätseffekt erhalten wurde, der sich in einer Erniedrigung der makroskopischen Dissoziationskonstante von K_{D}=10⁻⁷ M auf K_{D}=10⁻⁸ M äußerte (Fig.5).

### Beispiel 6

### Unspezifische Kopplung des Fluoreszenifarbstoffes Oyster^{®} 556 an die IgG bindende Affilin™ SPCI -A7BB

Der Fluoreszenzfarbstoff Oyster^{®} 556 (Molecular Probes) wurde an das IgG bindende Affilin™ SPC1-A7BB (ohne freies Cystein!) gekoppelt und Untersuchungen zur Bindung durchgeführt.

Die Kopplungsprozedur gestaltete sich wie folgt: 1 mg/ml SPC1-A7BB in 10 mM Phosphatpuffer (pH 8,5) wurden im molaren Verhältnis 1:2 mit dem Fluoreszenzfarbstoff Oyster^{®} 556 (in 20 µl trockenem DMF gelöst) versetzt und für 30 min bei RT inkubiert. Die Kopplungreaktion wurde durch Zugabe von 1 Volumen 10%-iger Glycinlösung abgestoppt und der Ansatz über eine PD-10 Säule gereinigt. Der Kopplungsgrad wurde im Anschluss daran spektroskopisch quantifiziert. Dazu wurde die Konzentration des Konjugates über die Absorption bei 280 nm bestimmt und mit einem mitgelieferten Korrekturfaktor (Molecular Probes) korrigiert. Der Kopplungsgrad ergab sich dann aus dem Quotienten aus den Konzentrationen von Oyster®556 und dem Konjugat und konnte mit 1 Molekül Affilin™/0.8 Molekül Oyster®556 bestimmt werden. Die Analyse der Bindungsfähigkeit des so erhaltenen Konjugates erfolgte durch einen konzentrationsabhängigen ELISA (Durchführung siehe Beispiel1), sowie durch Biacore Messungen (Abb.6). Es konnte gezeigt werden, dass die Bindungsfähigkeit des Affilin™ SPC1-A7BB nach Kopplung mit dem Fluoreszenzfarbstoff Oyster® nicht beeinträchtigt wird.

### Beispiel 7

### Unspezifische Kopplung des Enzyms Meerettich-Peroxidase (POD) an die IgG bindende Affilin™ SPCI -A7BB

Die Affilin™ Variante SPC1-A7BB konnte unspezifisch mit dem Enzym POD gekoppelt werden und Bindungsstudien zeigten den Erhalt der Bindungsaktivität des Affilin™ sowie der enzymatischen Aktivität der POD. Das Konjugat wurde nach folgendem Protokoll hergestellt:
5 mg lyophilisierte Meerettich-Peroxidase (POD, Sigma) wurden in 250 µl Reinstwasser gelöst, mit 37,5 µl 0,1 M Natriumperiodatlösung versetzt und 10 min bei 20 °C inkubiert. Danach wurden 25 µl Ethylenglycol dazugegeben und weitere 5 min bei 20 °C inkubiert. Die Peroxidase wurde durch Gelfiltration (G25, NAP-5 Säule) gegen Reinstwasser dialysiert.
250 µl gereinigtes Affilin™ SPC1-A7BB (IMAC, Gelfiltration, 4 mg/ml PBS) wurden mit 100 µl 0,1 M Carbonatpuffer (pH 9,6) versetzt und 1 mg aktivierte Peroxidase (Sigma) hinzugefügt (etwa 100 µl). Das Kopplungsgemisch wurde unter Rühren 2 h bei 20 °C inkubiert. Anschließend wurde pro ml Kopplungsgemisch 10 µl 0,5 M Natriumborhydrid dazugeben, kurz gemischt und für weitere 2 h bei 4 °C ohne Rühren inkubiert. Der Reaktionsansatz wurde gegen PBS mit einer G25-Säule umgepuffert. Zur Konservierung wurden 0,1% Thiomersal (Roth) zugesetzt. Die Untersuchung der Bindungsaktivität des Konjugates an human-IgG gestaltete sich wie folgt:
   Das markierte Affilin™ wurde in PBS (0,5 % BSA, 0,05 % Tween 20, 0,01 % Thiomersal) verdünnt und die Lösungen auf eine mit humanem IgG beschichtete Mikrotiterplatte aufgetragen (10 µg/ml, 100 µl/ml). Die Inkubationszeit betrug 1 h bei Raumtemperatur. Anschließend wurden die Vertiefungen 3mal mit je 250 µl PBS (0,1% Tween 20, 0,01 % Thiomersal) gewaschen und mit 100 µl TMB erneut 10 - 20 min bei Raumtemperatur inkubiert. Die Reaktion wurde durch Zugabe von 100 µl 0,5 M Schwefelsäure beendet. Die Absorption (450 nm gegen 620 nm Referenz) wurde in einem Mikrotiterplattenphotometer gemessen (Abb.7). Es konnte bis zu einer Verdünnung von 1:10000 des Reaktionsansatzes POD Aktivität nachgewiesen werden, was die erfolgreiche Kopplung von POD an SPC1-A7BB beweist. Durch Kontrollmessungen konnte ausgeschlossen werden, das ungekoppelte POD die Signale verfälscht.

### Beispiel 8

### Spezifische und unspezifische Kopplung von gamma-II-Kristallin und Ubiquitin basierter Affilin™ an Matrices

Die Kopplung von Affilin™ an Matrices konnte mit den folgenden Methoden realisiert werden:
1.) Kopplung des Affilin™ SPU3-A1_Cys über ein C-terminales Cystein an eine Dextranmatrix 2.) Kopplung des Affilin™ SPC7-E9 über primäre Aminogruppen an die Dextranmatrix des BIACORE Systems und 3.) Unspezifische Kopplung des Affilin™ SPC7-E9 mit Hilfe von EDC/NHS an eine Polymethacrylat-Matrix.
1.) Die Kopplung von SPU3-A1_Cys an die Dextranmatrix des BIACORE Systems wurde selektiv über das eingeführte C-terminale Cystein realisiert. Dazu wurden die Carboxylgruppen der Dextranmatrix mit NHS/EDC bei einer Kontaktzeit von 2 min aktiviert und anschließend mit dem Thiol-Kopplungsreagenz PDEA (2-(2-pyridinyldithio)ethanamin, in 0,1 M Boratpuffer pH 8,5) versetzt. Nach einer Reaktionszeit von 4 min wurde gereinigtes SPU3-A1_Cys (in 20 mM Phosphatpuffer, pH 6,0) auf den so modifizierten Dextran-Chip gegeben und die Reaktion für 7 min weitergeführt. Die Deaktivierung von nicht umgesetzten PDEA-Gruppen wurde mit 50 mM L-Cystein (1 M NaCl) für 4 min durchgeführt. Mit dieser Methode konnten 350 Einheiten (RU) SPU3-A1_Cys auf dem Chip immobilisiert und für weitere kinetische Analysen genutzt werden. Nach kinetischen Messungen wurde der Chip mit 0,1 Glycin (pH 2,2), 6 M Gua/HCl, 6 M Harnstoff und 20% Ethanol regeneriert. Die Bindungsaktivität des Affilin™-Chips blieb auch nach 20 - 30 Regeneartionszyklen unverändert (Abb.8).
2.) Weiterhin konnte SPC7-E9 unspezifisch über oberflächenexponierte Aminogruppen (Lysine) mit Hilfe von NHS/EDC an die Carboxylgruppen der BIACORE-Dextranmatrix folgendermaßen gekoppelt werden: Der CM5-Chip wurde für 7 min mit NHS/EDC aktiviert und im Anschluss wurde gereinigtes SPC7-E9 (in 20 mM Na-Phosphatpuffer, pH 6,0) für weitere 7 min über den Chip geleitet. Nach erfolgter Kopplung wurden die restlichen reaktiven Gruppen mit 1M Ethanolamin (pH 8,5) für 7 min deaktiviert. Zur Analyse der Dissoziationskonstanten wurde das Target proNGF in verschiedenen Konzentrationen über den Chip geleitet, die Bindung online verfolgt (Abb.9) und die Kurven anschließend mit der BiaEvaluation Software ausgewertet. Der K_{D}-Wert konnte so mit 1,4 nM bestimmt werden. Nach kinetischen Messungen wurde der Chip mit 0,1 Glycin (pH 2,2), 10 mM HCl, 10 mM NaOH, 6M Gua/HCl, 6 M Harnstoff und 20% Ethanol regeneriert. Die Bindungsaktivität des Affilin™-Chips blieb auch nach mehreren Regeneartionszyklen unverändert.
3.) Gereinigtes SPC7-E9 Protein (4 mg) wurde über eine PD-10 Säule (Amersham) in 0,1 M BoratPuffer (0,5 M Na₂SO₄, pH 9) umgepuffert und an Fractogel® EMD Epoxy (M) gekoppelt. Dazu wurde das Gel (0,5 g) in 0,1 M Boratpuffer (0,5 M Na₂SO₄, pH 9) für 2h bei RT inkubiert und danach mehrmals mit diesem Puffer gewaschen. Die Kopplungsreaktion wurde durch Zugabe von SPC7-E9 zur Epoxy-Matrix initiiert und bei RT für 24 h unter kontinuierlichem Rühren weitergeführt. Eine Referenzsäule ohne SPC7-E9 wurde ebenfalls als Kontrolle mitgeführt und identisch behandelt. Mit Hilfe von 1M Ethanolamin (pH 9,5) wurde die Reaktion für 48 h bei RT abgestoppt und die Gelmatrix mit Natrium-acetat Puffer (0,1 M, pH 4,0), 1M NaCl und PBS (je 50 Säulenvolumen) gewaschen. Die so erzeugte Affilin™ SPC7-E9 - Affinitätsmatrix wurde in eine C-Säule (Amersham Biosciences, 1 x 10 cm) gefüllt und an eine Chromatographieanlage (Äkta Explorer, Amersham Biosciences) angeschlossen. Als Laufpuffer wurde in allen Fällen PBS (0,5 mM EDTA) bei einer Flussrate von 1 ml/min verwendet. Zum Test der Bindungsfähigkeit der so erzeugten Affilin™ -Säule wurde gereinigtes proNGF appliziert. Nach Spülen der Säule mit 10 - 20 Säulenvolumen Laufpuffer wurde gebundenes proNGF mit 0,1 M Glycin (pH 2,2) eluiert (Abb. 10). Die Separation von proNGF gelang weiterhin aus Substanzgemischen mit BSA und aus *E.coli* Rohextrakt. Dazu wurde 1 ml BSA-Lösung (5 mg/ml, Sigma) mit 0,5 ml proNGF (1,3 mg/ml) gemischt und auf die Affilin™ Säule appliziert. Nach Spülen der Säule mit 20 Säulenvolumen Laufpuffer wurde gebundenes proNGF mit Glycin (0,1 M, pH 2,2) eluiert. Nach einer Regeneration der Säule mit 10 Säulenvolumen 6 M Gua/HCl wurde des weiteren ein Gemisch aus 1 ml *E.coli* Rohextrakt (löslicher Überstand nach Aufschluss (Lysozym/Benzonase/Ultraschall) des Bakterienpellets von 50 ml B121 Übernachtkultur) und 0,5 ml proNGF (1,3 mg/ml) auf die Affilin™ -Säule aufgetragen. Nach Spülen der Säule mit 20 Säulenvolumen Laufpuffer wurde gebundenes proNGF ebenfalls mit Glycin (0,1 M, pH 2,2) eluiert Die Säule wurde anschließend mit 0,1 M Glycin (pH 2.2), 10 mM HCl, 10 mM NaOH, 6 M Gua/HCl, 6 M Harnstoff und 20% Ethanol regeneriert. Die eluierten Fraktionen aus der Separation von proNGF von BSA und *E.coli* Rohextrakt wurden mittels Gelelektrophorese analysiert (Abb. 11). Nach 10 Testläufen konnte eine unveränderte Bindung von proNGF an die SPC7-E9-Säule beobachtet werden.

### Anhang

**Tabelle 1:**

| **Variante** | **A₄₁₀** | **Leerwert1** | **Leerwert2** | **Korr. A₄₁₀** | **Eingesetzte Konzentration [µg/ml]** | **Anzahl Cysteine im Protein** | **Theor. zugängliche Cysteine** | **Anzahl titriert Cysteine** |
|---|---|---|---|---|---|---|---|---|
| SPC-1-A7BB | 0,2142 | 0,0897 | 0,1232 | 0,0013 | 50 | 7 | 0 | 0 |
| | 0,2254 | 0,1003 | 0,1232 | 0,0019 | 100 | | | |
| SPC-1-A7JJ | 0,3154 | 0,0832 | 0,1232 | 0,109 | 50 | 8 | 1 | 4 |
| | 0,4412 | 0,0738 | 0,1232 | 0,2442 | 100 | | | |
| SPC-1-A7_Cys | 0,235 | 0,0834 | 0,1232 | 0,0286 | 50 | 8 | 1 | 1 |
| | 0,272 | 0,0966 | 0,1232 | 0,0522 | 100 | | | |

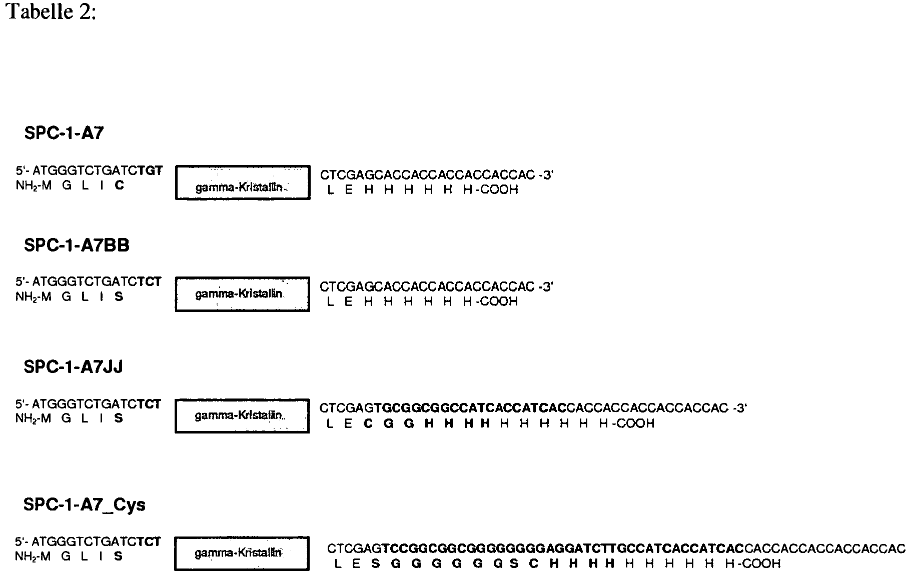

### DNA-Sequenz der humanen gamma-II-Kiistallin Bibliothek

CR20 (SEQ ID NO:1)

### DNA-Sequenzen gamma-II-Kristallin basierter Affilin™

SPC1-A1 (SEQ ID NO:2)
SPC1-A7 (SEQ ID NO:3)
SPC1-G3 (SEQ ID NO:4)
SPC1-A7BB (SEQ ID NO:5)
SPC1-A7JJ (inklusive His10) (SEQ ID NO:6)
SPC1-A7_Cys (inklusive His10) (SEQ ID NO:7)
SPC7-E9 (inklusive His6) (SEQ ID NO:8)

### DNA-Sequenz der humanen Ubiquitin Bibliothek

Ubiquitin Wildtyp (SEQ ID NO:9)
Modifiziertes Ubiquitin (MUBI) (SEQ ID NO:10)
UB10 (Bibliothek) (SEQ ID NO:11)

### DNA-Sequenzen Ubiquitin basierter Affilin™

SPU11-3-A1 (inklusive His6) (SEQ ID NO:12)
SPU11-3-A1_Cys (inklusive His10) (SEQ ID NO:13)
Primer
   *pCAN700 (5'-CCA TGA TTA CGC CAA GCT TTG GAG CC-3')* (SEQ ID NO:14)
   *A7Cys4Ser_for* (5'-CCA TGG GTC TGA TCT CTT TCT CTG AAG ACC G-3') (SEQ ID NO:15)
   *A7Cys4Ser_rev* (5'-CGG TCT TCA GAG AAA GAG ATC AGA CCC ATG G-3') (SEQ ID NO:16)
   *pETTerm* (5'-GCT AGT TAT TGC TCA GCG GTG GC-3') (SEQ ID NO:17)
   *A7Gly2Ser_for* (5'-GGA TTT GTA CCT CGA GTC CGG CGG CCA TCA CC-3') (SEQ ID NO:20) und A7Gly2Ser rev (5'-GGT GAT GGC CGC CGG ACT CGA GGT ACA AAT CC-3') (SEQ ID NO:21)
   *A7Gly2Ser_rev* (5'-GGT GAT GGC CGC CGG ACT CGA GGT ACA AAT CC-3') (SEQ ID NO:22)
   *A7Cys4Ser_Nde* (5'-GGA GAT ATA CAAT ATG GGT CTG ATC TCT TTC TCT G-3') (SEQ ID NO:24)
SEQ ID NO: 25: TACAACATCC AAAAAGAATC CACCCTCCAC CTGGCACTCC TCCTGCGGGC C 291
SEQ ID NO: 26
   ATGCAAATCT TCGTTAAAAC CCTGACGGGA AAGACTATCA CCCTGGAGGT 50
SEQ ID NO: 27
   GGATTTTAGC TTTGACATTT TCGATGGTGT CGGACGGTTC TACCTCCAGG GTG 53
SEQ ID NO: 28
   GTCAAAGCTA AAATCCAAGA CAAAGAAGGA ATTCCACCTG ACCAGCAACG CCT 53
SEQ ID NO: 29
   GGGTGAGCCC GTCCTCTAGT TGTCGTCCTG CGAAAGCTAG GCGTTGCTGG 50
SEQ ID NO: 30
   GACGGGCTCA CCCTGTCTGA CTACAACATC CAAAAAGAAT CCACCCTCCA 50
SEQ ID NO: 31
   GAGTGCTCGC AGCAGGAGTG CCAGGTGGAG GGTGGATTC 39
SEQ ID NO: 32
   GATATACATA TGCAAATCTT CG 22
SEQ ID NO: 33
   GTGGTGCTCG AGTGCTCG 18
SEQ ID NO: 34
   CCAGCCGGCC ATGGCCATGN NKATCNNKGT TNNKACCCTG ACGGGAAAGA CTATC 55
SEQ ID NO: 35
   CAGGAGGAGT GCCAGGTGGA GMNNMNNMNN MNNMNNGATG TTGTAGTCAG ACAGG 55
SEQ ID NO: 36
   GTTATTACTC GCGGCCCAGC CGGCCATGGC CATG 34
SEQ ID NO: 37
   GAGTTTTTGT TCGGCCTCGA GGGCCCGCAG GAGGAGTGCC AGGTGGAG 48

### Zitierte Literatur

Ausuebel, F.M., Brent, R., Kinston, R.E., Moore, D.D., Seidmann, J.G., Smith, J.A. and Struhl, K. (1994): Current protocols in molecular biology. John Wiley & Sons, Inc.
Berman, H. M., Westbrook, J., Feng, Z., Gilliland, G., Bhat, T. N., Weissig, H., Shindyalov, I. N. und Bourne, P. E. (2000) The Protein Data Bank. Nucleic Acid Res., 28, 235-242.
Blundell, T., Lindley, P., Miller, L., Moss, D., Slingsby, C., Tickle, I., Turnell, B., and Wistow, G. (1981). The molecular structure and stability of the eye lens: x-ray analysis of gamma-crystallin II. Nature 289, 771-777.
Buchberger A, Howard MJ, Proctor M, Bycroft M, National Library of Medicine, J Mol Bil. 2001 Mr 16; 307(1); 17-24.
Butt, T. R., Jonnalagadda, S., Monia, B. P., Sternberg, E. J., Marsh, J. A., Stadel, J. M., Ecker, D. J. and Crooke, S. T. (1989) Ubiquitin fusion augments the yield of cloned gene products in Escherichia coli. PNAS 86, 2540-2544.
Finucane, M. D., Tuna, M., Lees, J. H., and Woolfson, D. N. (1999). Core-directed protein design. I. An experimental method for selecting stable proteins from combinatorial libraries. Biochemistry 38, 11604-11612.
Finucane, M. D., and Woolfson, D. N. (1999). Core-directed protein design. II. Rescue of a multiply mutated and destabilized variant of ubiquitin. Biochemistry 38, 11613-11623.
Haber, A. F. S. A. (1972). Reaction of protein sulfhydryl groups with Ellman's reagent. In Methods Enzymology, C. H. Hirs, and S. N. Timasheff, eds., pp. 457-464.
Hanes, J. et al. (1997): In vitro selection and evolution of functional proteins by using ribosome display. Proc. Natl. Acad. Sci. USA. 94, 4937-42.
Hazes, B. and Hol, W. G. J. (1992): Comparison of the hemocyanin β-barrel with other greek key β-barrels: possible importance of the "β-zipper" in protein structure and folding. Proteins: Struct., Funct. Gen. 12, 278-298.
Hemmingsen, J. M., Gernert, K. M., Richardson, J. S. and Richardson, D. C. (1994): The tyrosine corner: a feature of most greek key β-barrel proteins. Prot. Science 3, 1927-1937.
Herrmann, J. E., and Morse, S. A. (1973) Coupling of peroxidase to poliovirus antibody: Characteristics of the conjugates and their use in virus detection. Infection and Immunity, 645-649.
Jaenicke, R. (1994). Eye-lens proteins: structure, superstructure, stability, genetics. Naturwissenschaften 81, 423-429.
Jaenicke, R. (1996). Stability and folding of ultrastable proteins: eye lens crystallins and enzymes from thermophiles. Faseb J 10, 84-92.
Jaenicke, R., and Slingsby, C. (2001). Lens crystallins and their microbial homologs: structure, stability, and function. Crit Rev Biochem Mol Biol 36, 435-499.
Kumaraswamy, V. S., Lindley, P. F., Slingsby, C., and Glover, I. D. (1996). An eye lens protein-water structure: 1.2 angstrom resolution structure of gamma B-crystallin at 150K. Acta Crystallogr D Biol Crystallogr 52, 611.
Larsen CN, Wang H., National Library of Medicine ; J Proteome Res. 2002 Sep-Oct; 1(5): 411-9.
Lazar, G. A., Desjarlais, J. R., and Handel, T. M. (1997). De novo design of the hydrophobic core of ubiquitin. Protein Sci 6, 1167-1178.
Ling, M. M. (2003). Large antibody display libraries for isolation of high-affinity antibodies. Comb Chem High Throughput Screen 6, 421-432.
Lottspeich, F., and Zorbas, H. (1998). Bioanalytik (Heidelberg, Spektrum Akademischer Verlag).
Mandal, K., Chakrabart, B., Thomson, J. and Siezen, R. J. (1987): Structure and stability of β-crystallins. Denaturation and proteolysis behaviour. J. Biol. Chem. 262, 8096-8102.
Mayr, E. M., Jaenicke, R., and Glockshuber, R. (1994). Domain interactions and connecting peptides in lens crystallins. J Mol Biol 235, 84-88.
Murzin A. G., Brenner S. E., Hubbard T. und Chothia C. (1995). SCOP: a structural classification of proteins database for the investigation of sequences and structures. J. Mol. Biol. 247, 536-540.
Najmudin, S., Nalini, V., Driessen, H. P., Slingsby, C., Blundell, T., Moss, D., and Lindley, P. (1993). Structure of bovine gB (g II)-crystalline at 1.47 Ä. Acta Crystallogr D Biol Crystallogr 49, 223-233.
Norledge, B. V., Mayr, E. M., Glockshuber, R., Bateman, O. A., Slingsby, C., Jaenicke, R., and Driessen, H. P. (1996). The X-ray structures of two mutant crystallin domains shed light on the evolution of multi-domain proteins. Nat Struct Biol 3, 267-274.
Reichlin, M. (1980) Use of glutaraldehyde as a coupling agent for proteins and peptides. Methods Enzymol 70, 159-165.
Richardson, J. S., Richardson, D. C., Tweedy, N. B., Gernert, K. M., Quinn, T. P., Hecht, M. H., Erickson, B. W., Yan, Y., McClain, R. D., Donlan, M. E. and Surles, M. C. (1992): Looking at proteins: representations, folding, packing and design. Biophys. J. 63, 1186-1209.
Riddle, D. S., Santiago, J. V., Bray-Hall, S. T., Doshi, N., Grantcharova, Q. Y and Baker, D. (1997): Functional rapidly folding proteins from simplified amino acid sequences. Nature structural biology 4, 805-809.
Rudolph, R., Siebendritt, R., Nesslauer, G., Sharma, A. K., and Jaenicke, R. (1990). Folding of an all-beta protein: independent domain folding in gamma II-crystallin from calf eye lens. Proc Natl Acad Sci U S A 87, 4625-4629.
Sambrook, J., Maniatis, T. and Fritsch, E.F. (1989): Molecular Cloning: A laboratory manual. Cold spring Harbor. Cold Spring Harbour Laboratory Press, New York.
Sharma, A. K., Minke-Gogl, V., Gohl, P., Siebendritt, R., Jaenicke, R., and Rudolph, R. (1990). Limited proteolysis of gamma II-crystallin from calf eye lens. Physicochemical studies on the N-terminal domain and the intact two-domain protein. Eur J Biochem 194, 603-609.
Slingsby, C.(1985): Structural variation in lens crystallins. TIBS 10, 281-284.
Smith, G. P (1985): Filamentous Fusion Phage: Novel expression vectors that display cloned antigens on the virion surface. Science 228, 1315-1317.
Stahl, S. and Uhlen, M. (1997): Bacterial surface display: trends and progress. TIBTECH 15, 185-192.
Takamiya, H., Shimizu, F., and Vogt, A. (1975) A two-stage method for cross-linking antibody globulin to ferritin by glutaraldehyde.III. Size and antibody activity of the conjugates. J Immunol Methods 8(4), 301-306.
Vijay-Kumar, S., Bugg, C. E., and Cook, W. J. (1987). Structure of ubiquitin refined at 1.8 A resolution. J Mol Biol 194, 531-544.
Voet, D., and Voet, J. G. (1995). Biochemistry, Second Edition edn (New York, John Wiley & Sons, Inc.).
Wistow, G., Turnell, B., Summers, L., Slingsby, C., Moss, D., Miller, L., Lindley, P., and Blundell, T. (1983). X-ray analysis of the eye lens protein gamma-II crystallin at 1.9 A resolution. J Mol Biol 170, 175-202.
Wistow, G. J., and Piatigorsky, J. (1988). Lens crystallins: the evolution and expression of proteins for a highly specialized tissue. Annu Rev Biochem 57, 479-504.

## Patentansprüche

1. Verfahren zur Herstellung eines Konjugats das die folgenden Bestandteile umfasst:
ein oder mehrere auf humanem Ubiquitin basierende Polypeptidmoleküle (I),
und, damit kovalent verknüpft, einen oder mehrere funktionelle Bestandteile (II) ausgewählt aus der Gruppe, die aus Polypeptiden und Proteinen, organischen Polymeren, Zuckern, niedermolekularen Substanzen, Peptiden, sowie Derivaten dieser Substanzen, besteht,
wobei nach Kopplung von (I) an (II) die Funktionalität aller Bestandteile erhalten bleibt,
und wobei das Verfahren folgende Schritte umfasst:
a) Bereitstellen von humanem Ubiquitin;
b) Bereitstellen eines liganden ansgewähtt aus (Poly) Peptiden Proteins
c) Auswählen der Aminosäuren 2, 4, 6, 62, 63, 64, 65 und 66 des humanen Ubiquitins;
d) Modifizieren der ausgewählten Aminosäuren durch Substitution unter Beibehaltung des Ubiquitin-artigen
e) Faltungsmotivs, modifizierten Proteins mit dem in Schritt b) bereitsgestellte liganden
f) Ermitteln derjenigen Proteine, die zu sperifischen Bindug an den liganden mit eine Dissoriationskanstante van K_{D}=10⁻⁵ M oder kleine geignet sud, Wobei der Nachweis der Bindugsabstiontät über ELISA-Technik oder obeflädrenplasmonsesonaur erfolgt,
g) Kopplung der Bestandteile (I) und (II) in einem Bereich außerhalb der oberflächenexponierten Region des β-Faltblattes des Polypeptidmoleküls (I), die zur spezifischen Bindung an den Liganden vorgesehen ist, über eine terminale Peptidfusion an (I), oder über Cystein- oder Lysinseitenketten in (I);
h) Isolierung des Konjugates; und
i) Nachweis der Funktionalität beider Komponenten des Konjugates.

2. Verfahren zur Herstellung eines wie in Anspruch 1 definierten Konjugats, ausgehend von Bestandteil (I) mit bekannter Sequenz, das folgende Schritte umfasst:
- Identifizierung für die Kopplung geeigneter Aminosäurereste durch Analyse der Raumstruktur des Proteins, bevorzugt von Resten außerhalb der Wechselwirkungsfläche von (I) mit dem Liganden;
- Aktivierung eines Kopplungspartners durch ein geeignetes Kopplungsreagenz;
- Durchführung der Kopplungsreaktion;
- Isolierung des Konjugates; und
- Nachweis der Funktionalität beider Komponenten des Konjugates.

3. Verfahren zur Herstellung eines wie in Anspruch 1 definierten Konjugates, ausgehend von Bestandteil (I) mit bekannter Sequenz, in dem keine für die Kopplung geeigneten Aminosäurereste identifiziert wurden, das folgende Schritte umfasst:
- Einführung für die Kopplung geeigneter Aminosäurereste durch Substitution, Insertion oder Fusion, bevorzugt oberflächenexponierter Reste außerhalb der Wechselwirkungsfläche von (I) mit dem Liganden;
- Nachweis der Zugänglichkeit der eingeführten Aminosäurereste;
- Nachweis der Funktionalität des derart veränderten Bestandteils (I);
- Aktivierung eines Kopplungspartners durch ein geeignetes Kopplungsreagenz;
- Durchführung der Kopplungsreaktion;
- Isolierung des Konjugates; und
- Nachweis der Funktionalität beider Komponenten des Konjugates.

4. Verfahren nach Anspruch 1, wobei die Kopplung über die Lysinreste 11, 29, 33 und 48 des Ubiquitinmoleküls erfolgt.

5. Verfahren nach Anspruch 2, wobei die zusätzliche terminale Peptidfusion an (I) einen oder mehrere Cysteinreste oder einen oder mehrere Lysinreste enthält, wobei diese Aminosäurereste vorzugsweise nicht an der Wechselwirkung von (I) mit dem Liganden beteiligt sind.

6. Verfahren nach einem oder mehreren der Ansprüche 1-5, wobei der funktionelle Bestandteil (II) ein Peptid, Polypeptid oder ein Protein, bevorzugt ein Protein-Chromophor, ein Enzym, ein Immunglobulin, ein Immunglobulin-Derivat, ein Toxin oder ein Polypeptid gemäß I, ist.

7. Verfahren nach einem oder mehreren der Ansprüche 1-5, wobei der funktionelle Bestandteil (II) ein Polymer, bevorzugt Dextran, Polymethacrylat, Sepharose, Agarose, Polyvinyl, Polystyren, Kieselgel, Cellulose, Polyethylenglykol, oder ein Polymerderivat ist.

8. Verfahren nach einem oder mehreren der Ansprüche 1-5, wobei der funktionelle Bestandteil (II) eine niedermolekulare Substanz, bevorzugt ein Farbstoff, Biotin, Digoxigenin, ein Schwermetall, ein Chelatbildner, ein Radioisotop, ein Antibiotikum oder eine cytotoxische Substanz ist.

9. Verfahren nach einem oder mehreren der vorhergehenden Ansprüche, wobei der Bestandteil (I) eine neu generierte Bindungseigenschaft zur spezifischen Bindung an einen Liganden aufweist, der ein Polypeptid oder ein Protein ist, bevorzugt Immunglobuline und Immunglobulinderivate, aus Blutplasma gewonnene Proteine, Blutgerinnungsfaktoren- und - inhibitoren, Wachstumsfaktoren, Interleukine, Cytokine, Rezeptorproteine, Glycoproteine, virale Proteine und Oberflächenzellmarker, bevorzugt CD 14, CD25, CD34.

10. Verfahren nach einem oder mehreren der vorhergehenden Ansprüche, wobei der Bestandteil (I) eine neu generierte Bindungseigenschaft zur spezifischen Bindung an einen Liganden aufweist, der ein Peptid ist, bevorzugt Affinitätsanhängsel, vorzugsweise S-Tag, T7-Tag, His-Tag, Strep-Tag, Myc-Tag, oder FLAG-Tag, oder Peptide viralen Ursprungs.

11. Verfahren nach einem oder mehreren der vorhergehenden Ansprüche, wobei der Bestandteil (I) eine neu generierte Bindungseigenschaft zur spezifischen Bindung an einen Liganden aufweist, der eine niedermolekulare Substanz ist, bevorzugt Steroide, Cholesterol und Schadstoffe, wie beispielsweise halogenierte Kohlenwasserstoffe.

12. Verfahren nach einem oder mehreren der vorhergehenden Ansprüche, wobei der Bestandteil (II) ein oder mehrere auf Ubiquitin basierende Polypeptide sind, die mit (I) identisch sind, und mit diesem kovalent verknüpft sind, wodurch eine Steigerung der Affinität zu dem Liganden von (I) über Aviditätseffekte erreicht wird.

13. Verfahren nach einem oder mehreren der vorhergehenden Ansprüche, wobei der Bestandteil (II) ein Polypeptid, Protein oder Polymer ist, an das Bestandteil (I) mehrfach kovalent verknüpft ist, wodurch eine Steigerung der Affinität zu dem Liganden von (I) über Aviditätseffekte erreicht wird.

14. Verfahren nach einem oder mehreren der vorhergehenden Ansprüche, wobei der Bestandteil (II) ein Polypeptid oder Polymer ist, das nach kovalenter Verknüpfung mit Bestandteil (I) kovalente oder nicht kovalente Bindung mit weiteren Konjugaten dieser Art eingeht, wodurch eine Steigerung der Affinität zu dem Liganden von (I) über Aviditätseffekte erreicht wird.

15. Verfahren nach einem oder mehreren der vorhergehenden Ansprüche, wobei der Bestandteil (I) SPU11-3-A1 (SEQ ID NO: 12 und 13) ist.

16. Verfahren nach einem oder mehreren der vorhergehenden Ansprüche, das weiterhin die Herstellung eines diagnostischen Kits mit einem nach einem oder mehreren der Ansprüche 1-15 hergestellten Konjugats umfasst.

17. Verfahren nach einem oder mehreren der vorhergehenden Ansprüche, das weiterhin die Herstellung einer pharmazeutischen Zusammensetzung mit einem nach einem oder mehreren der Ansprüche 1-15 hergestellten Konjugat umfasst.

18. Verfahren nach einem oder mehreren der vorhergehenden Ansprüche, das weiterhin die Herstellung einer Zusammensetzung für die Affinitätsanreicherung mit einem nach einem oder mehreren der Ansprüche 1-15 hergestellten Konjugat umfasst, wobei der funktionelle Bestandteil eine Membran, Polymerkügelchen oder ein chromatographisches Trägermaterial ist.

## Claims

1. Method for producing a conjugate comprising the following components:
one or more polypeptide molecules (I) based on human ubiquitin and, covalently linked thereto,
one or more functional components (II) selected from the group consisting of polypeptides and proteins, organic polymers, sugars, low-molecular-weight substances, peptides and derivatives of these substances,
wherein, after coupling of (I) to (II), the functionality of all components is retained,
and wherein the method comprises the following steps:
a) providing human ubiquitin;
b) providing a ligand selected from (poly)peptides/proteins;
c) selecting the amino acids 2, 4, 6, 62, 63, 64, 65 and 66 of the human ubiquitin;
d) modifying the selected amino acids by substitution whilst retaining the ubiquitin-like folding motif,
e) bringing the modified protein into contact with the ligand provided in step b);
f) determining those proteins which are suitable for specific binding to the ligand with a dissociation constant of K_{D} = 10⁻⁵ M or less, the binding activity being detected by means of the ELISA technique or surface plasmon resonance,
g) coupling the components (I) and (II) in a region outside of the surface-exposed region of the β sheet of the polypeptide molecule (I), provided for specific binding to the ligand, via a terminal peptide fusion to (I) or via cysteine or lysine side chains in (I);
h) isolating the conjugate; and
i) detecting the functionality of the two components of the conjugate.

2. Method for producing a conjugate as defined in claim 1, starting from component (I) having a known sequence, comprising the following steps:
- identifying amino acid residues suitable for coupling by analysing the spatial structure of the protein, preferably of residues outside the surface of interaction of (I) with the ligand;
- activating a coupling partner by means of a suitable coupling reagent;
- performing the coupling reaction;
- isolating the conjugate; and
- detecting the functionality of the two components of the conjugate.

3. Method for producing a conjugate as defined in claim 1, starting from component (I) having a known sequence, in which no amino acid residues suitable for coupling were identified, comprising the following steps:
- introducing amino acid residues suitable for coupling by substitution, insertion or fusion, preferably surface-exposed residues outside of the surface of interaction of (I) with the ligand;
- detecting the accessibility of the introduced amino acid residues;
- detecting the functionality of the component (I) altered in this manner;
- activating a coupling partner by means of a suitable coupling reagent;
- performing the coupling reaction;
- isolating the conjugate; and
- detecting the functionality of the two components of the conjugate.

4. Method according to claim 1, wherein the coupling is performed via the lysine residues 11, 29, 33 and 48 of the ubiquitin molecule.

5. Method according to claim 2, wherein the additional terminal peptide fusion to (I) contains one or more cysteine residues or one or more lysine residues, these amino acid residues preferably not being involved in the interaction of (I) with the ligand.

6. Method according to one or more of claims 1 to 5, wherein the functional component (II) is a peptide, polypeptide or a protein, preferably a protein chromophore, an enzyme, an immunoglobulin, an immunoglobulin derivative, a toxin or a polypeptide according to 1.

7. Method according to one or more of claims 1 to 5, wherein the functional component (II) is a polymer, preferably dextrane, polymethacrylate, sepharose, agarose, polyvinyl, polystyrene, silica gel, cellulose, polyethylene glycol, or a polymer derivative.

8. Method according to one or more of claims 1 to 5, wherein the functional component (II) is a low-molecular-weight substance, preferably a dye, biotin, digoxigenin, a heavy metal, a chelating agent, a radioisotope, an antibiotic or a cytotoxic substance.

9. Method according to one or more of the preceding claims, wherein the component (I) has a newly generated binding property for specific binding to a ligand which is a polypeptide or a protein, preferably immunoglobulins and immunoglobulin derivatives, proteins obtained from blood plasma, blood-clotting factors and inhibitors, growth factors, interleukins, cytokins, receptor proteins, glycoproteins, viral proteins and cell surface markers, preferably CD14, CD25 and CD34.

10. Method according to one or more of the preceding claims, wherein the component (I) has a newly generated binding property for specific binding to a ligand which is a peptide, preferably affinity tag, preferably S-Tag, T7-Tag, His-Tag, Strep-Tag, Myc-Tag, or FLAG-Tag, or peptides of viral origin.

11. Method according to one or more of the preceding claims, wherein the component (I) has a newly generated binding property for specific binding to a ligand which is a low-molecular-weight substance, preferably steroids, cholesterol and noxious substances, for example halogenated hydrocarbons.

12. Method according to one or more of the preceding claims, wherein the component (II) is one or more polypeptides based on ubiquitin which are identical to (I) and are covalently linked thereto, whereby an increase of the affinity for the ligand of (I) is achieved through avidity effects.

13. Method according to one or more of the preceding claims, wherein the component (II) is a polypeptide, protein or polymer to which component (I) is covalently linked multiple times, whereby an increase of the affinity for the ligand of (I) is achieved through avidity effects.

14. Method according to one or more of the preceding claims, wherein the component (II) is a polypeptide or polymer which, after covalent linkage to component (I), undergoes covalent or non-covalent binding to other conjugates of this type, whereby an increase of the affinity for the ligand of (I) is achieved through avidity effects.

15. Method according to one or more of the preceding claims, wherein the component (I) is SPU11-3-A1 (SEQ ID NO: 12 and 13).

16. Method according to one or more of the preceding claims, further comprising the production of a diagnostic kit comprising a conjugate produced according to one or more of claims 1 to 15.

17. Method according to one or more of the preceding claims, further comprising the production of a pharmaceutical composition comprising a conjugate produced according to one or more of claims 1 to 15.

18. Method according to one or more of the preceding claims, further comprising the production of a composition for affinity enrichment comprising a conjugate produced according to one or more of claims 1 to 15, wherein the functional component is a membrane, polymer bead or a chromatographic support material.

## Revendications

1. Procédé de fabrication d'un conjugué comprenant les composants suivants :
une ou plusieurs molécules polypeptidiques (I) basées sur l'ubiquitine humaine, et, liés à celle-ci de manière covalente,
un ou plusieurs composants fonctionnels (II) choisis dans le groupe formé par les polypeptides et les protéines, les polymères organiques, les sucres, les substances de faible masse moléculaire, les peptides, ainsi que des dérivés de ces substances,
dans lequel, après le couplage de (I) à (II), la fonctionnalité de tous les composants reste intacte,
et dans lequel le procédé comprend les étapes suivantes :
a) mise à disposition d'ubiquitine humaine ;
b) mise à disposition d'un ligand choisi parmi les (poly)peptides/protéines ;
c) sélection des acides aminés 2, 4, 6, 62, 63, 64, 65 et 66 de l'ubiquitine humaine ;
d) modification des acides aminés sélectionnées par substitution avec conservation du motif de repliement de type ubiquitine ;
e) mise en contact de la protéine modifiée avec le ligand mis à disposition à l'étape b) ;
f) détermination des protéines appropriées pour la liaison spécifique au ligand avec une constante de dissociation K_{D} = 10⁻⁵ M ou inférieure, la démonstration de l'activité de liaison étant réalisée au moyen de la technique ELISA ou par résonance plasmonique de surface ;
g) couplage par l'intermédiaire d'une fusion peptidique terminale avec (I), ou d'une chaîne cystéine ou lysine en (I), des composants (I) et (II) dans une zone à l'extérieur de la région exposée en surface du feuillet bêta plissé de la molécule polypeptidique (I), laquelle région est prévue pour la liaison spécifique au ligand ;
h) isolement du conjugué ; et
i) démonstration de la fonctionnalité des deux composants du conjugué.

2. Procédé de fabrication d'un conjugué tel que défini dans la revendication 1, à partir du composant (I) de séquence connue, comprenant les étapes suivantes :
- identification des groupes d'acides aminés appropriés pour le couplage au moyen d'une analyse de la structure spatiale de la protéine, de préférence de groupes à l'extérieur de la surface d'interaction entre (I) et le ligand ;
- activation d'un partenaire de couplage au moyen d'un réactif de couplage approprié ;
- réalisation de la réaction de couplage ;
- isolement du conjugué ; et
- démonstration de la fonctionnalité des deux composants du conjugué.

3. Procédé de fabrication d'un conjugué tel que défini dans la revendication 1, à partir du composant (I) de séquence connue, dans lequel aucun groupe d'acide aminé approprié pour le couplage n'a été identifié, comprenant les étapes suivantes :
- introduction de groupes d'acides aminés appropriés pour le couplage au moyen d'une substitution, d'une insertion ou d'une fusion, de préférence de groupes exposés en surface à l'extérieur de la zone d'interaction entre (I) et le ligand ;
- démonstration de l'accessibilité des groupes d'acides aminés introduits ;
- démonstration de la fonctionnalité du composant (I) ainsi modifié ;
- activation d'un partenaire de couplage au moyen d'un réactif de couplage approprié ;
- réalisation de la réaction de couplage ;
- isolement du conjugué ; et
- démonstration de la fonctionnalité des deux composants du conjugué.

4. Procédé selon la revendication 1, dans lequel le couplage est réalisé par l'intermédiaire des groupes lysine 11, 29, 33 et 48 de la molécule d'ubiquitine.

5. Procédé selon la revendication 2, dans lequel la fusion peptidique terminale supplémentaire avec (I) contient un ou plusieurs groupes cystéine ou un ou plusieurs groupes lysine, ces groupes d'acides aminés n'étant de préférence pas impliqués dans l'interaction entre (I) et le ligand.

6. Procédé selon une ou plusieurs des revendications 1 à 5, dans lequel le composant fonctionnel (II) est un peptide, un polypeptide ou une protéine, de préférence un assemblage chromophore-protéine, une enzyme, une immunoglobuline, un dérivé d'immunoglobuline, une toxine ou un polypeptide selon I.

7. Procédé selon une ou plusieurs des revendications 1 à 5, dans lequel le composant fonctionnel (II) est un polymère, de préférence le dextrane, un polyméthacrylate, le sépharose, l'agarose, un polyvinyle, le polystyrène, le gel de silice, la cellulose, le polyéthylèneglycol ou un dérivé de polymère.

8. Procédé selon une ou plusieurs des revendications 1 à 5, dans lequel le composant fonctionnel (II) est une substance de faible masse moléculaire, de préférence un colorant, la biotine, la digoxygénine, un métal lourd, un agent chélatant, un radio-isotope, un antibiotique ou une substance cytotoxique.

9. Procédé selon une ou plusieurs des revendications précédentes, dans lequel le composant (I) présente une propriété de liaison nouvellement générée destinée à la liaison spécifique à un ligand, lequel ligand est un polypeptide ou une protéine, de préférence des immunoglobulines et des dérivés d'immunoglobuline, des protéines obtenues à partir de plasma sanguin, des facteurs et inhibiteurs de la coagulation sanguine, des facteurs de croissance, des interleukines, des cytokines, des protéines de récepteur, des glycoprotéines, des protéines virales et des marqueurs cellulaires de surface, de préférence CD14, CD25, CD34.

10. Procédé selon une ou plusieurs des revendications précédentes, dans lequel le composant (I) présente une propriété de liaison nouvellement générée destinée à la liaison spécifique à un ligand, lequel ligand est un peptide, de préférence une étiquette d'affinité, de manière plus particulièrement préférée une étiquette S, une étiquette T7, une étiquette His, une étiquette Strep, une étiquette Myc, ou une étiquette FLAG, ou des peptides d'origine virale.

11. Procédé selon une ou plusieurs des revendications précédentes, dans lequel le composant (I) présente une propriété de liaison nouvellement générée destinée à la liaison spécifique à un ligand, lequel ligand est une substance de faible masse moléculaire, de préférence des stéroïdes, le cholestérol et des substances nocives telles que, par exemple, les hydrocarbures halogénés.

12. Procédé selon une ou plusieurs des revendications précédentes, dans lequel le composant (II) correspond à un ou plusieurs polypeptides à base d'ubiquitine, qui sont identiques à (I), et reliés à celui-ci par une liaison covalente, ce qui entraîne une augmentation de l'affinité envers le ligand de (I) au moyen d'effets d'avidité.

13. Procédé selon une ou plusieurs des revendications précédentes, dans lequel le composant (II) est un polypeptide, une protéine ou un polymère, lié au composant (I) par l'intermédiaire de plusieurs liaisons covalentes, ce qui entraîne une augmentation de l'affinité envers le ligand de (I) au moyen d'effets d'avidité.

14. Procédé selon une ou plusieurs des revendications précédentes, dans lequel le composant (II) est un polypeptide ou un polymère qui, une fois lié de manière covalente au composant (I), entre dans une liaison covalente ou non covalente avec d'autres conjugués de ce type, ce qui entraîne une augmentation de l'affinité envers le ligand de (I) au moyen d'effets d'avidité.

15. Procédé selon une ou plusieurs des revendications précédentes, dans lequel le composant (I) est SPU11-3-A1 (SEQ ID N° : 12 et 13).

16. Procédé selon une ou plusieurs des revendications précédentes, comprenant en outre la fabrication d'une trousse diagnostique avec un conjugué fabriqué selon l'une ou plusieurs des revendications 1 à 15.

17. Procédé selon une ou plusieurs des revendications précédentes, comprenant en outre la fabrication d'une composition pharmaceutique avec un conjugué fabriqué selon l'une ou plusieurs des revendications 1 à 15.

18. Procédé selon une ou plusieurs des revendications précédentes, comprenant en outre la fabrication d'une composition pour l'enrichissement basé sur l'affinité avec un conjugué fabriqué selon l'une ou plusieurs des revendications 1 à 15, dans lequel le composant fonctionnel est une membrane, une bille de polymère ou un matériau support chromatographique.


### SEQUENCE LISTING

<110> Scil Proteins GmbH
<120> Proteinkonjugate zur Verwendung in Therapie, Diagnose und Chromatographie
<130> P19718
<140>
   <141> 2005-10-11
<150> DE 10 2004 049 479.7
   <151> 2004-10-11
<160> 37
<170> PatentIn version 3.3
<210> 1
   <211> 534
   <212> DNA
   <213> Artificial
<220>
   <223> DNA-Sequenz der humanen gamma-II-Kristallin Bibliothek - CR20
<220>
   <221> misc_feature
   <222> (7)..(8)
   <223> n is a, c, g, or t
<220>
   <221> misc_feature
   <222> (13)..(14)
   <223> n is a, c, g, or t
<220>
   <221> misc_feature
   <222> (19)..(20)
   <223> n is a, c, g, or t
<220>
   <221> misc_feature
   <222> (46)..(47)
   <223> n is a, c, g, or t
<220>
   <221> misc_feature
   <222> (52)..(53)
   <223> n is a, c, g, or t
<220>
   <221> misc_feature
   <222> (58)..(59)
   <223> n is a, c, g, or t
<220>
   <221> misc_feature
   <222> (109)..(110)
   <223> n is a, c, g, or t
<220>
   <221> misc_feature
   <222> (115)..(116)
   <223> n is a, c, g, or t
<400> 1
   atgggtnnka tcnnkttcnn kgaagaccgt gctttccagg gtcgtnnkta cnnktgcnnk    60
   accgactgcc cgaacctgca gccgtacttc tcccgttgca actccatcnn kgttnnktcc   120
   ggttgctgga tgatctacga acgtccgaac taccagggtc accgtcacca gtacttcctg   180
   cggcgtgggg agtaccccga ctaccagcaa tggatgggcc tcagcgactc catccgctcc   240
   tgctgcctca tcccccccca ctctggcgct tacagaatga agatctacga cagagatgaa   300
   ttgaggggac aaatgtcaga gctcacagac gactgtctct ctgttcagga ccgcttccac   360
   ctcactgaaa ttcactccct caatgtgctg gagggcagct ggatcctcta tgagatgccc   420
   aactacaggg ggaggcagta tctgctgagg ccgggggagt acaggaggtt tcttgattgg   480
   ggggctccaa atgccaaagt tggctctctt agacgagtca tggatttgta cgcg         534
<210> 2
   <211> 534
   <212> DNA
   <213> Artificial
<220>
   <223> DNA-Sequenz eines gamma-II-Kristallin basierten Affilins - SPC1-A1
<400> 2
   atgggtttta tctggttcat ggaagaccgt gctttccagg gtcgtaggta cgattgcggt      60
   accgactgcc cgaacctgca gccgtacttc tcccgttgca actccatcaa ggttaagtcc     120
   ggttgctgga tgatctacga acgtccgaac taccagggtc accgtcacca gtacttcctg     180
   cggcgtgggg agtaccccga ctaccagcaa tggatgggcc tcagcgactc catccgctcc     240
   tgctgcctca tcccccccca ctctggcgct tacagaatga agatctacga cagagatgaa     300
   ttgaggggac aaatgtcaga gctcacagac gactgtctct ctgttcagga ccgcttccac     360
   ctcactgaaa ttcactccct caatgtgctg gagggcagct ggatcctcta tgagatgccc     420
   aactacaggg ggaggcagta tctgctgagg ccgggggagt acaggaggtt tcttgattgg     480
   ggggctccaa atgccaaagt tggctctctt agacgagtca tggatttgta cgcg           534
<210> 3
   <211> 534
   <212> DNA
   <213> Artificial
<220>
   <223> DNA-Sequenzen eines gamma-II-Kristallin basierten Affilins - SPC1-A7
<400> 3
   atgggtctga tctgtttctc tgaagaccgt gctttccagg gtcgtaggta catgtgcctg      60
   accgactgcc cgaacctgca gccgtacttc tcccgttgca actccatcaa tgtttggtcc     120
   ggttgctgga tgatctacga acgtccgaac taccagggtc accgtcacca gtacttcctg     180
   cggcgtgggg agtaccccga ctaccagcaa tggatgggcc tcagcgactc catccgctcc     240
   tgctgcctca tcccccccca ctctggcgct tacagaatga agatctacga cagagatgaa     300
   ttgaggggac aaatgtcaga gctcacagac gactgtctct ctgttcagga ccgcttccac     360
   ctcactgaaa ttcactccct caatgtgctg gagggcagct ggatcctcta tgagatgccc     420
   aactacaggg ggaggcagta tctgctgagg ccgggggagt acaggaggtt tcttgattgg     480
   ggggctccaa atgccaaagt tggctctctt agacgagtca tggatttgta cgcg           534
<210> 4
   <211> 534
   <212> DNA
   <213> Artificial
<220>
   <223> DNA-Sequenzen eines gamma-II-Kristallin basierten Affilins - SPC1-G3
<400> 4
   atgggttcta tcattttcct tgaagaccgt gctttccagg gtcgtattta cggttgcact    60
   accgactgcc cgaacctgca gccgtacttc tcccgttgca actccatcgt ggttcagtcc   120
   ggttgctgga tgatctacga acgtccgaac taccagggtc accgtcacca gtacttcctg   180
   cggcgtgggg agtaccccga ctaccagcaa tggatgggcc tcagcgactc catccgctcc   240
   tgctgcctca tcccccccca ctctggcgct tacagaatga agatctacga cagagatgaa   300
   ttgaggggac aaatgtcaga gctcacagac gactgtctct ctgttcagga ccgcttccac   360
   ctcactgaaa ttcactccct caatgtgctg gagggcagct ggatcctcta tgagatgccc   420
   aactacaggg ggaggcagta tctgctgagg ccgggggagt acaggaggtt tcttgattgg   480
   ggggctccaa atgccaaagt tggctctctt agacgagtca tggatttgta cgcg         534
<210> 5
   <211> 549
   <212> DNA
   <213> Artificial
<220>
   <223> DNA-Sequenzen eines gamma-II-Kristallin basierten Affilins - SPC1-A7BB
<400> 5
   atgggtctga tctctttctc tgaagaccgt gctttccagg gtcgtaggta catgtgcctg    60
   accgactgcc cgaacctgca gccgtacttc tcccgttgca actccatcaa tgtttggtcc   120
   ggttgctgga tgatctacga acgtccgaac taccagggtc accagtactt cctgcggcgt   180
   ggggagtacc ccgactacca gcaatggatg ggcctcagcg actccatccg ctcctgctgc   240
   ctcatccccc cccactctgg cgcttacaga atgaagatct acgacagaga tgaattgagg   300
   ggacaaatgt cagagctcac agacgactgt ctctctgttc aggaccgctt ccacctcact   360
   gaaattcact ccctcaatgt gctggagggc agctggatcc tctatgagat gcccaactac   420
   agggggaggc agtatctgct gaggccgggg gagtacagga ggtttcttga ttggggggct   480
   ccaaatgcca aagttggctc tcttagacga gtcatggatt tgtacctcga gcaccaccac   540
   caccaccac                                                           549
<210> 6
   <211> 570
   <212> DNA
   <213> Artificial
<220>
   <223> DNA-Sequenzen eines gamma-II-Kristallin basierten Affilins - SPC1-A7JJ
<400> 6
   atgggtctga tctctttctc tgaagaccgt gctttccagg gtcgtaggta catgtgcctg 60
   accgactgcc cgaacctgca gccgtacttc tcccgttgca actccatcaa tgtttggtcc 120
   ggttgctgga tgatctacga acgtccgaac taccagggtc accagtactt cctgcggcgt 180
   ggggagtacc ccgactacca gcaatggatg ggcctcagcg actccatccg ctcctgctgc 240
   ctcatccccc cccactctgg cgcttacaga atgaagatct acgacagaga tgaattgagg 300
   ggacaaatgt cagagctcac agacgactgt ctctctgttc aggaccgctt ccacctcact 360
   gaaattcact ccctcaatgt gctggagggc agctggatcc tctatgagat gcccaactac 420
   agggggaggc agtatctgct gaggccgggg gagtacagga ggtttcttga ttggggggct 480
   ccaaatgcca aagttggctc tcttagacga gtcatggatt tgtacctcga gtgcggcggc 540
   catcaccatc accaccacca ccaccaccac 570
<210> 7
   <211> 588
   <212> DNA
   <213> Artificial
<220>
   <223> DNA-Sequenzen eines gamma-II-Kristallin basierten Affilins - SPC1-A7-Cys
<400> 7
   atgggtctga tctctttctc tgaagaccgt gctttccagg gtcgtaggta catgtgcctg 60
   accgactgcc cgaacctgca gccgtacttc tcccgttgca actccatcaa tgtttggtcc 120
   ggttgctgga tgatctacga acgtccgaac taccagggtc accagtactt cctgcggcgt 180
   ggggagtacc ccgactacca gcaatggatg ggcctcagcg actccatccg ctcctgctgc 240
   ctcatccccc cccactctgg cgcttacaga atgaagatct acgacagaga tgaattgagg 300
   ggacaaatgt cagagctcac agacgactgt ctctctgttc aggaccgctt ccacctcact 360
   gaaattcact ccctcaatgt gctggagggc agctggatcc tctatgagat gcccaactac 420
   agggggaggc agtatctgct gaggccgggg gagtacagga ggtttcttga ttggggggct 480
   ccaaatgcca aagttggctc tcttagacga gtcatggatt tgtacctcga gtccggcggc 540
   ggggggggag gatcttgcca tcaccatcac caccaccacc accaccac 588
<210> 8
   <211> 549
   <212> DNA
   <213> Artificial
<220>
   <223> DNA-Sequenzen eines gamma-II-Kristallin basierten Affilins - SPC7-E9
<400> 8
   atgggtttta tctgtttctt ggaagaccgt gctttccagg gtcgttctta cgcttgcgat 60
   actgactgcc cgaacctgca gccgtacttc tcccgttgca actccatcag tgttctgtcc 120
   ggttgctgga tgatctacga acgtccgaac taccagggtc accagtactt cctgcggcgt 180
   ggggagtacc ccgactacca gcaatggatg ggcctcagcg actccatccg ctcctgctgc 240
   ctcatccccc cccactctgg cgcttacaga atgaagatct acgacagaga tgaattgagg 300
   ggacaaatgt cagagctcac agacgactgt ctctctgttc aggaccgctt ccacctcact 360
   gaaattcact ccctcaatgt gctggagggc agctggatcc tctatgagat gcccaactac 420
   agggggaggc agtatctgct gaggccgggg gagtacagga ggtttcttga ttggggggct 480
   ccaaatgcca aagttggctc tcttagacga gtcatggatt tgtacctcga gcaccaccac 540
   caccaccac 549
<210> 9
   <211> 228
   <212> DNA
   <213> Artificial
<220>
   <223> DNA-Sequenz der humanen Ubiquitin Bibliothek - Ubiquitin Wildtyp
<400> 9
   atgcagatct tcgtgaagac cctgaccggc aagaccatca ctctggaggt ggagcccagt 60
   gacaccatcg aaaatgtgaa ggccaagatc caagataaag aaggcattcc ccccgaccag 120
   cagaggctca tctttgcagg caagcagctg gaagatggcc gcactctttc tgactacaac 180
   atccagaaag agtcgaccct gcacctggtc ctccgcctga ggggcggc 228
<210> 10
   <211> 225
   <212> DNA
   <213> Artificial
<220>
   <223> Modifiziertes Ubiquitin (MUBI)
<400> 10
   atgcaaatct tcgttaaaac cctgacggga aagactatca ccctggaggt agaaccgtcc 60
   gacaccatcg aaaatgtcaa agctaaaatc caagacaaag aaggaattcc acctgaccag 120
   caacgcctag ctttcgcagg acgacaacta gaggacgggc tcaccctgtc tgactacaac 180
   atccaaaaag aatccaccct ccacctggca ctcctcctgc gggcc 225
<210> 11
   <211> 225
   <212> DNA
   <213> Artificial
<220>
   <223> UB10 (Bibliothek)
<220>
   <221> misc_feature
   <222> (4)..(5)
   <223> n is a, c, g, or t
<220>
   <221> misc_feature
   <222> (10)..(11)
   <223> n is a, c, g, or t
<220>
   <221> misc_feature
   <222> (16)..(17)
   <223> n is a, c, g, or t
<220>
   <221> misc_feature
   <222> (184)..(185)
   <223> n is a, c, g, or t
<220>
   <221> misc_feature
   <222> (187)..(188)
   <223> n is a, c, g, or t
<220>
   <221> misc_feature
   <222> (190)..(191)
   <223> n is a, c, g, or t
<220>
   <221> misc_feature
   <222> (193)..(194)
   <223> n is a, c, g, or t
<220>
   <221> misc_feature
   <222> (196)..(197)
   <223> n is a, c, g, or t
<400> 11
   atgnnkatcn nkgttnnkac cctgacggga aagactatca ccctggaggt agaaccgtcc      60
   gacaccatcg aaaatgtcaa agctaaaatc caagacaaag aaggaattcc acctgaccag     120
   caacgcctag ctttcgcagg acgacaacta gaggacgggc tcaccctgtc tgactacaac     180
   atcnnknnkn nknnknnkct ccacctggca ctcctcctgc gggcc                     225
<210> 12
   <211> 249
   <212> DNA
   <213> Artificial
<220>
   <223> DNA-Sequenz eines Ubiquitin basierten Affilins - SPU11-3-A1
<400> 12
   atgcggatcc gtgttgctac cctgacggga aagactatca ccctggaggt agaaccgtcc 60
   gacaccatcg aaaatgtcaa agctaaaatc caagacaaag aaggaattcc acctgaccag 120
   caacgcctag ctttcgcagg acgacaacta gaggacgggc tcaccctgtc tgactacgac 180
   atccgtcatg gtacgtcgct ccacctggca ctcctcctgc gggccctcga gcaccaccac 240
   caccaccac 249
<210> 13
   <211> 288
   <212> DNA
   <213> Artificial
<220>
   <223> DNA-Sequenz eines Ubiquitin basierten Affilins - SPU11-3-A1_Cys
<400> 13
   atgcggatcc gtgttgctac cctgacggga aagactatca ccctggaggt agaaccgtcc 60
   gacaccatcg aaaatgtcaa agctaaaatc caagacaaag aaggaattcc acctgaccag 120
   caacgcctag ctttcgcagg acgacaacta gaggacgggc tcaccctgtc tgactacgac 180
   atccgtcatg gtacgtcgct ccacctggca ctcctcctgc gggccctcga gtccggcggc 240
   ggggggggag gatcttgcca tcaccatcac caccaccacc accaccac 288
<210> 14
   <211> 26
   <212> DNA
   <213> Artificial
<220>
   <223> Primer
<400> 14
   ccatgattac gccaagcttt ggagcc 26
<210> 15
   <211> 31
   <212> DNA
   <213> Artificial
<220>
   <223> Primer
<400> 15
   ccatgggtct gatctctttc tctgaagacc g 31
<210> 16
   <211> 31
   <212> DNA
   <213> Artificial
<220>
   <223> Primer
<400> 16
   cggtcttcag agaaagagat cagacccatg g 31
<210> 17
   <211> 23
   <212> DNA
   <213> Artificial
<220>
   <223> Primer
<400> 17
   gctagttatt gctcagcggt ggc 23
<210> 18
   <211> 66
   <212> DNA
   <213> Artificial
<220>
   <223> Primer
<400> 18
   ggatttgtac ctcgagtgcg gcggccatca ccatcaccac caccaccacc accactgaga 60
   tccggc 66
<210> 19
   <211> 66
   <212> DNA
   <213> Artificial
<220>
   <223> Primer
<400> 19
   gccggatctc agtggtggtg gtggtggtgg tgatggtgat ggccgccgca ctcgaggtac 60
   aaatcc 66
<210> 20
   <211> 32
   <212> DNA
   <213> Artificial
<220>
   <223> Primer
<400> 20
   ggatttgtac ctcgagtccg gcggccatca cc 32
<210> 21
   <211> 32
   <212> DNA
   <213> Artificial
<220>
   <223> Primer
<400> 21
   ggtgatggcc gccggactcg aggtacaaat cc 32
<210> 22
   <211> 32
   <212> DNA
   <213> Artificial
<220>
   <223> Primer
<400> 22
   ggtgatggcc gccggactcg aggtacaaat cc 32
<210> 23
   <211> 54
   <212> DNA
   <213> Artificial
<220>
   <223> Primer
<400> 23
   gggggaagct tttatcagtg gtggtggtgg tggtggtgat ggtgatggca agat 54
<210> 24
   <211> 35
   <212> DNA
   <213> Artificial
<220>
   <223> Primer
<400> 24
   ggagatatac aatatgggtc tgatctcttt ctctg 35
<210> 25
   <211> 291
   <212> DNA
   <213> Artificial
<220>
   <223> DNA-Sequenz für ein modifiziertes Ubiquitin-Proteingerüst
<400> 25
   atgaaatacc tattgcctac ggcagccgct ggattgttat tactcgcggc ccagccggcc 60
   atggccatgc aaatcttcgt taaaaccctg acgggaaaga ctatcaccct ggaggtagaa 120
   ccgtccgaca ccatcgaaaa tgtcaaagct aaaatccaag acaaagaagg aattccacct 180
   gaccagcaac gcctagcttt cgcaggacga caactagagg acgggctcac cctgtctgac 240
   tacaacatcc aaaaagaatc caccctccac ctggcactcc tcctgcgggc c 291
<210> 26
   <211> 50
   <212> DNA
   <213> Artificial
<220>
   <223> Template
<400> 26
   atgcaaatct tcgttaaaac cctgacggga aagactatca ccctggaggt 50
<210> 27
   <211> 53
   <212> DNA
   <213> Artificial
<220>
   <223> Template
<400> 27
   ggattttagc tttgacattt tcgatggtgt cggacggttc tacctccagg gtg 53
<210> 28
   <211> 53
   <212> DNA
   <213> Artificial
<220>
   <223> Template
<400> 28
   gtcaaagcta aaatccaaga caaagaagga attccacctg accagcaacg cct 53
<210> 29
   <211> 50
   <212> DNA
   <213> Artificial
<220>
   <223> Template
<400> 29
   gggtgagccc gtcctctagt tgtcgtcctg cgaaagctag gcgttgctgg 50
<210> 30
   <211> 50
   <212> DNA
   <213> Artificial
<220>
   <223> Template
<400> 30
   gacgggctca ccctgtctga ctacaacatc caaaaagaat ccaccctcca 50
<210> 31
   <211> 39
   <212> DNA
   <213> Artificial
<220>
   <223> Template
<400> 31
   gagtgctcgc agcaggagtg ccaggtggag ggtggattc 39
<210> 32
   <211> 22
   <212> DNA
   <213> Artificial
<220>
   <223> Primer
<400> 32
   gatatacata tgcaaatctt cg 22
<210> 33
   <211> 18
   <212> DNA
   <213> Artificial
<220>
   <223> Primer
<400> 33
   gtggtgctcg agtgctcg 18
<210> 34
   <211> 55
   <212> DNA
   <213> Artificial
<220>
   <223> Primer
<220>
   <221> misc_feature
   <222> (20)..(21)
   <223> n is a, c, g, or t
<220>
   <221> misc_feature
   <222> (26)..(27)
   <223> n is a, c, g, or t
<220>
   <221> misc_feature
   <222> (32)..(33)
   <223> n is a, c, g, or t
<400> 34
   ccagccggcc atggccatgn nkatcnnkgt tnnkaccctg acgggaaaga ctatc 55
<210> 35
   <211> 55
   <212> DNA
   <213> Artificial
<220>
   <223> Primer
<220>
   <221> misc_feature
   <222> (23)..(24)
   <223> n is a, c, g, or t
<220>
   <221> misc_feature
   <222> (26)..(27)
   <223> n is a, c, g, or t
<220>
   <221> misc_feature
   <222> (29)..(30)
   <223> n is a, c, g, or t
<220>
   <221> misc_feature
   <222> (32)..(33)
   <223> n is a, c, g, or t
<220>
   <221> misc_feature
   <222> (35)..(36)
   <223> n is a, c, g, or t
<400> 35
   caggaggagt gccaggtgga gmnnmnnmnn mnnmnngatg ttgtagtcag acagg 55
<210> 36
   <211> 34
   <212> DNA
   <213> Artificial
<220>
   <223> Primer
<400> 36
   gttattactc gcggcccagc cggccatggc catg 34
<210> 37
   <211> 48
   <212> DNA
   <213> Artificial
<220>
   <223> Primer
<400> 37
   gagtttttgt tcggcctcga gggcccgcag gaggagtgcc aggtggag 48